# FASCICULE DE BREVET EUROPEEN

(11) **EP 3 445 270 B1**
(45) Date de publication et mention de la délivrance du brevet: **06.07.2022**
(21) Numéro de dépôt: 17717766.4
(22) Date de dépôt: 21.04.2017
(51) Int. Cl.: A61C 7/00, A61C 7/08

(54) **SYSTÈME DE FABRICATION D'UN APPAREIL ORTHODONTIQUE**
SYSTEM ZUR HERSTELLUNG EINER ORTHODONTISCHEN VORRICHTUNG
SYSTEM FOR PRODUCING AN ORTHODONTIC APPARATUS

(30) Priorité: 22.04.2016 FR 1653595
(43) Date de publication de la demande: 27.02.2019
(60) Demande divisionnaire: 22175789.1
(73) Titulaire: Dental Monitoring, 75008 Paris (FR)
(72) Inventeur: SALAH, Philippe, 75020 Paris (FR); AYACHE, William, 92200 Neuilly sur Seine (FR); GHYSELINCK, Guillaume, 59169 Cantin (FR); DEBRAUX, Laurent, 75020 Paris (FR); PELLISSARD, Thomas, 92110 Clichy (FR)
(74) Mandataire: Nony
(86) Numéro de dépôt international: PCT/EP2017/059555
(87) Numéro de publication internationale: WO 2017/182654

(56) Documents cités:
- WO-A1-2008/149221
- WO-A1-2016/066637
- WO-A1-2016/066642
- WO-A1-2016/066654
- US-A1- 2009 191 503

## Description

### Domaine technique

La présente invention concerne un système de fabrication d'un appareil orthodontique en substitution à un appareil orthodontique porté par un patient.

### Etat de la technique

Il est souhaitable que chacun fasse régulièrement contrôler sa dentition, notamment afin de vérifier que la position et/ou la forme et/ou l'aspect de ses dents n'évolue pas défavorablement.

Lors d'un traitement orthodontique, cette évolution défavorable peut notamment conduire à modifier le traitement. Après un traitement orthodontique, cette évolution défavorable, appelée « récidive », peut conduire à une reprise d'un traitement. Enfin, de manière plus générale et indépendamment de tout traitement, chacun peut souhaiter suivre les déplacements éventuels et/ou le changement de forme et/ou d'aspect de ses dents.

Classiquement, les contrôles sont effectués par un orthodontiste ou un dentiste qui seuls disposent d'un appareillage adapté. Ces contrôles sont donc coûteux. En outre, les visites sont contraignantes.

US 2009/0291417 décrit un procédé permettant de créer, puis de modifier des modèles tridimensionnels, notamment pour la fabrication d'appareils orthodontiques.

Un objectif de la présente invention est de répondre, au moins partiellement, aux problèmes susmentionnés.

### Résumé de l'invention

On décrit un procédé de contrôle du positionnement et/ou de la forme de dents d'un patient, ledit procédé comportant les étapes suivantes :
a) réalisation d'un modèle de référence tridimensionnel numérique d'au moins une partie d'une arcade, de préférence d'au moins une arcade du patient, ou « modèle de référence initial » et, de préférence, pour chaque dent, définition, à partir du modèle de référence initial, d'un modèle de référence tridimensionnel numérique de ladite dent, ou « modèle de dent » ;
b) acquisition d'au moins une image bidimensionnelle des arcades du patient, dite « image actualisée », dans des conditions d'acquisition réelles ;
c) analyse de chaque image actualisée et réalisation, pour chaque image actualisée, d'une carte actualisée relative à une information discriminante ;
d) optionnellement, détermination, pour chaque image actualisée, de conditions d'acquisition virtuelles grossières approximant lesdites conditions d'acquisition réelles ;
e) recherche, pour chaque image actualisée, d'un modèle de référence actualisé correspondant au positionnement et/ou à la forme des dents lors de l'acquisition de l'image actualisée, la recherche étant de préférence effectuée au moyen d'une méthode métaheuristique, de préférence évolutionniste, de préférence par recuit simulé, et
f) pour chaque modèle de dent, comparaison des positionnements dudit modèle de dent dans le modèle de référence initial et dans le modèle de référence obtenu à l'issue des étapes précédentes, dit « modèle de référence actualisé », afin de déterminer le déplacement des dents entre les étapes a) et b), et/ou
   comparaison des formes du modèle de référence initial et du modèle de référence obtenu à l'issue des étapes précédentes, dit « modèle de référence actualisé », afin de déterminer la déformation et/ou le déplacement de dents entre les étapes a) et b).

Comme on le verra plus en détail dans la suite de la description, un tel procédé de contrôle du positionnement et/ou de la forme de dents permet, à partir d'une simple image des dents, prise sans pré-positionnement précis des dents par rapport à l'appareil d'acquisition d'images, par exemple d'une photographie prise par le patient, d'évaluer avec précision le déplacement et/ou la déformation des dents depuis la réalisation du modèle de référence initial. Cette évaluation peut en outre être effectuée par un simple ordinateur, un serveur ou un téléphone mobile.

De préférence, l'étape e) comporte :
- une première opération d'optimisation permettant de rechercher des conditions d'acquisition virtuelles correspondant au mieux aux conditions d'acquisition réelles dans un modèle de référence à tester déterminé à partir du modèle de référence initial, et
- une deuxième opération d'optimisation permettant de rechercher, en testant une pluralité de dits modèles de référence à tester, le modèle de référence correspondant au mieux au positionnement et/ou à la forme des dents du patient lors de l'acquisition de l'image actualisée à l'étape b).

De préférence, une première opération d'optimisation est effectuée pour chaque test d'un modèle de référence à tester lors de la deuxième opération d'optimisation.

De préférence, la première opération d'optimisation et/ou la deuxième opération d'optimisation, de préférence la première opération d'optimisation et la deuxième opération d'optimisation mettent en œuvre une méthode métaheuristique, de préférence évolutionniste, de préférence un recuit simulé.

De préférence, l'étape e) comporte les étapes suivantes :
e1) définition d'un modèle de référence à tester comme étant le modèle de référence initial puis,
e2) suivant les étapes suivantes, test de conditions d'acquisition virtuelles avec le modèle de référence à tester afin d'approximer finement lesdites conditions d'acquisition réelles ;
   e21) détermination de conditions d'acquisition virtuelles à tester;
   e22) réalisation d'une image de référence bidimensionnelle du modèle de référence à tester dans lesdites conditions d'acquisition virtuelles à tester ;
   e23) traitement de l'image de référence pour réaliser au moins une carte de référence représentant, au moins partiellement, ladite information discriminante ;
   e24) comparaison des cartes actualisée et de référence de manière à déterminer une valeur pour une première fonction d'évaluation, ladite valeur pour la première fonction d'évaluation dépendant des différences entre lesdites cartes actualisée et de référence et correspondant à une décision de poursuivre ou de stopper la recherche de conditions d'acquisition virtuelles approximant lesdites conditions d'acquisition réelles avec plus d'exactitude que lesdites conditions d'acquisition virtuelles à tester déterminées à la dernière occurrence de l'étape e21) ;
   e25) si ladite valeur pour la première fonction d'évaluation correspond à une décision de poursuivre ladite recherche, modification des conditions d'acquisition virtuelles à tester, puis reprise à l'étape e22) ;
e3) détermination d'une valeur pour une deuxième fonction d'évaluation, ladite valeur pour la deuxième fonction d'évaluation dépendant des différences entre les cartes actualisée et de référence dans les conditions d'acquisition virtuelles approximant au mieux lesdites conditions d'acquisition réelles et résultant de la dernière occurrence de l'étape e2), ladite valeur pour la deuxième fonction d'évaluation correspondant à une décision de poursuivre ou de stopper la recherche d'un modèle de référence approximant la forme et/ou le positionnement des dents lors de l'acquisition de l'image actualisée avec plus d'exactitude que ledit modèle de référence à tester utilisé à la dernière occurrence de l'étape e2), et si ladite valeur pour la deuxième fonction d'évaluation correspond à une décision de poursuivre ladite recherche, modification du modèle de référence à tester par déformation du modèle de référence à tester et/ou par déplacement et/ou déformation d'un ou plusieurs modèles de dents, puis reprise à l'étape e2).

Un tel procédé de contrôle du positionnement et/ou de la forme de dents peut encore comporter une ou plusieurs des caractéristiques optionnelles suivantes :
- à l'étape a), on détermine un plan d'occlusion suivant les opérations suivantes :
   I. détermination des points du modèle de référence initial qui appartiennent à une arcade et qui sont à une distance de l'autre arcade qui est inférieure à une distance prédéterminée, de préférence à une distance inférieure à 3 mm de l'autre arcade, dits « points de contact » ;
   II. optionnellement, filtrage d'une partie des points de contact, de préférence de manière que le nombre de points de contact appartenant à l'arcade supérieure soit identique au nombre de points de contact appartenant à l'arcade inférieure, de préférence en éliminant les points d'une arcade les plus éloignés de l'autre arcade ;
   III. régression linéaire, de préférence par la méthode des moindres carrés, sur l'ensemble des points de contact restant de manière à déterminer le plan d'occlusion ;
- à l'étape a), on effectue les opérations suivantes :
   i. projection, dans un plan d'occlusion, des points de contact entre les dents des arcades supérieure et inférieure du patient, les points de contact et/ou le plan d'occlusion étant de préférence déterminés, suivant les étapes I à III ;
   ii. détermination du barycentre des projections desdits points de contact et création d'un référentiel, dans le plan d'occlusion, centré sur ledit barycentre ;
   iii. détermination, dans ledit référentiel, de la fonction parabolique, présentant le plus grand coefficient de corrélation avec l'ensemble des projections des points de contact ;
   iv. rotation de l'ensemble des projections des points de contact autour du barycentre, et reprise de l'opération précédente *iii* jusqu'à ce que l'ensemble des projections des points de contact ait parcouru un secteur déterminé, de préférence supérieur à 90°, supérieur à 180°, voire d'environ 360° ;
   v. identification du coefficient de corrélation le plus élevé pour l'ensemble des positions angulaires de l'ensemble des projections des points de contact autour du barycentre, et de l'axe de la fonction parabolique correspondante ;
   vi. détermination d'un plan longitudinal médian du modèle de référence initial, ledit plan passant par ledit axe et étant perpendiculaire au plan d'occlusion ;
- à l'étape a), on délimite au moins partiellement un modèle de dent en suivant les opérations suivantes :
   i'. détermination, au moins partielle, des bords gingivaux intérieur et extérieur de l'arcade de la dent concernée, de préférence par analyse des variations de l'orientation de la surface du modèle de référence initial ;
   ii'. projection, dans le plan d'occlusion, des bords gingivaux intérieur et extérieur ;
   iii'. identification des déformations des projections des bords gingivaux intérieur et extérieur correspondant aux régions interdentaires, les sommets de ces déformations étant appelés « point de rapprochement » (dans une région interdentaire, les deux projections présentent chacune une pointe, les deux pointes pointant sensiblement l'une vers l'autre, l'extrémité d'une pointe étant un point de rapprochement) ;
   iv'. détermination du plus court chemin, à la surface du modèle de référence initial, entre deux points de rapprochement de bords gingivaux intérieur et extérieur, respectivement, d'une région interdentaire, de préférence par une méthode métaheuristique, de préférence évolutionniste, de préférence par recuit simulé, ledit plus court chemin délimitant au moins partiellement un modèle de dent ;
- une image actualisée est acquise moins de 7 jours après l'étape a), puis les étapes c) à f) sont mises en œuvre à partir de cette image actualisée ;
- l'intervalle de temps entre les étapes a) et b) ou entre les étapes A et B peut être supérieur à 1 semaine, à 2 semaines, à 1 mois, à 2 mois ou à 6 mois ;
- à l'étape b), on utilise un appareil d'acquisition porté à la main (et en particulier qui n'est pas immobilisé, par exemple au moyen d'un support reposant sur le sol) et/ou la tête du patient n'est pas immobilisée ;
- on utilise un appareil individuel choisi dans le groupe formé par un appareil photo connecté, une montre intelligente, une tablette numérique, un scanner 3D portable et un ordinateur couplé un système d'acquisition d'images, telle une webcam ou un appareil photo numérique, pour mettre en œuvre l'étape b) et, de préférence au moins une des étapes, de préférence toutes les étapes c) à f) ;
- à l'étape b), on utilise un écarteur comportant une, de préférence plus de deux marques de repérage, de préférence non alignées, et, de préférence, on utilise la représentation des marques de repérage sur l'image actualisée pour
   - à l'étape c), redécouper l'image et/ou,
   - à l'étape d), évaluer grossièrement les conditions d'acquisition réelles ;
- à l'étape c), l'information discriminante est choisie dans le groupe constitué par une information de contour, une information de couleur, une information de densité, une information de distance, une information de brillance, une information de saturation, une information sur les reflets et des combinaisons de ces informations ;
- à l'étape c), l'information discriminante est une information discriminante optimale obtenue au moyen d'un procédé d'optimisation décrit ci-après ;
- à l'étape d), on utilise des données fournies par l'appareil d'acquisition et, de préférence, concernant son orientation ;
- à l'étape e2), les conditions d'acquisition virtuelles recherchées comprennent des paramètres de calibration de l'appareil d'acquisition mis en œuvre à l'étape b) ;
- on met en œuvre une optimisation par une méthode métaheuristique, de préférence évolutionniste, de préférence par recuit simulé pour :
   - à l'étape a), déterminer au moins partiellement un bord gingival délimitant un modèle de dent, et/ou
   - à l'étape e2), rechercher les conditions d'acquisition virtuelles correspondant aux conditions d'acquisition réelles, et/ou
   - à l'étape e), rechercher un modèle de référence correspondant à l'image actualisée ;
- ladite méthode métaheuristique est choisie dans le groupe formé par
   - les algorithmes évolutionnistes, de préférence choisie parmi: les stratégies d'évolution, les algorithmes génétiques, les algorithmes à évolution différentielle, les algorithmes à estimation de distribution, les systèmes immunitaires artificiels, la recomposition de chemin Shuffled Complex Evolution, le recuit simulé, les algorithmes de colonies de fourmis, les algorithmes d'optimisation par essaims particulaires, la recherche avec tabous, et la méthode GRASP ;
   - l'algorithme du kangourou,
   - la méthode de Fletcher et Powell,
   - la méthode du bruitage,
   - la tunnelisation stochastique,
   - l'escalade de collines à recommencements aléatoires,
   - la méthode de l'entropie croisée, et
   - les méthodes hybrides entre les méthodes métaheuristiques citées ci-dessus ;
- à partir de la comparaison à l'étape f), on produit une cartographie faisant apparaître les changements de forme du modèle de référence initial et/ou le déplacement d'un ou plusieurs modèles de dent ;
- l'information discriminante utilisée pour la carte actualisée et/ou pour la carte de référence est, préalablement à cette utilisation, optimisée au moyen d'un procédé d'optimisation décrit ci-après, comportant des étapes C1. à C3., l'image acquise étant l'image actualisée ou l'image de référence, respectivement, et le modèle de référence étant le modèle de référence initial ou le modèle de référence à tester, respectivement.

On décrit aussi l'utilisation d'un procédé de contrôle du positionnement de dents pour
- détecter une récidive, et/ou
- déterminer une vitesse d'évolution d'un changement de positionnement des dents, et/ou
- optimiser la date de prise de rendez-vous chez un orthodontiste ou un dentiste, et/ou
- évaluer l'efficacité d'un traitement orthodontique, et/ou
- évaluer l'évolution du positionnement de dents vers un modèle théorique correspondant à un positionnement déterminé des dents, en particulier un positionnement amélioré des dents, et/ou
- en dentisterie.

Le procédé peut notamment être mis en œuvre pendant un traitement orthodontique, notamment pour en contrôler le déroulement, l'étape a) étant mise en œuvre moins de 3 mois, moins de 2 mois, moins de 1 mois, moins d'une semaine, moins de 2 jours après le début du traitement, c'est-à-dire après la pose d'un appareil destiné à corriger le positionnement des dents du patient, dit « appareil de contention actif ».

Le procédé peut être également mis en œuvre après un traitement orthodontique, pour vérifier que le positionnement des dents n'évolue pas de façon défavorable (« récidive »). L'étape a) est alors de préférence mise en œuvre moins de 3 mois, moins de 2 mois, moins de 1 mois, moins d'une semaine, moins de 2 jours après la fin du traitement, c'est-à-dire après la pose d'un appareil destiné maintenir les dents en position, dit « appareil de contention passif ».

Dans un mode de réalisation, pour contrôler exclusivement le déplacement des dents, on considère que les modèles de dents sont indéformables lors de l'étape e). En particulier, à l'étape e3), le modèle de référence à tester ne peut être modifié que par déplacement d'un ou plusieurs modèles de dents.

On décrit encore un procédé d'adaptation d'un appareil orthodontique en fonction de résultats obtenus avec ledit appareil orthodontique.

Classiquement, au début d'un traitement orthodontique, l'orthodontiste détermine le positionnement des dents qu'il souhaite obtenir à l'issue du traitement, dit "set-up final". Le set-up final peut être défini au moyen d'une empreinte ou à partir d'un scan tridimensionnel des dents du patient. L'orthodontiste fabrique alors, en conséquence, un appareil orthodontique adapté à ce traitement.

A intervalles réguliers, le patient se déplace chez l'orthodontiste pour un contrôle visuel. En fonction de son diagnostic, l'orthodontiste modifie éventuellement l'appareil orthodontique.

Par exemple, dans le cas où l'appareil orthodontique est un appareil comportant un arc orthodontique métallique attaché aux dents, il peut modifier la tension exercée par l'arc orthodontique. Le cas échéant, il peut également faire fabriquer un nouvel appareil orthodontique mieux ajusté.

L'appareil orthodontique peut être une gouttière (*« aligner »* en anglais). Une gouttière orthodontique se présente classiquement sous la forme un appareil monobloc amovible, classiquement en un matériau polymère transparent, qui comporte une goulotte conformée pour que plusieurs dents d'une arcade, généralement toutes les dents d'une arcade, puissent y être logées. La forme de la goulotte est adaptée pour maintenir en position la gouttière sur les dents, tout en exerçant une action de correction de la position de certaines dents.

Le traitement au moyen de gouttières est avantageusement moins contraignant pour le patient. En particulier, le nombre de rendez-vous chez l'orthodontiste est limité. En outre, la douleur est plus faible qu'avec un arc orthodontique métallique attaché aux dents.

Le marché des gouttières orthodontiques est donc en croissance.

Dans le cas où l'appareil orthodontique est une gouttière, on détermine classiquement, au début du traitement, les formes que doivent prendre les différentes gouttières à différents moments du traitement, puis on fait fabriquer l'ensemble des gouttières correspondantes. A 10 des instants prédéterminés, le patient change de gouttière. Des contrôles peuvent être effectués à intervalles réguliers. Si l'orthodontiste diagnostique une inadaptation du traitement, il effectue une nouvelle empreinte des dents, ou, de manière équivalente, un nouveau scan tridimensionnel des dents, puis commande une nouvelle série de gouttières. On considère qu'en moyenne, le nombre de gouttières finalement fabriquées est d'environ 45, au lieu des 20 gouttières classiquement prévues au début du traitement.

WO2008/149221 décrit un procédé permettant de détecter une dérive dans le traitement. Cette dérive peut être mesurée en comparant des modèles numériques des dents du patient. En cas de dérive, le traitement peut être recalculé. WO 2008/149221 ne suggère cependant aucune solution permettant d'obtenir une mesure précise de la dérive sans rendez-vous chez l'orthodontiste.

La nécessité de devoir se déplacer chez l'orthodontiste est une contrainte pour le patient. La confiance du patient en son orthodontiste peut être également entamée. Enfin, il en résulte un coût supplémentaire.

Le nombre de contrôles chez l'orthodontiste doit donc être limité.

Un but de l'invention est de répondre, au moins partiellement, à ce problème.

L'invention propose un système selon la revendication 1, les revendications dépendantes portant sur des caractéristiques optionnelles.

Un système selon l'invention est destiné à la mise en œuvre d'un procédé d'adaptation d'un appareil orthodontique porté par un patient, le procédé comportant les étapes suivantes :
a') Réalisation d'un modèle de référence initial d'au moins une partie d'une arcade, de préférence d'au moins une arcade, de préférence des deux arcades du patient, conformément à l'étape a) ; b') Indépendamment des étapes a') et c'), mais avant l'étape d'), réalisation d'un modèle de référence tridimensionnel numérique, dit "modèle de référence objectif", correspondant à un positionnement, souhaité à un instant du traitement, en particulier souhaité à la fin du traitement avec ledit appareil orthodontique, des dents de ladite au moins une partie de ladite arcade du patient ;
c') Acquisition d'au moins une image actualisée, dans des conditions d'acquisition réelles, et recherche, par déformation du modèle de référence initial, d'un modèle de référence actualisé correspondant au positionnement des dents lors de l'acquisition de l'image actualisée, la recherche étant de préférence effectuée au moyen d'une méthode métaheuristique, de préférence évolutionniste, de préférence par recuit simulé, de préférence conformément aux étapes b) à e) ;
d') Détermination, en fonction du modèle de référence initial et/ou du modèle de référence objectif, d'un modèle de comparaison dans lequel les dents du modèle de référence initial sont dans un positionnement anticipé, de préférence tel que prévu sensiblement pour l'instant de l'acquisition réalisée à l'étape c') ;
e') Comparaison du modèle de référence actualisé avec le modèle de comparaison ;
f) Si, à l'étape e'), ladite comparaison aboutit à un résultat insatisfaisant, fabrication d'un appareil orthodontique de substitution, par fabrication d'un nouvel appareil orthodontique, l'appareil orthodontique de substitution étant configuré en fonction dudit résultat.

L'appareil orthodontique de substitution est conçu en fonction des résultats de la comparaison entre un positionnement réel des dents, évalué au moyen du modèle de référence actualisé, et un positionnement anticipé, correspondant au modèle de comparaison. L'appareil orthodontique de substitution est configuré au moment du cycle de contrôle (étapes c') à f')) et peut donc être bien adapté à la réalité du traitement.

Avantageusement, le cycle de contrôle peut être fait à distance, à partir de simples photographies prises par un téléphone mobile, sans que le patient ait à se déplacer, chez l'orthodontiste en particulier. Comme on le verra plus en détail dans la suite de la description, la fabrication du modèle de référence actualisé à l'étape c') est en effet possible sans précaution particulière, notamment parce que le positionnement réel des dents est mesuré avec un modèle de référence actualisé qui résulte d'une déformation du modèle de référence initial afin qu'il corresponde aux observations fournies par les images actualisées, c'est-à-dire afin que les images actualisées soient des vues du modèle de référence initial déformé.

Le patient et/ou l'orthodontiste peuvent donc facilement évaluer le traitement, et en particulier l'efficacité de l'appareil orthodontique porté par le patient, sans avoir besoin de se déplacer chez un orthodontiste. L'étape c') peut donc être répétée autant que souhaité.

En outre, il devient possible, en pratique, de ne prévoir un rendez-vous chez l'orthodontiste que lorsque la comparaison de l'étape e') aboutit à un résultat insatisfaisant et qu'un appareil orthodontique de substitution doit être fabriqué.

Par ailleurs, la précision du modèle de référence initial permet d'obtenir un modèle de référence actualisé également très précis, sans qu'un nouveau scan des dents ne soit nécessaire. Les essais effectués ont montré que les images actualisées, et en particulier les photographies prises à l'étape c') suffisent pour déterminer, avec précision, la déformation à appliquer au modèle de référence initial. La position des dents représentées sur les images actualisées déterminée par déformation du modèle de référence initial par analyse des images actualisées correspond avec une très grande précision à la réalité mesurée.

Pour améliorer encore la précision, on utilise de préférence plusieurs images actualisées.

Le modèle de référence actualisé est avantageusement exclusivement déterminé à partir d'informations relatives au patient, indépendamment de toute considération statistique sur des traitements orthodontiques de tiers. Le modèle de référence actualisé est ainsi spécifique au patient.

L'invention repose donc sur la possibilité de combiner deux sources d'informations de natures différentes, à savoir un modèle de référence initial précis mais n'apportant aucune information sur le déplacement des dents, et une ou plusieurs images actualisées comportant peu d'informations sur le positionnement précis des dents mais dont l'analyse permet, au moyen du modèle de référence initial, de déterminer avec précision le déplacement des dents représentées depuis l'instant auquel le modèle de référence intial a été créé. De préférence, la déformation du modèle est effectuée au moyen d'une méthode métaheuristique par laquelle l'image actualisée est comparée à une succession de modèles de référence obtenus par déformation du modèle de référence initial, le modèle le plus proche de l'image actualisée, c'est-à-dire permettant une observation dudit modèle correspondant à l'image actualisée, étant retenu pour constituer le modèle de référence actualisé.

Dans le cas d'un traitement orthodontique au moyen de gouttières, le procédé permet également de limiter le nombre de gouttières fabriquées. Les gouttières peuvent être en particulier fabriquées tout au long du traitement, ce qui leur permet d'être parfaitement adaptées à la situation réelle au moment où elles doivent être utilisées.

Enfin, le coût et la durée de traitement en sont réduits.

Le procédé d'adaptation peut encore comporter, notamment, une ou plusieurs des caractéristiques optionnelles suivantes :
- le modèle de comparaison peut être en particulier le modèle de référence objectif ou, de préférence, un modèle tridimensionnel numérique fournissant une estimation du positionnement, prévu pour un instant intermédiaire, des dents du modèle de référence initial, dit "modèle de référence intermédiaire" ;
- de préférence, le modèle de comparaison correspond à un positionnement des dents anticipé sensiblement pour l'instant de ladite acquisition. De préférence, il fournit une estimation du positionnement, prévu pour un instant intermédiaire, des dents du modèle de référence initial, la durée entre ledit instant intermédiaire et l'instant de l'acquisition réalisée à l'étape c') étant inférieur à 1 mois, de préférence inférieur à 1 semaine, de préférence inférieur à 24 heures ;
- le procédé comporte plus de 2, plus de 5, plus de 10, voire plus de 20 cycles de contrôle, chaque cycle de contrôle étant constitué par une exécution des étapes c') à f') ;
- à l'étape d'), le modèle de comparaison est déterminé en fonction de modèles de référence actualisés déterminés lors d'une ou plusieurs étapes c') de cycles de contrôle antérieurs au cycle de contrôle comportant ladite étape d'), en particulier en fonction du ou des modèles de référence actualisés déterminés lors d'étapes c') de cycles de contrôle précédant immédiatement le cycle de contrôle comportant ladite étape d') ;
- à l'étape c'), ladite image actualisée est acquise au moyen d'un téléphone du patient, la recherche du modèle de référence actualisé est de préférence réalisée au moyen dudit téléphone, et le modèle de référence actualisé est transmis, par ledit téléphone, à un orthodontiste ;
- avant chaque date prévue pour une étape c'), de préférence moins de 3 semaines, de préférence moins de 2 semaines, de préférence moins d'une semaine avant ladite date, on envoie un message de rappel au patient, de préférence sur son téléphone, afin qu'il effectue ladite étape c') ;
- les étapes c') à e') de chaque cycle de contrôle sont effectuées « à distance », c'est-à-dire sans que le patient se déplace chez l'orthodondiste ;
- à l'étape d'), le modèle de comparaison est déterminé en fonction de valeurs de paramètres de l'appareil orthodontique porté ;
- à l'étape e'), on établit, à partir de ladite comparaison, un rapport fournissant des informations de diagnostic et/ou des préconisations pour modifier le traitement du patient ;
- à l'étape e'),
   - on établit, à partir de ladite comparaison, un score, dit « actualisé », représentatif de l'efficacité du traitement, et,
   - de préférence, on compare ledit score actualisé à un score de référence représentatif de l'efficacité d'un traitement standard équivalent et on présente au patient le résultat de la comparaison entre les scores actualisé et de référence ;
- à l'étape f'), on modifie la tension d'un arc orthodontique de l'appareil orthodontique porté et/ou on change un arc orthodontique de l'appareil orthodontique porté et/ou on fabrique une gouttière orthodontique pour remplacer l'appareil orthodontique porté ;
- à l'étape f), on détermine plusieurs traitements potentiels permettant d'atteindre un positionnement des dents correspondant au modèle de référence objectif, on présente lesdits traitements potentiels au patient et/ou à un orthodontiste de manière qu'il(s) choisisse(nt) un desdits traitements potentiels, puis on fabrique ledit appareil orthodontique de substitution correspondant au traitement choisi ;
- à l'étape f'), le patient détermine si le résultat est insatisfaisant ;
- l'appareil orthodontique porté est une première gouttière, et l'appareil orthodontique de substitution est une deuxième gouttière et, de préférence, on envoie ladite deuxième gouttière au patient.

On décrit également un procédé d'évaluation du comportement d'un appareil orthodontique comportant
- une mise en œuvre, à plusieurs reprises, d'un procédé d'adaptation, avec ledit appareil orthodontique et/ou des appareils orthodontiques du même type,
- pour chaque occurrence du procédé d'adaptation, une collecte de données, lesdites données comportant au moins le modèle de référence initial et/ou le modèle de référence objectif, d'une part, et un ou plusieurs modèles de référence actualisés d'autre part, ainsi que des valeurs de paramètres de l'appareil orthodontique porté par le patient pendant ladite occurrence,
- une analyse statistique desdites données de manière à établir une corrélation entre un paramètre du ou des appareils orthodontiques porté(s) pendant lesdites occurrences et le comportement du ou desdits appareils orthodontiques porté(s).

En facilitant les contrôles, le procédé d'adaptation permet la collecte d'un grand nombre de données sur l'action des appareils orthodontiques. Avantageusement, il rend possible une exploitation statistique de ces données.

Le procédé d'évaluation du comportement d'un appareil orthodontique peut encore comporter, notamment, une ou plusieurs des caractéristiques optionnelles suivantes :
- ledit procédé d'adaptation est mis en œuvre plus de 10, de préférence plus de 100, de préférence plus de 1 000, de préférence plus de 10 000, de préférence plus de 100 000 fois, c'est-à-dire pour un grand nombre de patients ;
- après ladite analyse statistique, on utilise les résultats de ladite analyse statistique pour optimiser :
   - la conception d'un appareil orthodontique, et/ou
   - le choix d'un appareil orthodontique pour un traitement particulier, et/ou
   - l'établissement d'un diagnostic, et/ou
   - la fabrication d'un appareil orthodontique de substitution, et/ou
   - la détermination d'un modèle prédictif pour fabriquer un modèle de comparaison.

On décrit encore un procédé de suivi de l'efficacité d'un appareil orthodontique porté par un patient, le procédé comportant les étapes suivantes :
a") Réalisation d'un modèle de référence initial d'au moins une partie d'une arcade, de préférence d'au moins une arcade, de préférence des deux arcades du patient, conformément à l'étape a) ;
b") Acquisition d'au moins une image actualisée, dans des conditions d'acquisition réelles, et recherche, par déformation du modèle de référence initial, d'un modèle de référence actualisé correspondant au positionnement des dents lors de l'acquisition de l'image actualisée, la recherche étant de préférence effectuée au moyen d'une méthode métaheuristique, de préférence évolutionniste, de préférence par recuit simulé, de préférence conformément aux étapes b) à e) ;
c") détermination, pour au moins un point d'une dent, de la valeur d'au moins un paramètre de positionnement dans le modèle de référence actualisé ;
d") répétition du cycle des étapes b") et c") et, à chaque cycle, représentation graphique de ladite valeur dudit paramètre de positionnement de manière à visualiser l'évolution temporelle de ladite valeur.

Un paramètre de positionnement est un paramètre utile pour déterminer la position ou l'orientation d'une dent. Par exemple, *x, y* et z sont des paramètres de positionnement dans un référentiel Oxyz cartésien. La coordonnée radiale, souvent notée *r* ou p, et appelée rayon, la coordonnée angulaire, également appelée angle polaire ou azimut, et souvent notée *t* ou *θ*, et la hauteur, souvent notée *h* ou z, sont des paramètres de positionnement dans un référentiel cylindrique. Les valeurs de ces paramètres de positionnement permettent de définir la position d'un point dans l'espace.

Un procédé de suivi de l'efficacité d'un appareil orthodontique permet de visualiser la dynamique de déplacement d'au moins un point d'une dent. De préférence, ce point est un point remarquable, choisi pour être représentatif de l'intensité de l'action de l'appareil orthodontique.

Le point de la dent est de préférence le barycentre de la dent. Il peut être également, par exemple, le centre de la face vestibulaire de la couronne.

Le paramètre de positionnement est de préférence choisi en fonction de l'action souhaitée pour l'appareil orthodontique.

La représentation graphique, de préférence une courbe, l'échelle du temps étant de préférence linéaire, permet immédiatement de percevoir la dynamique de l'action de l'appareil orthodontique. En particulier, le constat que la courbe croît ou décroît moins vite à mesure que le temps s'écoule, peut être interprété comme signifiant que l'appareil orthodontique perd de son efficacité.

Une telle courbe est particulièrement simple à comprendre, ce qui rend son utilisation possible par le patient lui-même.

Dans un mode de réalisation préféré, la représentation graphique comporte des indications sur le caractère satisfaisant ou insatisfaisant de ladite évolution. Par exemple, la courbe peut changer de couleur si la pente est considérée comme anormale. De préférence, la courbe peut être affichée sur un appareil d'acquisition, de préférence sur un téléphone mobile, ayant servi à ladite acquisition. En particulier, elle peut être visualisable par le patient. Avantageusement, le patient peut ainsi décider de lui-même, au moment le plus opportun, de prendre des mesures pour modifier ou changer son appareil orthodontique, par exemple de prendre rendez-vous chez l'orthodontiste.

On décrit encore un document, électronique ou papier, présentant ladite représentation graphique.

Dans un mode de réalisation, le nombre de paramètres de positionnement dont l'évolution est représentée graphiquement est inférieur à 10, de préférence inférieur à 5, de préférence inférieur à 4, de préférence inférieur à 3, de préférence inférieur à 2. Dans un mode de réalisation, le nombre de points de dent pour lesquels l'évolution d'un ou plusieurs paramètres de positionnement est représentée graphiquement est inférieur à 10, de préférence inférieur à 5, de préférence inférieur à 4, de préférence inférieur à 3, de préférence inférieur à 2. La prise de décision en est facilitée.

La réduction du nombre de paramètres de positionnement dont l'évolution est représentée graphiquement et du nombre de points de dents exige cependant un choix approprié de ces paramètres et de ces points de dent.

On décrit aussi l'utilisation d'un dit procédé de contrôle de la forme de dents pour :
- visualiser et/ou mesurer et/ou détecter une plaque dentaire, et/ou un début de carie, et/ou une microfissure, et/ou une usure, par exemple résultant de bruxisme ou de la mise en œuvre d'un appareil orthodontique, actif ou passif, notamment en cas de rupture ou de détachement d'un arc orthodontique ;
- visualiser et/ou mesurer et/ou détecter un changement de volume, en particulier lors de la croissance des dents ou suite à une intervention d'un dentiste ou d'un orthodontiste, par exemple un dépôt de colle à la surface des dents ;
- évaluer l'opportunité d'un traitement interceptif, avant tout traitement orthodontique, notamment pour évaluer l'intérêt d'un traitement orthodontique.

Dans un mode de réalisation, pour contrôler exclusivement la déformation des dents, on considère, lors de l'étape e), que les modèles de dents sont fixes, c'est-à-dire ne se sont pas déplacés entre les étapes a) et b). En particulier, à l'étape e3), le modèle de référence à tester ne peut être modifié que par déformation d'un ou plusieurs modèles de dents.

La comparaison des formes du modèle de référence initial et du modèle de référence actualisé afin de déterminer la déformation de dents entre les étapes a) et b) peut en particulier résulter d'une comparaison de la forme d'un ou plusieurs modèles de dent dans le modèle de référence initial et dans le modèle de référence actualisé.

On décrit encore un procédé de contrôle d'une propriété d'aspect de dents d'un patient, ledit procédé comportant les étapes successives suivantes :
A. acquisition, au moyen d'un premier appareil d'acquisition, d'au moins une première image bidimensionnelle desdites dents et d'une première jauge de référence, dite « image initiale » ;
B. acquisition, au moyen d'un deuxième appareil d'acquisition, d'au moins une deuxième image bidimensionnelle desdites dents et d'une deuxième jauge de référence présentant un même aspect que la première jauge de référence, dite « image actualisée » ;
C. normalisation des images initiale et actualisée de manière que les représentations des première et deuxième jauges de référence sur les images initiale et actualisée normalisées présentent un même aspect ;
D. avant ou après l'étape C., repérage d'une même région des dents sur les images initiale et actualisée ;
E. comparaison de l'aspect de ladite région sur les images initiale et actualisée normalisées.

Un tel procédé de contrôle d'une propriété d'aspect de dents comporte de préférence une ou plusieurs des caractéristiques optionnelles suivantes :
- l'intervalle de temps entre les étapes A. et B. est supérieur à 1 semaine ;
- le premier appareil d'acquisition et/ou le deuxième appareil d'acquisition sont des appareils photo et/ou des téléphones mobiles ;
- les acquisitions aux étapes A. et/ou B. sont effectuées sous l'éclairage d'un flash ;
- les jauges de référence utilisées pour chacune des étapes A. et B. présentent un même aspect ;
- les jauges de référence utilisées pour chacune des étapes A. et B. sont fixées sur un écarteur dentaire ;
- les acquisitions aux étapes A. et/ou B. sont effectuées au moyen d'un kit d'acquisition décrit ci-après ;
- les jauges de référence utilisées pour chacune des étapes A. et B. sont des marques de repérage ;
- le premier appareil d'acquisition et/ou le deuxième appareil d'acquisition comporte un programme d'ordinateur comprenant des instructions de code de programme pour repérer, en temps réel, la ou les marques de repérage sur l'écarteur, analyser sa ou leurs positions et/ou dimensions, en particulier les positions relatives de plusieurs marques de repérage et, en conséquence, fournir une information, de préférence lumineuse ou sonore, de manière à informer l'utilisateur dudit appareil d'acquisition ;
- à l'étape D., le repérage comprend une comparaison d'informations discriminantes communes aux deux images initiale et actualisée, puis une identification de la position de ladite région par rapport aux dites informations discriminantes communes ;
- à l'étape D., le repérage de la région sur les images initiale et/ou actualisée comprend une recherche de conditions d'acquisition virtuelles dans lesquelles le premier et/ou deuxième appareil d'acquisition, respectivement, aurait acquis ladite image initiale et/ou actualisée, respectivement, en observant un modèle de référence tridimensionnel numérique des arcades du patient.
- à l'étape D., le repérage de la région sur les images initiale et/ou actualisée comprend la mise en œuvre d'un procédé d'évaluation des conditions d'acquisition réelles décrit ci-après.

On décritégalement un kit d'acquisition, notamment pour la mise en œuvre d'une étape b), A. ou B., ledit kit d'acquisition comportant :
- un écarteur dentaire destiné à être placé dans la bouche d'un patient et comportant une marque de repérage ;
- un appareil d'acquisition d'images comportant ;
   - un écran de visualisation,
   - une mémoire informatique contenant des informations sur des conditions d'acquisition cibles,
   - un programme d'ordinateur comprenant des instructions de code de programme pour afficher simultanément, sur ledit écran, une image acquérable et une référence, ladite référence étant dans une position telle que, lorsque la marque de repérage correspond à la référence sur l'écran, l'appareil d'acquisition respecte les conditions d'acquisition cibles.

Un tel kit d'acquisition peut notamment être mis en œuvre à l'étape b) d'un dit procédé de contrôle du positionnement et/ou de la forme des dents, ou aux étapes A. et/ou B. d'un procédé de contrôle d'une propriété d'aspect de dents, et plus généralement pour tout procédé comportant une évaluation des conditions d'acquisition d'une image.

Comme on le verra plus en détail dans la suite de la description, un tel kit d'acquisition permet en particulier de positionner l'appareil d'acquisition dans une position qui correspond sensiblement à une position prédéterminée, par exemple considérée comme optimale pour le contrôle souhaité. Un tel kit d'acquisition permet donc d'améliorer considérablement la rapidité du traitement des informations pour la mise en œuvre desdits procédés de contrôle.

Le kit d'acquisition présente de préférence une ou plusieurs des caractéristiques optionnelles suivantes :
- l'appareil d'acquisition est un téléphone mobile ;
- la référence est choisie dans le groupe constitué par
   - un point,
   - une forme géométrique, de préférence un cercle, un carré, un rectangle ou une ligne,
   - une zone colorée de la même couleur que ladite marque de repérage,
   - une forme identique à la forme de ladite marque de repérage,
   - une forme complémentaire à la forme de ladite marque de repérage, en particulier pour constituer une forme ayant un sens, comme une forme géométrique, une lettre ou un texte, un dessin, ou un motif, et
   - leurs combinaisons ;
- l'écarteur comporte plusieurs marques de repérage non alignées et, de préférence coplanaires ;
- la marque de repérage est disposée de manière que, lorsqu'elle correspond à ladite référence sur l'écran, elle est à moins de 1 cm d'un bord de l'écran.

On décrit aussi un procédé d'acquisition d'une image bidimensionnelle d'une partie d'une arcade dentaire, ou d'une arcade dentaire ou des deux arcades dentaires d'un patient au moyen d'un dit kit d'acquisition, notamment pour la mise en œuvre d'une étape b), A. et/ou B.. Ce procédé est remarquable en ce qu'il comporte les étapes successives suivantes :
(a) détermination de conditions d'acquisition cibles, par exemple en fonction d'un traitement à appliquer, en particulier d'un traitement orthodontique, et détermination de conditions de correspondance adaptées pour qu'une mise en correspondance d'une marque de repérage de l'écarteur avec une référence affichée sur l'écran de l'appareil d'acquisition résulte dans l'application des conditions d'acquisition cibles;
(b) programmation de l'appareil d'acquisition de manière à afficher la référence dans une position telle que ladite mise en correspondance résulte en l'application des conditions d'acquisition cibles ;
(c) mise en place de l'écarteur dans la bouche du patient ;
(d) positionnement de l'appareil d'acquisition de manière à faire correspondre la marque de repérage et la référence et ainsi appliquer les conditions d'acquisition cibles ;
(e) acquisition de l'image acquérable dans le positionnement de l'appareil d'acquisition adopté à l'étape précédente.

Les conditions d'acquisition cibles sont les conditions permettant un positionnement adapté de l'appareil d'acquisition, de préférence un positionnement optimal de l'appareil d'acquisition pour acquérir l'image. De préférence, les conditions d'acquisition cibles sont donc déterminées en fonction des dents à observer.

De préférence le procédé d'acquisition est mis en œuvre pour les étapes desdits procédés de contrôle nécessitant l'acquisition d'une image bidimensionnelle d'une partie d'une arcade ou d'une arcade dentaire ou des deux arcades dentaires d'un patient, en en particulier pour les étapes b).

De préférence, le cycle des étapes (a) à (e) est répété plusieurs fois, de préférence plus de deux fois, voire plus de trois fois, avec des conditions d'acquisition cibles différentes.

Par exemple, pour mesurer le déplacement d'une dent, des premières conditions d'acquisition cibles pourront correspondre à un positionnement à 40 cm de l'écarteur, en face et à hauteur de l'écarteur. Des deuxièmes et troisièmes conditions d'acquisition cibles pourront correspondre à un positionnement à 40 cm de l'écarteur, à hauteur de l'écarteur, à 45° à droite et à gauche du plan sagital, respectivement.

De préférence, les étapes (c), (d) et (e) sont exécutées par une personne sans formation universitaire à l'orthodontie et/ou en dehors de tout cabinet médical, de dentisterie ou d'orthodontie, et/ou sans recours à un dispositif de stabilisation mécanique de l'appareil d'acquisition et/ou sans recours à d'autres appareils qu'un téléphone mobile et/ou sans recours à une jauge étalon de calibration.

On décrit aussi un procédé d'optimisation d'une information discriminante extraite d'une image bidimensionnelle des arcades dentaires d'un patient, dite « image acquise », au moyen d'un modèle de référence tridimensionnel numérique d'au moins une partie d'une arcade du patient, ou d'une arcade ou des deux arcades du patient ledit procédé comportant les étapes suivantes :
C1. évaluation de la qualité de l'information discriminante et d'un seuil de qualité, filtrage de manière à ne retenir que de l'information discriminante présentant une qualité supérieure au seuil de qualité, et définition de « l'information discriminante à tester » comme étant l'information discriminante retenue ;
C2. test d'une concordance entre l'information discriminante à tester et ledit modèle de référence ;
C3. évaluation du résultat du test, et en fonction de ladite évaluation :
   - ajout d'information discriminante non retenue à l'information discriminante à tester et/ou suppression d'information discriminante dans l'information discriminante à tester, puis reprise à l'étape C2. ou,
   - définition de l'information discriminante optimale comme étant l'information discriminante à tester.

De préférence, le procédé d'optimisation comporte encore une ou plusieurs des caractéristiques optionnelles suivantes :
- l'information discriminante est choisie dans le groupe constitué par une information de contour, une information de couleur, une information de densité, une information de distance, une information de brillance, une information de saturation, une information sur les reflets, et les combinaisons de ces informations ;
- l'étape C2. comporte les étapes suivantes :
   - recherche de conditions d'acquisition virtuelles approximant, de préférence de manière optimale, des conditions d'acquisition réelles dans lesquelles ladite image acquise a été acquise et observation du modèle de référence dans lesdites conditions d'acquisitions virtuelles de manière à obtenir une image de référence ;
   - traitement de l'image acquise et de l'image de référence pour réaliser au moins une carte acquise et une carte de référence, respectivement, lesdites cartes acquise et de référence représentant de ladite information discriminante ;
   - comparaison des cartes acquise et de référence de manière à déterminer un degré de concordance, le résultat du test de l'étape C2. dépendant, de préférence étant égal audit degré de concordance ;
- la recherche des conditions d'acquisition virtuelles approximant des conditions d'acquisition réelles comporte les étapes suivantes :
   01) optionnellement, détermination de conditions d'acquisition virtuelles grossières approximant lesdites conditions d'acquisition réelles, de préférence par analyse de la représentation, sur l'image acquise, d'un écarteur utilisé lors de l'acquisition de l'image acquise;
   02) détermination de conditions d'acquisition virtuelles à tester;
   03) réalisation d'une image de référence bidimensionnelle du modèle de référence observé dans les conditions d'acquisition virtuelles à tester ;
   04) traitement de l'image de référence pour réaliser au moins une carte de référence représentant ladite information discriminante ;
   05) comparaison des cartes acquise et de référence de manière à déterminer une valeur pour une fonction d'évaluation, ladite valeur pour la fonction d'évaluation dépendant des différences entre lesdites cartes acquise et de référence et correspondant à une décision de poursuivre ou de stopper la recherche de conditions d'acquisition virtuelles approximant les conditions d'acquisition réelles avec plus d'exactitude que lesdites conditions d'acquisition virtuelles à tester ;
      si ladite valeur pour la fonction d'évaluation correspond à une décision de poursuivre ladite recherche, modification desdites conditions d'acquisition virtuelles à tester, puis reprise à l'étape 03) ;
      sinon, évaluation des conditions d'acquisition réelles par lesdites conditions d'acquisition virtuelles à tester ;
- la détermination de l'information discriminante à ajouter et/ou à ajouter à l'étape C3. résulte de la mise en œuvre d'une méthode métaheuristique, de préférence évolutionniste.

On décrit aussi un procédé d'évaluation des conditions d'acquisition réelles d'une image bidimensionnelle des dents d'un patient, dite « image acquise », ledit procédé comportant les étapes suivantes :
001) réalisation d'un modèle de référence tridimensionnel numérique d'au moins une partie d'une arcade, de préférence d'une arcade, de préférence des deux arcades du patient ;
002) analyse de l'image acquise et réalisation d'une carte relative à une information discriminante, dite « carte acquise » ;
003) recherche des conditions d'acquisition virtuelles approximant lesdites conditions d'acquisition réelles, de préférence suivant les étapes 01) à 05).

Une ou plusieurs des caractéristiques, éventuellement optionnelles, de l'étape a) sont applicables à l'étape 001). En particulier, le modèle de référence peut être préparé, par un scan, à partir de mesures effectuées sur les dents du patient ou sur un modèle physique de ses dents, par exemple un modèle en plâtre.

Une ou plusieurs des caractéristiques, éventuellement optionnelles, de l'étape c) sont applicables à l'étape 002).

On décrit également :
- un programme d'ordinateur, et en particulier un applicatif spécialisé pour téléphone mobile, comprenant des instructions de code de programme pour l'exécution d'une ou plusieurs, de préférence toutes les étapes b) à f) ou A. à E. ou C1 à C3, lorsque ledit programme est exécuté par un ordinateur,
- un support informatique sur lequel est enregistré un tel programme, par exemple une mémoire ou un CD-ROM, et
- un appareil personnel, en particulier un téléphone mobile ou une tablette, dans lequel est chargé un tel programme.

On décrit aussi un système comportant :
- un scanner tridimensionnel apte à mettre en œuvre l'étape a) d'un dit procédé de contrôle du positionnement et/ou de la forme de dents, ou une étape 001).
- un appareil personnel, de préférence un téléphone mobile, chargé avec un dit programme.

### Définitions

Par « patient », on entend toute personne pour laquelle un procédé est mis en œuvre afin d'en contrôler les dents, que cette personne soit malade ou non, ou que cette personne soit en cours de traitement ou non.

Par « professionnel des soins dentaires », on entend un dentiste, un orthodontiste ou un laboratoire d'orthodontie.

Par « dentiste », on entend un dentiste ou un assistant dentaire travaillant sous la responsabilité d'un dentiste.

Par « dentition », on entend les deux arcades dentaires du patient.

Une image d'une arcade est bien entendu une représentation partielle de cette arcade.

On appelle "téléphone mobile" un appareil de moins de 500 g, doté d'un capteur lui permettant de capturer des images, capable d'échanger des données avec un autre appareil éloigné de plus de 500 km du téléphone mobile, et capable d'afficher lesdites données, et notamment lesdites images.

Pour un procédé de contrôle du positionnement des dents, les « conditions d'acquisition » précisent la position et l'orientation dans l'espace d'un appareil d'acquisition d'images relativement aux dents du patient ou à un modèle de dents du patient, et de préférence la calibration de cet appareil d'acquisition d'images.

La « calibration » d'un appareil d'acquisition est constituée par l'ensemble des valeurs des paramètres de calibration. Un paramètre de calibration est un paramètre intrinsèque à l'appareil d'acquisition (à la différence de sa position et de son orientation) dont la valeur influence l'image acquise. Par exemple, l'ouverture de diaphragme est un paramètre de calibration qui modifie la profondeur de champ. Le temps d'exposition est un paramètre de calibration qui modifie la luminosité (ou « l'exposition ») de l'image. La distance focale est un paramètre de calibration qui modifie l'angle de vue, c'est-à-dire le degré de « zoom ». La « sensibilité » est un paramètre de calibration qui modifie la réaction du capteur d'un appareil d'acquisition numérique à la lumière incidente.

De préférence, les paramètres de calibration sont choisis dans le groupe formé par l'ouverture de diaphragme, le temps d'exposition, la distance focale et la sensibilité.

Le "plan d'occlusion" est le plan qui fournit la meilleure corrélation linéaire avec l'ensemble des points de contact entre les dents de l'arcade supérieure d'une part et les dents de l'arcade inférieure d'autre part.

Le "plan longitudinal médian" est le plan sensiblement vertical lorsque le patient tient la tête droite, qui sépare de manière sensiblement symétrique des parties droite et gauche de chaque arcade.

Une « tablette » est un ordinateur portable à écran tactile.

Un scanner 3D est un appareil permettant d'obtenir une représentation en trois dimensions d'un objet.

Par "image", on entend une image en deux dimensions, comme une photographie. Une image est formée de pixels.

Une image « acquérable » ("preview") est l'image que l'appareil d'acquisition peut enregistrer à un instant donné. Pour un appareil photo ou un téléphone, c'est l'image qui apparaît sur l'écran lorsque l'applicatif d'acquisition de photo ou de vidéo est en fonctionnement.

Une "information discriminante" est une information caractéristique qui peut être extraite d'une image (*"image feature"*), classiquement par un traitement informatique de cette image.

Une information discriminante peut présenter un nombre variable de valeurs. Par exemple, une information de contour peut être égale à 1 ou 0 selon qu'un pixel appartient ou non à un contour. Une information de brillance peut prendre un grand nombre de valeurs. Le traitement de l'image permet d'extraire et de quantifier l'information discriminante.

Des conditions d'acquisition sont dites "virtuelles" lorsqu'elles correspondent à une simulation dans laquelle l'appareil d'acquisition serait dans lesdites conditions d'acquisition (positionnement et de préférence calibration théoriques de l'appareil d'acquisition).

Par "comprenant un" ou "comportant un" ou "présentant un", on entend "comportant au moins un", sauf indication contraire.

Dans les différents procédés décrits, les références des étapes sont identiques si ces étapes sont similaires ou identiques.

### Brève description des figures

D'autres caractéristiques et avantages de l'invention apparaîtront encore à la lecture de la description détaillée qui va suivre et à l'examen du dessin annexé dans lequel :

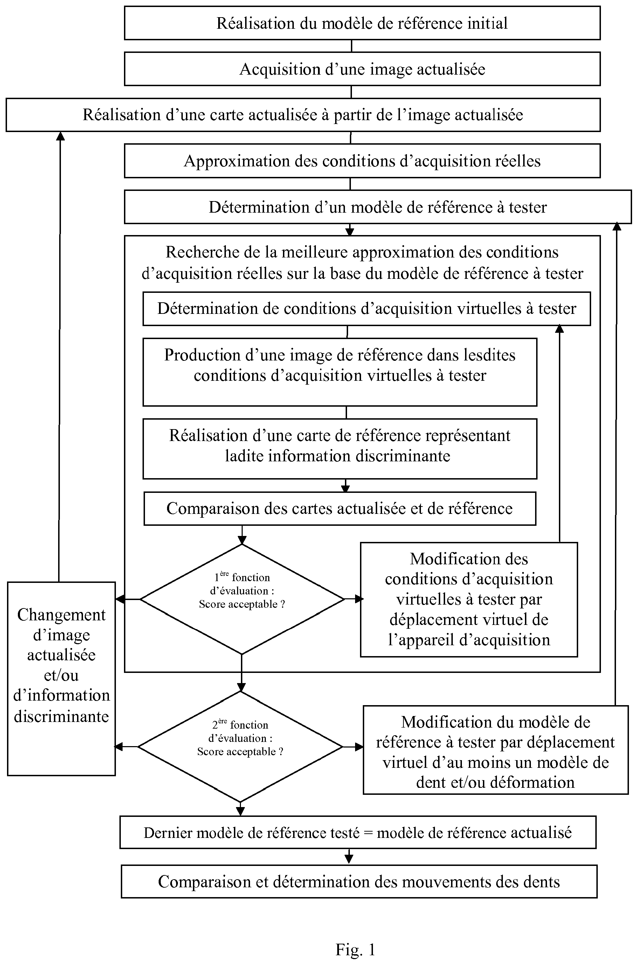

- la figure 1 représente un logigramme illustrant la mise en œuvre d'un dit procédé de contrôle du positionnement et/ou de la forme de dents s,
- la figure 2 représente un exemple de modèle de référence initial,
- la figure 3 illustre le traitement effectué pour déterminer le plan d'occlusion,
- la figure 4 (4a-4d) illustre l'étape nécessaire pour déterminer les modèles de dent dans un modèle de référence,
- la figure 5 (5a-5d) illustre l'acquisition d'images actualisées, ainsi que l'opération de découpage,
- la figure 6 (6a-6b) illustre le traitement d'une image actualisée permettant de déterminer le contour des dents,
- la figure 7 illustre schématiquement la position relative de marques de repérage 12 sur des images actualisées 14i et 142 d'un écarteur 10, selon la direction d'observation (trait interrompu),
- la figure 8 représente une cartographie résultant de la mise en œuvre d'un dit procédé de contrôle de la forme de dents d'un patient,
- la figure 9 représente un logigramme illustrant la mise en œuvre d'un dit procédé de contrôle de l'aspect de dents,
- la figure 10 représente un logigramme illustrant la mise en œuvre d'un dit procédé de d'optimisation d'une information discriminante, et
- la figure 11 représente, sur une image acquise, une carte de référence établie pour une information de reflet,
- les figures 12a et 12b illustrent deux vues d'un modèle tridimensionnel avec deux distances focales différentes,
- la figure 13 représente un exemple d'évolutions réelle et théorique de la valeur d'un paramètre de quantité de mouvement, en fonction du temps. L'axe des abscisses fournit le temps, en semaines. L'axe des ordonnées fournit la valeur du paramètre, en pourcentage par rapport à la valeur souhaitée en fin de traitement,
- la figure 14 représente un logigramme illustrant la mise en œuvre d'un dit procédé d'adaptation d'un appareil orthodontique.

### Description détaillée d'un procédé de contrôle du positionnement des dents

Un dit procédé de contrôle du positionnement de dents comporte les étapes mentionnées ci-dessus.

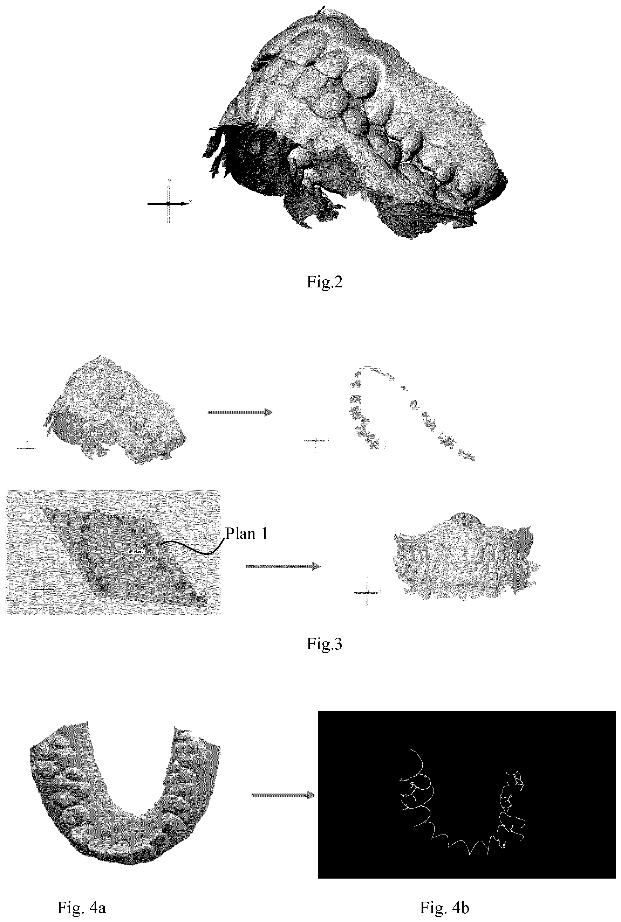

**A l'étape** a), un modèle de référence initial des arcades, ou d'une partie des arcades du patient est créé (voir figure 2).

Le modèle de référence initial est un modèle numérique en trois dimensions des arcades du patient, par exemple du type .stl ou .Obj, .DXF 3D, IGES, STEP, VDA, ou Nuages de points. Avantageusement, un tel modèle, dit « 3D », peut être observé selon un angle quelconque.

Pour le suivi d'un traitement orthodontique, le modèle de référence initial est de préférence préparé au début du traitement. Le modèle de référence initial peut correspondre à un positionnement des dents du patient avant le traitement ou à un positionnement des dents du patient que le traitement se propose d'atteindre. Dans ce cas, le modèle de référence initial est classiquement calculé à partir d'un premier modèle tridimensionnel correspondant au positionnement des dents du patient avant le traitement.

Pour le contrôle de la récidive, le modèle de référence initial est de préférence préparé moins de six mois, de préférence moins de trois mois, de préférence encore moins d'un mois après la fin du traitement orthodontique, généralement immédiatement après la fin du traitement. Il correspond ainsi à un positionnement sensiblement optimal des dents.

Le modèle de référence initial peut être également préparé indépendamment de tout traitement, par exemple parce que le patient souhaite surveiller les mouvements de ses dents.

Le modèle de référence initial peut être préparé à partir de mesures effectuées sur les dents du patient ou sur un modèle physique de ses dents, par exemple un modèle en plâtre.

Le modèle de référence initial est de préférence créé au moyen d'un appareil professionnel, par exemple au moyen d'un scanner 3D, de préférence mis en œuvre par un professionnel de la santé, par exemple par un orthodontiste ou un laboratoire d'orthodontie. Dans un cabinet d'orthodontie, le patient ou le modèle physique de ses dents peuvent être avantageusement disposés dans une position précise et l'appareil professionnel peut être perfectionné. Il en résulte un modèle de référence initial très précis. Le modèle de référence initial fournit de préférence une information sur le positionnement des dents avec une erreur inférieure à 5/10 mm, de préférence inférieure à 3/10 mm, de préférence inférieure à 1/10 mm.

### Orientation du modèle de référence initial :

De préférence, on détermine l'orientation du modèle de référence initial dans l'espace, et notamment, de préférence, le plan d'occlusion et le plan longitudinal médian.

Le plan d'occlusion et le plan longitudinal médian peuvent être déterminés manuellement, de manière approximative. Les inventeurs ont cependant découvert des procédés permettant de déterminer ces plans par traitement informatique.

De préférence, le modèle de référence est un modèle des arcades bouche fermée, c'est-à-dire dans une position dans laquelle des dents de l'arcade supérieure sont en contact avec des dents de l'arcade inférieure.

Classiquement, le modèle de référence initial fourni par un scanner tridimensionnel permet de distinguer l'arcade supérieure de l'arcade inférieure. Généralement, le modèle est fourni sous la forme de deux fichiers correspondant respectivement à ces arcades, et comportant des données permettant de positionner les modèles de ces arcades l'un par rapport à l'autre dans la position d'occlusion.

De préférence, pour estimer les points de contact entre les dents des arcades supérieure et inférieure, on détermine l'ensemble des points du modèle de l'arcade supérieure et de l'arcade inférieure qui sont à une distance inférieure à une limite prédéterminée, cette limite étant de préférence inférieure à 3 mm, de préférence d'environ 2 mm. Tous les autres points de ces modèles sont ensuite ignorés, ce qui conduit à la représentation de la figure 3b. Une régression linéaire permet alors de déterminer le plan d'occlusion (« plan 1 » sur la figure 3c).

Le modèle de référence initial peut ainsi être orienté suivant le plan d'occlusion (Fig. 3d).

Si le modèle de référence initial ne comporte pas de données permettant de positionner les arcades supérieure et inférieure l'une par rapport à l'autre, on utilise de préférence un mordu d'occlusion faisant apparaître les points de contact entre les dents supérieures et les dents inférieures, puis on repositionne les modèles des arcades supérieure et inférieure par rapport à ce mordu d'occlusion.

Le plan longitudinal médian est perpendiculaire au plan d'occlusion, mais son orientation n'est pas connue.

De préférence, on procède de la manière suivante pour déterminer l'orientation du plan longitudinal médian :
On considère des axes [Ox) et [Oy) dans le plan d'occlusion, le point O étant le barycentre des projections normales des points de contact sur le plan d'occlusion.
- Dans ce repère (xOy), on recherche la courbe, de préférence parabolique, présentant le plus grand coefficient de corrélation avec l'ensemble desdites projections.
- L'ensemble des projections des points de contact est ensuite déplacé dans le plan d'occlusion, par rotation autour du point O, et on recommence l'étape précédente à partir de cette nouvelle position angulaire des projections des points de contact.

Le cycle des opérations précédentes est poursuivi, de préférence jusqu'à avoir fait tourner l'ensemble des points de contact de 360° autour du barycentre O. On compare ensuite les coefficients de corrélation correspondant aux différentes orientations de l'ensemble des points de contact. L'axe de la courbe qui conduit au coefficient de corrélation le plus élevé est alors considéré comme inclus dans le plan longitudinal médian, ce qui permet de définir exactement l'orientation de ce dernier.

L'orientation dans l'espace du modèle de référence initial est ainsi parfaitement déterminée, de manière rapide.

### Création des modèles de dent

Dans le modèle de référence initial, une partie qui correspond à une dent, ou « modèle de dent », est délimitée par un bord gingival qui peut être décomposé en un bord gingival intérieur (du côté de l'intérieur de la bouche par rapport à la dent), un bord gingival extérieur (orienté vers l'extérieur de la bouche par rapport à la dent) et deux bords gingivaux latéraux. Les bords gingivaux correspondent à des régions dans lesquelles l'orientation de la surface définie par le modèle de référence initial subit des modifications de fortes amplitudes. Ces variations d'orientation peuvent être identifiées selon des techniques connues, par exemple en identifiant les changements de direction de la normale à la surface modélisée. La figure 4a représente une vue du modèle de référence initial traitée pour faire apparaître ces changements de direction. La figure 4b montre le bord gingival intérieur qui peut être extrait par l'analyse de l'image de la figure 4a.

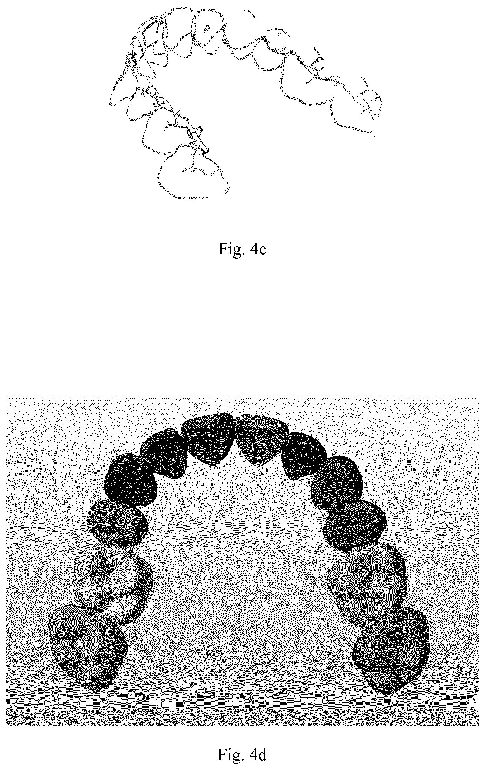

Plusieurs vues du modèle de référence initial sont ainsi analysées, ce qui permet de déterminer les bords gingivaux intérieur et extérieur en trois dimensions, comme représenté sur la figure 4c.

Par ailleurs, en projection dans le plan d'occlusion, les contours gingivaux intérieur et extérieur d'une arcade se rapprochent de part et d'autre d'une dent. Pour déterminer un bord gingival latéral d'une dent, on recherche le plus court chemin, sur la surface du modèle de référence initial, entre les deux points des bords gingivaux intérieur et extérieur ainsi rapprochés et qui se font sensiblement face. La recherche du plus court chemin entre deux points sur un modèle tridimensionnel fait appel à des techniques d'optimisation bien connues. De préférence, cette recherche résulte d'une méthode métaheuristique, de préférence évolutionniste, de préférence d'un recuit simulé.

Deux bords gingivaux latéraux adjacents et les parties des bords gingivaux intérieur et extérieur qui connectent ces bords gingivaux latéraux permettent ainsi de délimiter une dent au niveau de la gencive. En tenant compte du fait qu'une dent s'étend depuis le contour gingival vers le plan d'occlusion, il est ainsi possible de déterminer les parties du modèle de référence initial qui correspondent aux différentes dents (« modèles de dent »).

La figure 4d représente l'ensemble des modèles de dent d'une arcade.

Le modèle de référence initial peut être stocké dans une base de données centralisée, regroupant les modèles de référence initiaux d'une pluralité de patients. Cette base de données peut être physiquement installée dans un établissement spécialisé. Elle peut être également installée dans un laboratoire ou un cabinet d'orthodontie, ce qui limite les transferts d'informations confidentielles.

Dans un mode de réalisation, le modèle de référence initial est remis au patient. De préférence, un fichier informatique correspondant au modèle de référence initial est enregistré sur un support amovible, par exemple sur une clé USB ou sur une carte électronique, de préférence sur un téléphone mobile, une tablette ou un ordinateur portable du patient, et en particulier sur l'appareil personnel qui sera de préférence utilisé aux étapes b) et suivantes. De préférence, le patient ou un professionnel des soins dentaires charge le modèle de référence initial dans ledit appareil individuel ou le met à disposition pour chargement dans ledit appareil individuel. Le patient charge de préférence le modèle de référence initial à partir d'internet.

Dans un mode de réalisation préféré, le modèle de référence n'est pas remis au patient. De préférence, le modèle de référence est seulement rendu disponible à un établissement spécialisé pour mettre en œuvre les étapes c) à f). Il peut rester stocké dans l'établissement dans lequel il a été réalisé à l'étape a) et où, de préférence, les étapes c) à f) sont mises en œuvre.

**A l'étape** b), on prend une image actualisée d'une partie d'une arcade, d'une arcade ou des arcades au moyen d'un appareil d'acquisition d'images. L'étape b) est de préférence réalisée par le patient ou un proche du patient, mais peut être réalisée par un dentiste.

### Moment de l'acquisition

De préférence, l'image actualisée est prise après un intervalle de temps Δt après l'étape a). L'intervalle de temps Δt peut être prédéterminé. Il peut être constant, quelle que soit l'occurrence du procédé, c'est-à-dire que cet intervalle concerne la première exécution du procédé ou une exécution ultérieure. Il peut être variable, et dépendre par exemple des résultats obtenus suite à une exécution antérieure du procédé. En particulier, pour le contrôle de la récidive, l'intervalle de temps Δt peut être d'autant plus court que cette exécution a permis de détecter une dérive importante.

Dans un mode de réalisation préféré, l'intervalle de temps Δt est déterminé par l'orthodontiste, en fonction d'un planning des contrôles. En fonction de l'évolution de la position des dents, l'orthodontiste peut modifier ce planning et modifier en conséquence l'intervalle de temps Δt. Dans un mode de réalisation, le procédé de contrôle du positionnement de dents est exécuté plusieurs fois, les intervalles de temps entre chaque exécution pouvant être identiques ou différents. Les intervalles des temps entre deux exécutions successives peuvent être tous déterminés avant la première exécution pour correspondre à un planning de contrôles élaboré par l'orthodontiste.

L'intervalle de temps Δt peut être également indéterminé et dépendre par exemple de décisions du patient. Par exemple, la création d'une image actualisée peut être effectuée à l'occasion d'un rendez-vous chez le dentiste ou à tout moment lorsque le patient le souhaite, voire indépendamment de tout traitement orthodontique.

L'intervalle de temps Δt est de préférence déterminé pour correspondre à une évolution potentiellement significative du positionnement des dents.

Par exemple, pour le contrôle de la récidive, l'intervalle de temps Δt est de préférence inférieur à trois mois lors de la première année après le traitement. Après cette première année, l'intervalle de temps Δt est de préférence supérieur à un mois, voire supérieur à six mois ou supérieur à douze mois. En particulier pour la détection d'une dérive des dents, un intervalle de temps compris entre six mois et dix-huit mois est adapté.

De préférence, au moins un rappel informant le patient de la nécessité de créer une image actualisée est adressé au patient. Ce rappel peut être sous forme papier ou, de préférence, sous forme électronique, par exemple sous la forme d'un courriel, d'une alerte automatique de l'applicatif spécialisé mobile ou d'un SMS. Un tel rappel peut être envoyé par le cabinet ou le laboratoire d'orthodontie ou par le dentiste ou par l'applicatif spécialisé mobile du patient, par exemple.

Dans un mode de réalisation préféré, une image actualisée est acquise avant que les dents n'aient pu se déplacer significativement, sensiblement au même moment que la création du modèle de référence initial, de préférence moins de 7 jours, moins de 3 jours, moins de 1 jour après l'étape a), c'est-à-dire avant que les dents n'aient pu se déplacer significativement. La mise en œuvre du procédé avec cette image actualisée permet avantageusement de vérifier que le procédé ne conduit à la détection d'aucune différence entre les modèles de référence initial et actualisé, et donc fonctionne correctement.

Dans un mode de réalisation, l'image actualisée peut être acquise avant la création du modèle de référence initial. Par exemple, les étapes a) et b) peuvent être effectuées à la fin et au début d'un traitement orthodontique, respectivement. Il est ainsi notamment possible d'évaluer l'efficacité du traitement en l'absence de modèle 3D en début de traitement. L'intervalle de temps Δt' séparant les étapes a) et b) dans ce mode de réalisation peut notamment prendre les valeurs décrites ci-dessus pour Δt.

### Appareil d'acquisition d'images

De préférence, l'appareil d'acquisition d'images est un appareil personnel couramment disponible dans le commerce, par exemple un téléphone mobile, un appareil photo dit « connecté », une montre dite « intelligente », ou « smartwatch », ou une tablette ou un ordinateur personnel, fixe ou portable, comportant un système d'acquisition d'images, comme une webcam ou un appareil photo, de préférence un appareil photo numérique. Même si l'image actualisée peut en particulier être créée par un dentiste, elle l'est de préférence par le patient lui-même ou par un de ses proches.

En particulier, l'image actualisée peut être créée par une personne qui n'a pas de connaissances particulières en orthodontie, et en particulier qui ne possède aucun diplôme d'orthodontie ou de dentisterie.

De préférence, le même appareil d'acquisition est utilisé pour prendre toutes les images actualisées.

L'appareil d'acquisition d'images pèse de préférence moins de 3 kg, moins de 2 kg, moins de 1 kg, moins de 500 g, de préférence moins de 300 g.

L'étape b) peut donc avantageusement être réalisée à distance de l'étape a), c'est-à-dire dans un lieu différent de celui dans lequel est réalisée l'étape a), en particulier à plus de 50 m, plus de 100 m, plus de 1 km du lieu où est réalisée l'étape a), en particulier hors cabinet orthodontique. Dans un mode de réalisation, l'étape b) n'est pas réalisée dans un cabinet dentaire, un cabinet d'orthodontie ou un laboratoire d'orthodontie, sauf, éventuellement, lors d'une séance destinée à former le patient.

De préférence, l'image actualisée est une photographie, en particulier une photographie panoramique. Dans un mode de réalisation, l'image actualisée est extraite d'un film.

Dans un mode de réalisation préféré, le procédé utilise plusieurs images actualisées pour disposer d'au moins une représentation de chaque dent, de préférence au moins trois images actualisées correspondant à une vue de face, une vue à droite et une vue à gauche des dents du patient.

De préférence, à l'étape b), on prend au moins une image actualisée en position bouche fermée et au moins une image actualisée en position bouche ouverte. L'image bouche fermée permet avantageusement d'identifier les déplacements relatifs entre les deux arcades. L'image actualisée bouche ouverte permet avantageusement de bien identifier les contours des dents, sans que les dents de l'arcade supérieure ne viennent masquer les dents de l'arcade inférieure ou réciproquement.

Des images actualisées peuvent être prises soit pour l'arcade supérieure, soit pour l'arcade inférieure, soit, de préférence, pour les deux arcades, en tout ou partie.

Plusieurs images similaires (représentant sensiblement les mêmes dents) peuvent être également utiles afin de rechercher le meilleur score. En fonction des conditions d'acquisition, une information discriminante pourra en particulier conduire à des scores différents selon l'image actualisée utilisée.

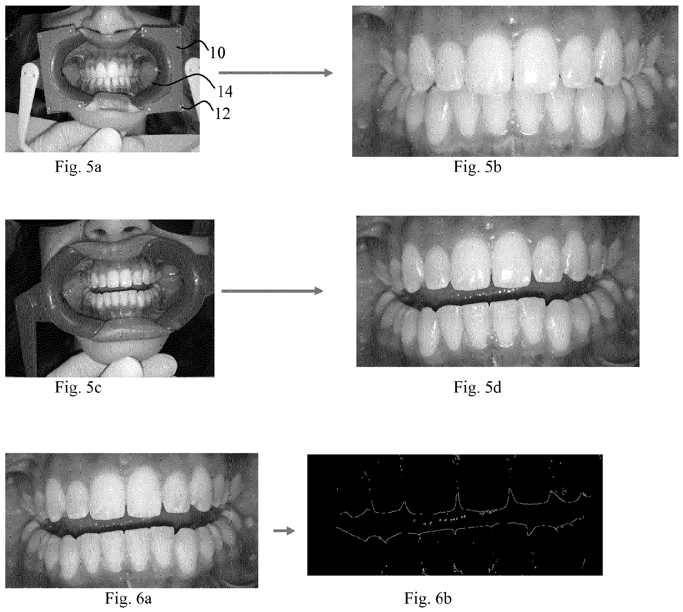

De préférence, on utilise un écarteur dentaire lors de l'étape b), comme représenté sur les figures 5a et 5c. La première fonction de l'écarteur est d'écarter les lèvres afin d'améliorer la visibilité des dents. De préférence, un écarteur est remis au patient, par exemple lors d'un rendez-vous avec son orthodontiste ou son dentiste.

L'appareil d'acquisition d'images fournit de préférence des images en couleurs, et/ou des images infrarouges de la bouche du patient, voire du visage du patient. Les images en couleurs représentent de préférence la bouche du patient avec les couleurs réelles de cette bouche. Les images infrarouges permettent avantageusement de faire apparaître les dents avec un excellent contraste.

De préférence, l'appareil d'acquisition d'images comporte un applicatif spécialisé permettant de mettre en œuvre l'étape b), mais aussi, de préférence, des étapes suivantes, de préférence l'ensemble des étapes suivantes. De préférence encore, cet applicatif gère des rappels et informe le patient de la nécessité de créer une image actualisée.

De préférence, l'applicatif spécialisé est chargé dans l'appareil d'acquisition d'images à partir d'un support physique comme une clé USB ou un CD-ROM, ou est téléchargé sur internet ou par voie hertzienne. Dans un mode de réalisation, l'applicatif spécialisé est fourni au patient par le cabinet et/ou le laboratoire d'orthodontie. Il peut en particulier prendre la forme d'un applicatif du type de ceux couramment téléchargés sur les iPhones de la marque Apple^{®} ou les appareils de toutes marques mettant en œuvre les systèmes d'exploitation Android^{®} ou tout autre système d'exploitation.

L'appareil d'acquisition d'images comporte de préférence un appareil photo ou une caméra vidéo ou infrarouge, que l'utilisateur, par exemple le patient ou un de ses proches, positionne au moyen d'un viseur ou d'un écran, avant de l'actionner.

### Moyens détrompeurs et kit d'acquisition

Un procédé de contrôle du positionnement de dents ne requiert pas un positionnement précis de l'appareil d'acquisition d'images par rapport aux dents.

Dans un mode de réalisation, aucune contrainte de positionnement permettant d'assurer une disposition de l'appareil d'acquisition d'images à moins de 30 cm, moins de 20 cm, de 10 cm ou moins de 5 cm d'un emplacement déterminé n'est imposée.

De préférence, l'appareil d'acquisition d'images comporte cependant des moyens détrompeurs facilitant son positionnement approximatif par rapport au patient avant l'acquisition de l'image actualisée.

L'utilisateur peut être guidé par des messages écrits et/ou vocaux pour l'acquisition. Par exemple, l'appareil personnel peut annoncer « prenez une photo de face », émettre un signal pour informer l'utilisateur que la photo est acceptable ou qu'au contraire, il doit refaire une photo, annoncer « prenez une photo de droite », de préférence en affichant une flèche pour orienter l'utilisateur, etc. La fin du processus d'acquisition peut être également annoncée par l'appareil. L'appareil peut également aider au positionnement, par exemple par des messages visuels (par exemple par affichage de flèches), et/ou sonores (comme une succession de bips dont la fréquence augmente à mesure que le positionnement de l'appareil s'améliore), et/ou écrits et/ou vocaux (« plus haut », plus bas », etc.).

Les moyens détrompeurs peuvent en particulier comporter des références qui apparaissent sur le viseur ou l'écran. Les références peuvent par exemple comporter une ligne destinée à être alignée avec la direction générale de la jointure entre les dents supérieures et les dents inférieures lorsque les dents sont serrées par le patient, et/ou une ligne verticale destinée à être alignée avec la jointure entre les deux incisives supérieures. Les références peuvent également faire référence à d'autres parties du patient. Par exemple, elles peuvent être constituées par des marques correspondant à la position des yeux ou prendre la forme d'un contour dans lequel doit être positionné la bouche ou le visage du patient.

La ou les références sont de préférence "immobiles" sur l'écran, c'est-à-dire qu'elles ne se déplacent pas sur l'écran lorsque l'appareil d'acquisition est en mouvement.

Dans un mode de réalisation préféré, la ou les références correspondent chacune à une marque de repérage portée par un référentiel rapporté sur le patient, c'est-à-dire que le patient ne présentait pas avant la mise en œuvre du procédé, de préférence portée par un écarteur dentaire. Un référentiel peut être également une pièce mordue par le patient.

La marque de repérage peut présenter une surface supérieure à 0,5 mm², de préférence supérieure à 1 mm², de préférence supérieure à 2 mm², de préférence supérieure à 5 mm², de préférence supérieure à 10 mm², voire supérieure à 20 mm², voire supérieure à 30 mm², et/ou inférieure à 50 mm².

De grandes dimensions conférées à une marque de repérage ou une multiplication des marques de repérage permettent avantageusement d'améliorer la précision du positionnement de l'appareil d'acquisition.

Les marques de repérage peuvent être identiques ou différentes.

Les marques de repérage peuvent être notamment différentes selon leur position, par exemple selon qu'elles sont en partie supérieure ou en partie inférieure du référentiel, et en particulier de l'écarteur, ou à droite ou à gauche du référentiel, et en particulier de l'écarteur.

La marque de repérage peut être identique ou différente de la référence correspondante. Elle est de préférence de forme géométrique, par exemple un point, un ou plusieurs traits, par exemple parallèles, une étoile, un cercle, un oval, un polygone régulier, notamment un carré, un rectangle ou un losange.

La marque de repérage peut être également une image, une lettre, un chiffre ou une séquence de lettres et/ou de chiffres.

La marque de repérage est de préférence d'une couleur différente de la surface de l'écarteur qui l'environne, de préférence de manière à offrir un contraste élevé.

Une marque de repérage peut être visible ou invisible à l'œil nu, pourvu qu'elle apparaisse sur l'écran de l'appareil d'acquisition.

Pour améliorer la précision, les marques de repérage sont de préférence écartées les unes des autres de manière que, lorsqu'elles correspondent à leurs références respectives sur l'écran, au moins des première et deuxième marques de repérage soient à moins de 3 cm, de préférence moins de 2 cm, de préférence moins de 1 cm, de préférence moins de 0,5 cm, de premier et deuxième bords, respectivement, de l'écran. Les premier et deuxième bords sont de préférence des bords opposés de l'écran.

La marque de repérage peut présenter une ou plusieurs dimensions et/ou une forme et/ou une couleur identique(s) ou différente(s) de celle(s) de la référence correspondante.

La « correspondance » d'une référence et d'une marque de repérage est une disposition prédéfinie de l'une par rapport à l'autre. Elle indique un positionnement particulier de l'appareil d'acquisition par rapport à la marque de repérage. La correspondance dépend de la nature de la référence et de la marque de repérage. La situation prédéfinie, qui correspond à des conditions d'acquisition cibles, peut notamment être une superposition, totale ou partielle, une juxtaposition, ou un alignement de la référence et de la marque de repérage.

La superposition exacte de la référence et de la marque de repérage permet non seulement déterminer la direction vers laquelle l'objectif de l'appareil d'acquisition doit pointer et/ou la distance entre l'appareil d'acquisition et l'écarteur, mais aussi, si la référence et/ou la marque de repérage sont asymétriques, l'orientation de l'appareil d'acquisition autour de cette direction.

Les dimensions et/ou les surfaces d'une marque de repérage et de la référence correspondante et/ou la distance entre plusieurs marques de repérage et entre des références correspondantes peuvent être utilisées pour régler la distance entre l'appareil d'acquisition et les arcades.

La référence peut être par exemple
- une ligne fixe, sur laquelle l'utilisateur doit par exemple aligner des marques de repérage,
- une forme, de préférence asymétrique, correspondant à la forme d'une marque de repérage à superposer, par exemple un point que l'utilisateur doit par exemple superposer à la marque de repérage, ou un cercle dans lequel l'utilisateur doit par exemple placer la marque de repérage,
- une forme colorée correspondant à la couleur d'une marque de repérage à superposer,
- une forme complémentaire à la forme d'une marque de repérage, de préférence de manière que la mise en correspondance de la marque de repérage et de la référence conduise à une forme ayant un sens, comme une forme géométrique, une lettre ou un texte, un dessin, ou un motif, par exemple.

Dans un mode de réalisation préféré, les références sont définies, au moins partiellement, à partir d'informations fournies par le modèle de référence initial. Par exemple, suivant les principes de la « réalité augmentée », la référence peut être une vue du modèle de référence initial, par exemple une vue de face ou une vue de droite ou une vue de gauche du modèle de référence initial, rendue visible, en transparence, sur l'écran de l'appareil d'acquisition d'images lors de l'acquisition. Il est ainsi très facile pour le patient de superposer approximativement une telle vue avec les dents qu'il doit prendre en photo.

Dans un mode de réalisation préféré, l'acquisition est effectuée au moyen d'un kit d'acquisition comportant :
- un écarteur dentaire, de préférence en un matériau biocompatible, comportant une marque de repérage ;
- un appareil d'acquisition d'images, de préférence du type décrit ci-dessus, comportant un écran de visualisation d'une image acquérable, et un programme d'ordinateur comprenant des instructions de code de programme pour afficher au moins une référence sur ledit écran, ladite référence étant de préférence immobile sur l'écran, et disposée dans une position dite "position de correspondance" dans laquelle, lorsque la marque de repérage correspond à la référence sur l'écran, l'image acquérable représente l'écarteur sous un angle de vue et/ou à une distance prédéterminé(e)(s).

Un kit permet avantageusement une acquisition d'images sans avoir recours à une personne spécialisée, notamment un orthodontiste. L'acquisition d'images peut être en particulier effectuée par le patient lui-même ou par un de ses proches, avec un simple téléphone mobile, n'importe où, et en particulier en dehors d'un cabinet médical, dentaire ou d'orthodontie.

En outre, l'appareil d'acquisition d'images n'a pas besoin d'être stabilisé mécaniquement, par exemple au moyen d'un trépied ou par intégration dans un appareil posé sur le sol.

Bien entendu, un kit d'acquisition ne permet pas un positionnement très précis de l'appareil d'acquisition par rapport aux dents.

En particulier, la précision du positionnement de l'écarteur par rapport aux dents est limitée. La personne qui crée les images positionne également l'appareil d'acquisition d'images de manière approximative, en dépit de la mise en correspondance de la marque de repérage par rapport à la référence sur l'écran. Comme on le verra plus en détails dans la suite de la description, le traitement des images ne nécessite cependant pas une grande précision du positionnement de l'appareil d'acquisition au moment où les images sont acquises.

A la différence de la technique antérieure, par exemple décrite dans WO2006/065955, il n'est notamment pas nécessaire, au moment de l'acquisition d'images, d'utiliser des marques de repérage dont le positionnement est parfaitement défini par rapport aux dents, en particulier parce qu'elles ont été fixées sur les dents elles-mêmes ou parce qu'elles résultent d'une modification locale, à un endroit précis, d'une dent, par exemple par laser. La possibilité d'acquérir les images avec une précision limitée constitue un avantage considérable, puisqu'elle rend possible cette acquisition en tout lieu et par toute personne. Le patient n'a en particulier plus besoin de se déplacer chez l'orthodontiste.

De préférence, aucune mesure sur les dents n'est effectuée pour disposer l'appareil d'acquisition dans la position de correspondance.

De préférence, aucune marque de repérage correspondant à une référence apparaissant sur l'écran n'est fixée directement sur les dents ou sur la gencive ou sur une arcade dentaire du patient.

L'appareil d'acquisition peut être en particulier un téléphone mobile et le programme peut être un applicatif spécialisé pour téléphone mobile.

L'écarteur peut présenter les caractéristiques des écarteurs utilisés jusqu'à présent. Il comporte classiquement un support muni d'un rebord s'étendant autour d'une ouverture et agencé de manière que les lèvres du patient puissent y reposer en laissant les dents du patient apparaître à travers ladite ouverture (figure 5a et figure 5c).

Le support, par exemple en plastique, présente de préférence une forme sensiblement plane et un poids inférieur à 500 g, de préférence inférieur à 300 g. L'ouverture est de préférence ménagée sensiblement au centre du support. La surface de l'ouverture est de préférence supérieure à 15 cm², supérieure à 30 cm², supérieure à 40 cm², supérieure à 50 cm², et/ou inférieure à 100 cm², inférieure à 80 cm², inférieure à 60 cm².

De préférence, comme représenté sur la figure 5a, l'écarteur comporte plusieurs marques de repérage, de préférence non alignées, de préférence coplanaires.

De préférence, l'écarteur comporte au moins trois marques de repérage et le programme d'ordinateur permet d'afficher sur l'écran de l'appareil d'acquisition une ou plusieurs références correspondantes.

Dans un mode de réalisation, le positionnement de l'appareil d'acquisition d'images résulte de la seule mise en concordance de références apparaissant sur l'écran dudit appareil d'acquisition avec des marques de repérage correspondantes, de préférence avec des marques de repérage d'un écarteur dentaire.

Dans un mode de réalisation, la ou les références qui apparaissent sur l'écran sont déterminées en fonction du patient et/ou du traitement thérapeutique. Autrement dit, le programme d'ordinateur est paramétré en fonction du patient afin que les images acquises correspondent spécifiquement aux besoins du patient. Avantageusement, au moment de l'acquisition des images, l'appareil d'acquisition est donc positionné dans une position sensiblement optimale au regard des particularités du patient et/ou du traitement thérapeutique appliqué.

Comme on le verra plus en détails dans la suite de la description, les marques de repérage de l'écarteur ont de préférence plusieurs fonctions. Elles permettent d'abord de guider le positionnement de l'appareil d'acquisition d'images au moment de l'acquisition des images, au moyen de références correspondantes apparaissant sur l'écran de l'appareil d'acquisition. Elles permettent également, à l'étape c), un redécoupage des images actualisées. Enfin, les marques de repérage de l'écarteur, qui apparaissent sur les images, permettent, à l'étape d), de déterminer grossièrement des conditions d'acquisition virtuelles approximant les conditions d'acquisition réelles, ce qui permet d'accélérer le traitement informatique.

Les étapes c) et suivantes sont de préférence réalisées soit sur un appareil personnel du patient, de préférence avec l'appareil utilisé à l'étape b), soit avec un applicatif chez un professionnel des soins dentaires, soit avec un serveur tiers dédié.

**A l'étape c),** chaque image actualisée est analysée de manière à réaliser, pour chaque image actualisée, une carte actualisée relative à au moins une information discriminante.

### Redécoupage

L'analyse de l'image peut comporter un redécoupage de l'image actualisée afin d'isoler la partie pertinente, en particulier pour supprimer, au moins partiellement, de l'image actualisée les éléments qui n'ont pas fait l'objet du modèle de référence initial, comme le nez ou les yeux du patient ou l'écarteur. Ce redécoupage, ou « croppage », est facilité par la représentation de marques de repérage sur l'image actualisée.

En particulier, de préférence, comme représenté sur les figures 5a et 5c, l'écarteur 10 porte au moins trois marques de repérage 12 non alignées. Si l'écarteur est en plusieurs parties, par exemple classiquement en deux parties, chaque partie porte de préférence au moins trois marques de repérage non alignées.

La forme d'une marque de repérage, par exemple une forme asymétrique, peut être également utilisée pour repérer la position de l'écarteur sur l'image actualisée.

De préférence, les marques de repérage présentent des formes et/ou des couleurs facilitant leur identification sur une image actualisée. Par exemple, elles peuvent être de couleur noire alors que le reste de l'écarteur est de couleur blanche.

Dans un mode de réalisation, les marques de repérage présentent des formes et/ou des couleurs permettant de les identifier individuellement. Par exemple, elles peuvent être chacune de couleur différente.

L'identification des marques de repérage sur l'image actualisée permet d'identifier la zone de l'image actualisée contenant les éléments ayant fait l'objet du modèle de référence initial, c'est-à-dire les dents et les gencives. L'image actualisée peut alors être rognée en conséquence. La comparaison des figures 5a et 5b, ou 5c et 5d, illustre l'effet du redécoupage sur une image actualisée.

### Carte actualisée

Une carte actualisée représente une information discriminante dans le référentiel de l'image actualisée. Par exemple, la figure 6b est une carte actualisée relative au contour des dents obtenue à partir de l'image actualisée de la figure 6a.

L'information discriminante est de préférence choisie dans le groupe constitué par une information de contour, une information de couleur, une information de densité, une information de distance, une information de brillance, une information de saturation, une information sur les reflets et des combinaisons de ces informations.

L'homme de l'art sait comment traiter une image actualisée pour faire apparaître l'information discriminante. Ce traitement comporte par exemple l'application de masques ou de filtres bien connus, fournis avec des logiciels de traitement d'images. De tels traitements permettent par exemple de détecter les régions de fort contraste pour déterminer des contours.

Ces traitements comprennent notamment une ou plusieurs des méthodes connues et préférées suivantes, à savoir :
- par application d'un filtre Canny, notamment pour rechercher des contours en utilisant l'algorithme de Canny ;
- par application d'un filtre Sobel, notamment pour calculer des dérivées au moyen de l'opérateur étendu de Sobel;
- par application d'un filtre de Laplace, pour calculer le laplacien d'une image;
- par détection tâches sur une image (« Blobdetecor ») ;
- par application d'un seuil (« Threshold ») pour appliquer un seuil fixe à chaque élément d'un vecteur ;
- par redimensionnement, en utilisant des relations entre les zones de pixels (« Resize(Area) ») ou des interpolations bi-cubiques sur l'environnement des pixels ;
- par érosion de l'image au moyen de d'un élément spécifique structurant ;
- par dilatation, de l'image au moyen de d'un élément spécifique structurant ;
- par retouche, en particulier en utilisant des régions au voisinage de la zone restaurée ;
- par application d'un filtre bilatéral ;
- par application d'un flou gaussien ;
- par application d'un filtre d'Otsu, pour rechercher le seuil qui minimise la variance intra-classes ;
- par application d'un filtre A^{∗}, pour rechercher un chemin entre des points ;
- par application d'un seuil adaptatif (« Adaptive Threshold ») pour appliquer un seuil adaptatif à un vecteur ;
- par application d'un filtre d'égalisation d'un histogramme d'une image en nuances de gris en particulier;
- par détection de flou ("BlurDetection"), pour calculer l'entropie d'une image en utilisant son laplacien ;
- par détection de contours (« FindContour ») d'une image binaire ;
- par remplissage de couleurs ("FloodFill"), notamment pour remplir un élément connecté avec une couleur déterminée.

Les méthodes non limitatives suivantes, bien qu'elles ne soient pas préférées, peuvent être également mises en œuvre :
- par application d'un filtre "MeanShift", de manière à trouver un objet sur une projection de l'image ;
- par application d'un filtre « CLAHE », pour « Contrast Limited Adaptive Histogram Equalization » ;
- par application d'un filtre « Kmeans", pour déterminer le centre de clusters et de groupes d'échantillons autour de clusters ;
- par application d'un filtre DFT, de manière à effectuer une transformation de Fourier discrète, directe ou inverse d'un vecteur ;
- par calcul de moments ;
- par application d'un filtre « HuMoments » pour calculer des invariants de Hu invariants ;
- par calcul de l'intégrale d'une image ;
- par application d'un filtre Scharr, permettant de calculer une dérivée de l'image en mettant en œuvre un opérateur de Scharr ;
- par recherche de l'enveloppe convexe de points ("ConvexHull") ;
- par recherche de points de convexité d'un contour ("ConvexityDefects") ;
- par comparaison de formes (« MatchShapes ») ;
- par vérification si des points sont dans un contour (« PointPolygonTest ») ; Par détection des contours Harris (« CornerHarris ») ;
- par la recherche des valeurs propres minimales de matrices de gradients, pour détecter les coins ("CornerMinEigenVal ») ;
- par application d'une transformée de Hough pour trouver des cercles dans une image en nuances de gris (« HoughCircles ») ;
- par "Active contour modeling" (traçage du contour d'un objet à partir d'une image 2D potentiellement « bruitée » ;
- par calcul d'un champ de forces, appelées GVF ("gradient vector flow"), dans une partie de l'image ;
- par classement en cascade (« CascadeClassification ») ;
- par « Deepleraning ».

De préférence, l'information discriminante est optimisée au moyen d'un dit procédé d'optimisation comportant des étapes C1 à C3.

**A l'étape optionnelle d),** on détermine, de manière grossière, les conditions d'acquisition réelles lors de l'étape b). Autrement dit, on détermine au moins la position relative de l'appareil d'acquisition d'images au moment où il a pris l'image actualisée (position de l'appareil d'acquisition dans l'espace et orientation de cet appareil). L'étape d) permet avantageusement de limiter le nombre de tests sur des conditions d'acquisition virtuelles lors de l'étape e), et permet donc d'accélérer considérablement l'étape e).

On utilise de préférence une ou plusieurs règles heuristiques. Par exemple, de préférence, on exclut des conditions d'acquisition virtuelles susceptibles d'être testées à l'étape e), les conditions qui correspondent à une position de l'appareil d'acquisition d'images derrière les dents ou à une distance des dents supérieure à 1 m.

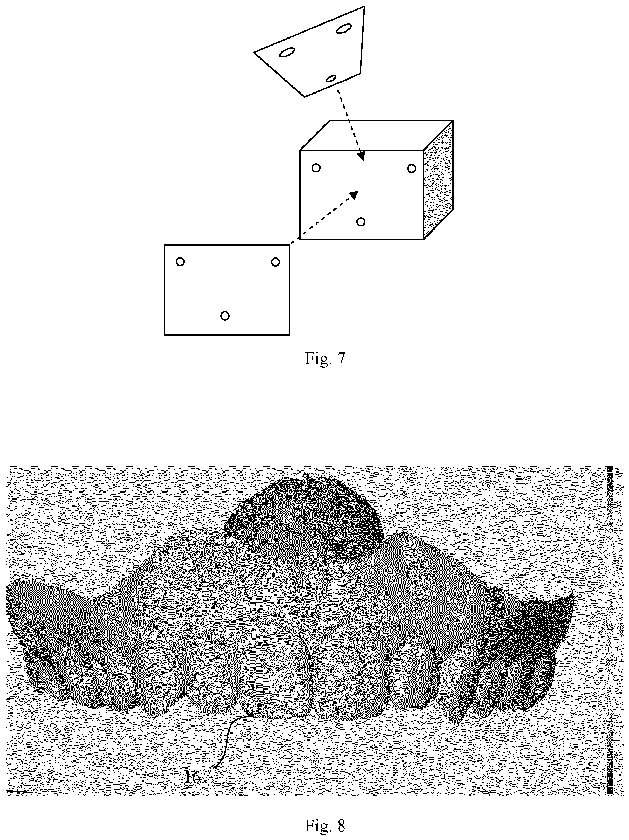

Dans un mode de réalisation préféré, comme illustré sur la figure 7, on utilise des marques de repérage représentées sur l'image actualisée, et en particulier des marques de repérage 12 de l'écarteur, pour déterminer une région de l'espace sensiblement conique délimitant des conditions d'acquisition virtuelles susceptibles d'être testées à l'étape e), ou "cône de test".

Précisément, on dispose de préférence au moins trois marques de repérage 12 non alignées sur l'écarteur 10, par exemple, et on mesure précisément leurs positions relatives sur l'écarteur.

Les marques de repérage sont ensuite repérées sur l'image actualisée, comme décrit précédemment. De simples calculs trigonométriques permettent de déterminer approximativement la direction selon laquelle l'image actualisée a été prise. Un cône orienté selon cette direction, dont le sommet est au niveau de l'écarteur et dont le demi-angle au sommet est de préférence inférieur à 10°, de préférence inférieur à 5°, par exemple de 3° peut alors être défini comme "cône de test". Le demi-angle au sommet correspond à un degré d'incertitude. Plus le demi-angle au sommet est petit, plus grande est la probabilité que les conditions d'acquisition virtuelles correspondant aux conditions d'acquisition réelles soient hors du cône de test.

Par exemple, lorsque l'image actualisée est prise perpendiculairement au plan des trois marques de repérage sur l'écarteur, on peut en déduire que l'appareil d'acquisition était sensiblement dans un cône de test dont l'axe est sensiblement perpendiculaire à ce plan lors de la prise de l'image actualisée. Si les positions relatives des trois marques de repérage sur l'image actualisée sont différentes de celles que les marques de repérage occupent sur l'écarteur, l'axe du cône de test dans lequel est limitée la recherche du positionnement de l'appareil d'acquisition lors de l'acquisition de l'image actualisée est incliné par rapport au plan des marques de repérage, comme représenté sur la figure 7.

Dans un mode de réalisation particulier, comme illustré sur les figures 5a et 5c, l'écarteur comporte des parties gauche et droite indépendantes, qui comportent chacune au moins trois marques de repérage, de préférence au moins quatre marques de repérage. Un cône de test gauche peut être ainsi déterminé au moyen des marques de repérage de la partie gauche et un cône de test droit peut être déterminé au moyen des marques de repérage de la partie droite de l'écarteur. Les conditions d'acquisition virtuelles susceptibles d'être testées peuvent être ensuite limitées à des positions de l'appareil d'acquisition dans l'espace appartenant à ces deux cônes de test. On peut aussi considérer que la meilleure évaluation de la position de l'appareil d'acquisition correspond à la position moyenne entre la meilleure position dans le cône de test gauche et la meilleure position dans le cône de recherche droit.

La position des marques de repérage sur l'image actualisée permet également d'évaluer l'assiette de l'appareil d'acquisition lors de la capture de l'image actualisée. Par exemple, si on sait que deux marques de repérage sont sensiblement alignées selon une direction horizontale lors de l'acquisition de l'image actualisée, la direction de la droite contenant ces deux points sur l'image actualisée fournit une indication sur l'orientation de l'appareil d'acquisition dans les conditions d'acquisition réelles.

Enfin, la taille et la surface des marques de repérage sur l'image actualisée ou leur espacement peuvent permettre d'évaluer la distance entre l'appareil d'acquisition d'images et les dents lors de l'acquisition de l'image actualisée, et donc de réduire le cône de test à un tronc de cône.

A l'étape optionnelle d), on peut également utiliser des données fournies par l'appareil d'acquisition et concernant son orientation, par exemple des données gyroscopiques.

De préférence, à l'étape d), on détermine, de manière grossière, la calibration de l'appareil d'acquisition réelle lors de l'étape b).

La façon dont chaque paramètre de calibration agit sur l'image acquise est bien connue. En particulier, le fonctionnement d'un appareil d'acquisition peut être classiquement modélisé de manière à pouvoir tester une calibration particulière sur l'image acquise. Les inventeurs ont inversé un tel modèle, sans difficulté technique particulière, afin que par l'analyse de la représentation de l'écarteur, il soit possible d'évaluer grossièrement la calibration de l'appareil d'acquisition lors de l'étape b).

Par exemple, le rapport entre la surface des marques de repérage sur l'image actualisée et la surface de l'image actualisée permet d'évaluer la distance focale de l'appareil d'acquisition lors de l'étape b). La représentation d'une marque de repérage dont on connait les caractéristiques optiques permettent d'évaluer le temps d'exposition et la sensibilité.

Dans un mode de réalisation préféré, une marque de repérage est un relief qui ne s'étend pas exclusivement dans le plan général de l'écarteur, correspondant à un plan parallèle au frontal (ou coronal). De préférence, une marque de repérage est un relief qui s'étend dans un plan sensiblement perpendiculaire au plan général de l'écarteur. Le relief peut en particulier présenter la forme d'une languette qui, lorsque l'écarteur est dans sa position de service, s'étend vers le fond de la bouche.

L'analyse de la représentation de ce relief permet avantageusement d'évaluer la profondeur de champ. Alternativement, deux marques de repérage qui ne sont pas dans un même plan frontal peuvent être utilisées à cet effet.

L'étape d) ne permet qu'une évaluation grossière des conditions d'acquisition réelles. L'étape d) permet cependant de déterminer un ensemble restreint de conditions d'acquisition virtuelles susceptibles de correspondre aux conditions d'acquisition réelles, et, dans cet ensemble, des conditions d'acquisition virtuelles constituant le meilleur point de départ pour l'étape e1) décrite ci-après.

L'étape d) permet également de détecter des images actualisées inadaptées pour la poursuite du procédé, par exemple une image actualisée qui ne ferait pas apparaître les marques de repérage. De préférence, le procédé est alors repris à l'étape c) avec une nouvelle image actualisée.

Bien entendu, les différentes méthodes pouvant être mises en œuvre à l'étape d) peuvent être combinées.

L'objectif de l'étape e) est de modifier le modèle de référence initial jusqu'à obtenir un modèle de référence actualisé qui corresponde à l'image actualisée. Idéalement, le modèle de référence actualisé est donc un modèle de référence tridimensionnel numérique à partir duquel l'image actualisée aurait pu être prise si ce modèle avait été réel.

On teste donc une succession de modèles de référence « à tester », le choix d'un modèle de référence à tester étant de préférence dépendant du niveau de correspondance des modèles de référence « à tester » précédemment testés avec l'image actualisée. Ce choix est de préférence effectué en suivant un procédé d'optimisation connu, en particulier choisi parmi les procédés d'optimisation métaheuristiques, de préférence évolutionnistes, en particulier dans les procédés de recuit simulé.

**A l'étape e1),** on détermine que le modèle de référence à tester est le modèle de référence initial lors de la première exécution de l'étape e2).

**A l'étape e2),** on commence par déterminer des conditions d'acquisition virtuelles à tester, c'est-à-dire une position et une orientation virtuelles susceptibles de correspondre à la position et l'orientation réelles de l'appareil d'acquisition lors de la capture de l'image actualisée, mais aussi, de préférence, une calibration virtuelle susceptible de correspondre à la calibration réelle de l'appareil d'acquisition lors de la capture de l'image actualisée.

Les premières conditions d'acquisition virtuelles à tester peuvent être aléatoires. De préférence, elles sont choisies dans l'ensemble limité déterminé à l'étape d), et de préférence encore, correspondent à des conditions d'acquisition virtuelles correspondant, d'après l'étape d), aux conditions d'acquisition virtuelles les plus prometteuses, c'est-à-dire constituant le meilleur tremplin pour approcher, le plus rapidement possible, les conditions d'acquisition réelles (étape e21)).

On configure ensuite virtuellement l'appareil d'acquisition d'images dans les conditions d'acquisition virtuelles à tester afin d'acquérir une image de référence du modèle de référence à tester dans ces conditions d'acquisition virtuelles à tester. L'image de référence correspond donc à l'image qu'aurait prise l'appareil d'acquisition d'images s'il avait été placé, par rapport au modèle de référence à tester, et optionnellement calibré, dans les conditions d'acquisition virtuelles à tester (étape e22)).

Si l'image actualisée a été prise alors que la position des dents était exactement celle dans le modèle de référence à tester, et si les conditions d'acquisition virtuelles sont exactement les conditions d'acquisition réelles, l'image de référence est donc exactement superposable à l'image actualisée. Les différences entre l'image actualisée et l'image de référence résultent d'erreurs dans l'évaluation des conditions d'acquisition virtuelles (si elles ne correspondent pas exactement aux conditions d'acquisition réelles) et de déplacements des dents entre l'étape b) et le modèle de référence à tester.

Pour comparer les images actualisées et de référence, on compare l'information discriminante sur ces deux images. Plus précisément, on réalise, à partir de l'image de référence, une carte de référence représentant l'information discriminante (étape e23)).

Les cartes actualisées et de référence, portant toutes les deux sur la même information discriminante, sont ensuite comparées et on évalue la différence entre ces deux cartes au moyen d'un score. Par exemple, si l'information discriminante est le contour des dents, on peut comparer la distance moyenne entre les points du contour des dents qui apparait sur l'image de référence et les points du contour correspondant qui apparaît sur l'image actualisée, le score étant d'autant plus élevé que cette distance est faible.

De préférence, les conditions d'acquisition virtuelles comprennent les paramètres de calibration de l'appareil d'acquisition. Le score est d'autant plus élevé que les valeurs des paramètres de calibration testées sont proches des valeurs des paramètres de calibration de l'appareil d'acquisition utilisé à l'étape b). Par exemple, si l'ouverture de diaphragme testée est éloignée de celle de l'appareil d'acquisition utilisé à l'étape b), l'image de référence présente des régions floues et des régions nettes qui ne correspondent pas aux régions floues et aux régions nettes de l'image actualisée. Si l'information discriminante est le contour des dents, les cartes actualisées et de référence ne représenteront donc pas les mêmes contours et le score sera faible.

Le score peut être par exemple un coefficient de corrélation.

Le score est ensuite évalué au moyen d'une première fonction d'évaluation. La première fonction d'évaluation permet de décider si le cyclage sur l'étape e2) doit être poursuivi ou stoppé. La première fonction d'évaluation peut par exemple être égale à 0 si le cyclage doit être stoppé ou être égale à 1 si le cyclage doit se poursuivre.

La valeur de la première fonction d'évaluation peut dépendre du score atteint. Par exemple, il peut être décidé de poursuivre le cyclage sur l'étape e2) si le score ne dépasse pas un premier seuil. Par exemple, si une correspondance exacte entre les images actualisée et de référence conduit à un score de 100%, le premier seuil peut être, par exemple, de 95%. Bien entendu, plus le premier seuil sera élevé, meilleure sera la précision de l'évaluation des conditions d'acquisition virtuelles si le score parvient à dépasser ce premier seuil.

La valeur de la première fonction d'évaluation peut également dépendre de scores obtenus avec des conditions d'acquisition virtuelles testées précédemment.

La valeur de la première fonction d'évaluation peut également dépendre de paramètres aléatoires et/ou du nombre de cycles de l'étape e2) déjà effectués.

En particulier, il est possible qu'en dépit de la répétition des cycles, on ne parvienne pas à trouver des conditions d'acquisition virtuelles qui soient suffisamment proches des conditions d'acquisition réelles pour que le score atteigne ledit premier seuil. La première fonction d'évaluation peut alors conduire à la décision de quitter le cyclage bien que le meilleur score obtenu n'ait pas atteint ledit premier seuil. Cette décision peut résulter, par exemple, d'un nombre de cycles supérieur à un nombre maximal prédéterminé.

Un paramètre aléatoire dans la première fonction d'évaluation peut également autoriser la poursuite de tests de nouvelles conditions d'acquisition virtuelles, bien que le score apparaisse satisfaisant.

Les fonctions d'évaluation classiquement utilisées dans les procédés d'optimisation métaheuristiques, de préférence évolutionnistes, en particulier dans les procédés de recuit simulé, peuvent être utilisées pour la deuxième fonction d'évaluation.

Si la valeur de la première fonction d'évaluation indique qu'il est décidé de poursuivre le cyclage sur l'étape e2), on modifie les conditions d'acquisition virtuelles testées (étape e25)) et on recommence un cycle (étape e2)) consistant à réaliser une image de référence et une carte de référence, puis à comparer cette carte de référence avec la carte actualisée pour déterminer un score.

La modification des conditions d'acquisition virtuelles correspond à un déplacement virtuel dans l'espace et/ou à une modification de l'orientation et/ou, de préférence, à une modification de la calibration de l'appareil d'acquisition. Cette modification peut être aléatoire, à condition cependant que les nouvelles conditions d'acquisition virtuelles à tester appartiennent toujours à l'ensemble déterminé à l'étape d). La modification est de préférence guidée par des règles heuristiques, par exemple en favorisant les modifications qui, d'après une analyse des précédents scores obtenus, apparaissent les plus favorables pour augmenter le score.

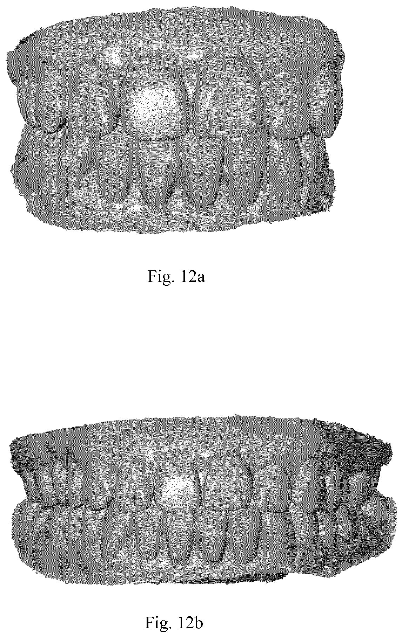

Les figures 12a et 12b illustrent par exemple l'effet d'une modification des conditions d'acquisition virtuelles, en l'occurrence d'une modification de la distance focale, sur l'image de référence.

Le cyclage sur e2) est poursuivi jusqu'à ce que la valeur de la première fonction d'évaluation indique qu'il est décidé de sortir de ce cyclage et de poursuivre à l'étape e3), par exemple si le score atteint ou dépasse ledit premier seuil.

L'optimisation des conditions d'acquisition virtuelles à l'étape e2) est de préférence effectuée en utilisant une méthode métaheuristique, de préférence évolutionniste, de préférence un algorithme de recuit simulé. Un tel algorithme est bien connu pour l'optimisation non linéaire.

Si on a quitté le cyclage sur l'étape e2), sans qu'un score satisfaisant ait pu être obtenu, par exemple sans que le score ait pu atteindre ledit premier seuil, le procédé peut être arrêté (situation d'échec) ou repris à l'étape c) avec une nouvelle information discriminante et/ou avec une nouvelle image actualisée. Le procédé peut être également poursuivi avec les conditions d'acquisition virtuelles correspondant au meilleur score atteint. Un avertissement peut être émis afin d'informer l'utilisateur de l'erreur sur le résultat.

Si on a quitté le cyclage sur l'étape e2) alors qu'un score satisfaisant a pu être obtenu, par exemple parce que le score a atteint, voire dépassé ledit premier seuil, les conditions d'acquisition virtuelles correspondent sensiblement aux conditions d'acquisition réelles.

De préférence, les conditions d'acquisition virtuelles comprennent les paramètres de calibration de l'appareil d'acquisition. Le procédé conduit permet ainsi d'évaluer les valeurs de ces paramètres sans qu'il soit nécessaire de connaître la nature de l'appareil d'acquisition ou son réglage. L'étape b) peut donc être réalisée sans précaution particulière, par exemple par le patient lui-même au moyen de son téléphone mobile.

En outre, la recherche de la calibration réelle est effectuée en comparant une image actualisée avec des vues d'un modèle de référence initial dans des conditions d'acquisition virtuelles que l'on teste. Avantageusement, elle ne nécessite pas que l'image actualisée fasse apparaître une jauge étalon de calibration, c'est-à-dire une jauge dont on connaît précisément les caractéristiques permettant déterminer la calibration de l'appareil d'acquisition.

WO2006/065955, décrit l'utilisation d'images pour fabriquer des modèles tridimensionnels dans le domaine des traitements orthodontiques. Cependant, ce document ne décrit pas de procédé permettant d'utiliser de simples photographies, présentant classiquement des images partielles des dents, des portions d'image floues et des reflets variables, prises généralement de manière non simultanée, sans avoir besoin de sélectionner des points remarquables sur les images, et avec un appareil d'acquisition dont la calibration n'est pas connue.

Dans un dit procédé de contrôle du positionnement de dents, les images actualisées ne servent pas à créer un modèle tridimensionnel actualisé totalement nouveau, mais seulement à modifier le modèle de référence initial, très précis. Un modèle tridimensionnel actualisé totalement nouveau créé à partir de simples photographies prises sans précautions particulières serait en particulier trop imprécis pour qu'une comparaison avec le modèle de référence initial puisse conduire à des conclusions sur le déplacement des dents.

Des différences peuvent subsister entre les conditions d'acquisition virtuelles déterminées et les conditions d'acquisition réelles, en particulier si des dents se sont déplacées entre les étapes a) et b). La corrélation entre les images actualisée et de référence peut alors être encore améliorée en reprenant l'étape e2), le modèle de référence à tester étant alors modifié par déplacement d'un ou plusieurs modèles de dents (étape e3)).

La recherche du modèle de référence approximant au mieux le positionnement des dents lors de l'acquisition de l'image actualisée peut être effectuée comme la recherche des conditions d'acquisition virtuelles approximant au mieux les conditions d'acquisition réelles (étape e2)).

En particulier, le score est évalué au moyen d'une deuxième fonction d'évaluation. La deuxième fonction d'évaluation permet de décider si le cyclage sur les étapes e2) et e3) doit être poursuivi ou stoppé. La deuxième fonction d'évaluation peut par exemple être égale à 0 si le cyclage doit être stoppé ou être égale à 1 si le cyclage doit se poursuivre.

La valeur de la deuxième fonction d'évaluation dépend de préférence du meilleur score obtenu avec le modèle de référence à tester, c'est-à-dire des différences entre les cartes actualisée et de référence, dans les conditions d'acquisition virtuelles approximant au mieux lesdites conditions d'acquisition réelles.

La valeur de la deuxième fonction d'évaluation peut également dépendre du meilleur score obtenu avec un ou plusieurs modèles de référence testés précédemment.

Par exemple, il peut être décidé de poursuivre le cyclage si le score ne dépasse pas un deuxième seuil minimal. La valeur de la deuxième fonction d'évaluation peut également dépendre de paramètres aléatoires et/ou du nombre de cycles des étapes e2) et e3) déjà effectués.

Les fonctions d'évaluation classiquement utilisées dans les procédés d'optimisation métaheuristiques, de préférence évolutionnistes, en particulier dans les procédés de recuit simulé, peuvent être utilisées pour la deuxième fonction d'évaluation.

Si la valeur de la deuxième fonction d'évaluation indique qu'il est décidé de poursuivre le cyclage sur les étapes e2) et e3), on modifie le modèle de référence à tester et on recommence un cycle (étapes e2) et e3)) avec le nouveau modèle de référence à tester.

La modification du modèle de référence à tester correspond à un déplacement d'un ou plusieurs modèles de dents. Cette modification peut être aléatoire. La modification est de préférence guidée par des règles heuristiques, par exemple en favorisant les modifications qui, d'après une analyse des précédents scores obtenus, apparaissent les plus favorables pour augmenter le score.

De préférence, on recherche le déplacement d'un modèle de dent qui a le plus fort impact sur le score, on modifie le modèle de référence à tester en déplaçant ce modèle de dent, puis on poursuit le cyclage sur les étapes e2) et e3) de manière à optimiser le score. On peut ensuite rechercher, parmi les autres modèles de dent, celui qui a le plus fort impact sur l'amélioration du score, et à nouveau rechercher le déplacement optimal de cet autre modèle de dent sur le score. On peut poursuivre ainsi avec chaque modèle de dent.

Ensuite, il est possible de reprendre un cycle sur l'ensemble des modèles de dent et de poursuivre ainsi jusqu'à l'obtention d'un score supérieur au deuxième seuil. Bien entendu, d'autres stratégies peuvent être utilisées pour déplacer un ou plusieurs modèles de dent dans le modèle de référence à tester et rechercher le score maximal.

Le cyclage sur les étapes e2) et e3) est poursuivi jusqu'à ce que la valeur de la deuxième fonction d'évaluation indique qu'il est décidé de sortir de ce cyclage et de poursuivre à l'étape f), par exemple si le score atteint ou dépasse ledit deuxième seuil.

La recherche d'un modèle de référence avec un cyclage sur les étapes e2) et e3) pour rechercher les positions des modèles de dent qui optimisent le score est de préférence effectuée en utilisant une méthode métaheuristique, de préférence évolutionniste, de préférence un algorithme de recuit simulé. Un tel algorithme est bien connu pour l'optimisation non linéaire.

Si on a quitté le cyclage sur les étapes e2) et e3) sans qu'un score satisfaisant ait pu être obtenu, par exemple sans que le score ait pu atteindre ledit deuxième seuil, le procédé peut être arrêté (situation d'échec) ou repris à l'étape c) avec une nouvelle information discriminante et/ou avec une nouvelle image actualisée.

S'il est décidé de relancer le procédé à l'étape c) à partir d'une autre information discriminante et/ou d'une autre image actualisée parce que le premier seuil ou le deuxième seuil n'a pas été atteint, le choix de la nouvelle information discriminante et/ou de la nouvelle image actualisée peut dépendre des scores obtenus précédemment, afin de favoriser l'information discriminante et/ou l'image actualisée qui, au regard de ces scores, apparaissent les plus prometteuses.

Une nouvelle information discriminante, obtenue par exemple par combinaison d'autres informations discriminantes déjà testées, peut être utilisée. Le cas échéant, il peut être également demandé d'acquérir une ou plusieurs nouvelles images actualisées. De préférence, on fournit des indications permettant de guider le positionnement de l'appareil d'acquisition pour la capture de cette nouvelle image actualisée. Par exemple, on peut indiquer au patient qu'il devrait prendre une photo de la partie droite de son arcade inférieure.

Si on a quitté le cyclage sur les étapes e2) et e3) sans qu'un score satisfaisant ait pu être obtenu, le procédé peut être également poursuivi avec le modèle de référence et les conditions d'acquisition virtuelles correspondant au meilleur score atteint. Un avertissement peut être émis afin d'informer l'utilisateur de l'erreur sur le résultat.

Si on a quitté le cyclage sur les étapes e2) et e3) alors qu'un score satisfaisant a pu être obtenu, par exemple parce que le score a atteint, voire dépassé ledit deuxième seuil, les conditions d'acquisition virtuelles correspondent sensiblement aux conditions d'acquisition réelles et les modèles de dents dans le modèle de référence obtenu (dit « modèle de référence actualisé ») sont sensiblement dans la position des dents du patient au moment de l'étape b).

Le cyclage sur les étapes e2) et e3) permet avantageusement d'améliorer l'évaluation des paramètres de calibration de l'appareil d'acquisition à l'étape b).

**A l'étape f),** on compare le modèle de référence actualisé, résultant de l'optimisation par déplacement des modèles de dents, avec le modèle de référence initial. Le modèle de référence actualisé correspond sensiblement à l'image actualisée. La comparaison de l'étape f) permet donc d'observer les différences entre le positionnement des dents à l'étape a) (modèle de référence initial) et lors de l'acquisition de l'image actualisée (étape b)). Le procédé permet ainsi de déterminer précisément, pour chacune des dents, les mouvements entre ces deux étapes.

En répétant les étapes b) et suivantes, il est également possible d'évaluer la vitesse d'évolution de la position des dents, et ainsi de mesurer, par exemple, l'efficacité d'un traitement orthodontique. Le procédé de contrôle du positionnement de dents peut par exemple être utilisé pour suivre à distance l'évolution d'un traitement orthodontique, et ainsi optimiser les rendez-vous des patients chez leurs orthodontistes.

Dans un mode de réalisation préféré, le procédé de contrôle est mis en œuvre plusieurs fois pour un même patient, de préférence successivement avec plusieurs informations discriminantes, de préférence plus de 2, plus de 3, plus de 5 informations discriminantes pour chaque image actualisée et/ou avec plusieurs images actualisées, de préférence plus de 2, plus de 3, plus de 5 images actualisées. L'évaluation du déplacement d'une dent peut être ainsi affinée en tenant compte des différents scores obtenus. La comparaison de ces scores permet également, le cas échéant, d'écarter les informations discriminantes et/ou les images actualisées insatisfaisantes.

En fonction du déplacement mesuré, une information pratique peut être générée. Si le déplacement est faible, cette information pratique peut être qu'aucune action n'est à engager. Au contraire, si une ou plusieurs dents se sont sensiblement déplacées, l'information peut être de planifier une visite chez le dentiste ou l'orthodontiste. De préférence, l'information pratique dépend du degré de déplacement des dents. Dans un mode de réalisation, un rendez-vous peut être automatiquement pris chez le dentiste ou l'orthodontiste, en fonction de l'amplitude et/ou de la nature des déplacements détectés.

Dans un mode de réalisation, l'information pratique est utilisée pour modifier l'intervalle de temps après lequel le patient devra être averti qu'une nouvelle image actualisée doit être créée.

Dans un mode de réalisation, l'appareil individuel permet d'afficher des images, voire une séquence d'images montrant le positionnement des dents à différentes dates. Ces images peuvent être présentées sous la forme d'une animation, par exemple sous la forme d'un diaporama ou d'un film.

De préférence, l'appareil d'acquisition d'images est un téléphone qui permet de transmettre les résultats obtenus par la mise en œuvre du procédé, de préférence de manière sécurisée.

La communication peut être par exemple effectuée, au moins en partie, par la voie hertzienne, de préférence suivant au moins un protocole choisi parmi les protocoles edge, 3G, 4G, udmsa, hpdmsa, bluetooth, et wifi, ou par tout autre protocole, adapté aux équipements mobiles ou nomades, par synchronisation filaire avec l'ordinateur personnel, ou par transmission optique.

Comme cela apparaît clairement à présent, le procédé de contrôle du positionnement de dents permet un contrôle précis et efficace du positionnement des dents du patient, sensiblement sans contrainte pour le patient. En particulier, de simples photographies prises sans précaution particulière, par exemple avec un téléphone mobile, suffit. Le patient peut donc facilement mettre en œuvre ce procédé.

### Description détaillée d'un procédé d'adaptation d'un appareil orthodontique porté

L'appareil orthodontique porté par le patient peut être le premier appareil orthodontique initialement mis à sa disposition pour son traitement orthodontique ou un appareil de substitution mis à sa disposition ultérieurement.

Le premier appareil orthodontique peut être fabriqué suivant un procédé conventionnel.

En particulier, pour un traitement au moyen de gouttières, l'orthodontiste peut, au début du traitement, déterminer une première série de gouttières adaptée pour que les dents atteignent une position correspondant à un modèle de référence objectif, représentant de préférence un set-up final, fabriqué suivant une étape b'), comme décrit ci-après. Cette détermination peut être réalisée de manière classique.

De préférence, seule la première gouttière de cette première série est réalisée et fournie au patient.

La possibilité d'évaluer le positionnement des dents à partir de simples photographies, prises sans précaution particulière, notamment à partir d'un téléphone mobile, permet de multiplier les contrôles lors d'un traitement orthodontique. Le traitement peut donc être adapté très régulièrement, notamment par adaptation ou changement de l'appareil orthodontique.

De manière générique, on appelle «appareil orthodontique de substitution» l'appareil résultant de cette adaptation du traitement. L'appareil orthodontique de substitution peut donc être l'appareil orthodontique qui était porté jusque-là, désigné par « appareil orthodontique porté », et adapté, par exemple par changement de l'arc orthodontique fixé aux dents ou modification de sa tension. L'appareil orthodontique de substitution peut être également un nouvel appareil orthodontique, notamment lorsque des gouttières actives sont utilisées pour le traitement.

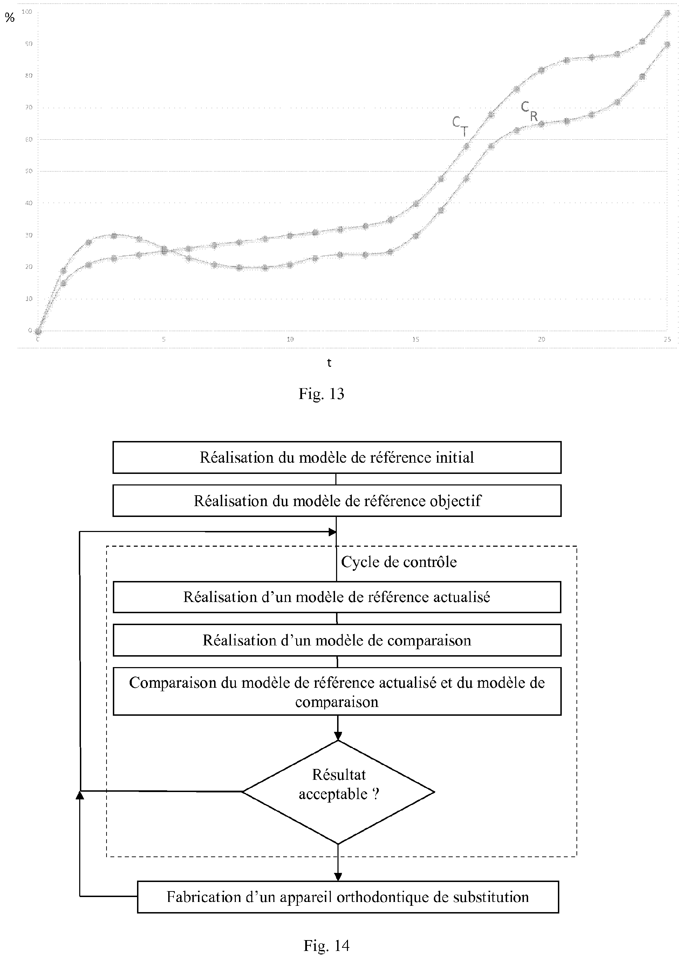

Un dit procédé d'adaptation d'un appareil orthodontique comporte les étapes a') à f) décrites ci-dessus, et illustrées sur la figure 14.

**L'étape a')** est identique à l'étape a) et peut comporter une ou plusieurs des caractéristiques optionnelles de l'étape a) décrites dans la présente description. Elle conduit à un modèle de référence initial qui représente numériquement, en trois dimensions, au moins la partie des arcades qui comporte les dents du patient à traiter. La fabrication du modèle de référence initial peut être effectuée par toutes les méthodes conventionnelles connues.

L'étape a') est de préférence la première étape. En particulier, le modèle de référence objectif est classiquement déterminé à partir du modèle de référence initial.

**L'étape b')** consiste à fabriquer un modèle de référence objectif qui représente numériquement, en trois dimensions, les dents à traiter dans une position à atteindre à un instant du traitement, en particulier à la fin du traitement (« set-up final ») ou à une étape intermédiaire prédéterminée du traitement (« set-up intermédiaire »), par exemple à un instant prévu pour changer de gouttière ou modifier la tension de l'arc orthodontique.

Tous les "set-ups intermédiaires" peuvent être réalisés au début de traitement. Le nombre de set-ups intermédiaires peut être supérieur à 1, à 2, à 10, à 20, à 30, à 40, à 50 ou à 60. La durée entre deux set-ups intermédiaires successifs peut être notamment inférieure à 10 semaines, à 8 semaines, à 6 semaines, à 4 semaines, à 2 semaines, ou à une semaine.

La fabrication du modèle de référence objectif peut être effectuée par toutes les méthodes conventionnelles connues.

De préférence, le modèle de référence objectif résulte d'une déformation du modèle de référence initial. En particulier, de préférence, les modèles de dent qui correspondent aux dents à traiter sont classiquement déplacés virtuellement jusqu'à leur position souhaitée.

Alternativement, un modèle physique, par exemple en plâtre, représentant les dents dans leur position souhaitée peut être également fabriqué, puis scanné en trois dimensions.

L'étape b') est de préférence effectuée sensiblement au même moment que l'étape a'). Elle peut cependant être réalisée à tout moment jusqu'à la première utilisation du modèle de référence objectif.

**L'étape c')** de réalisation d'un modèle de référence actualisé est identique à l'ensemble des étapes b) à e) et peut comporter une ou plusieurs des caractéristiques optionnelles de ces étapes décrites dans la présente description.

Elle est de préférence exécutée au moment où l'orthodontiste décide que le patient doit effectuer un contrôle de l'efficacité de son traitement, par exemple plus de 1 semaine ou plus de 2 semaines après l'étape a') de réalisation du modèle de référence initial. Elle peut être également décidée par le patient ou être planifiée.

Le modèle de référence actualisé est obtenu par déformation du modèle de référence initial, de préférence par déplacement des modèles de dent, en utilisant des informations issues des images actualisées. Plus précisément, la déformation est optimisée, de préférence par une méthode évolutionniste, de préférence par recuit simulé, pour que les images actualisées correspondent au mieux ("best fit") à des vues du modèle de référence initial déformé, alors appelé "modèle de référence actualisé". Autrement dit, ces vues sont sensiblement identiques aux images actualisées.

De préférence, le modèle de référence actualisé est rendu accessible à l'orthodontiste en temps réel. En particulier, s'il est déterminé avec un appareil du patient, par exemple avec son téléphone, il peut être envoyé à l'orthodontiste. Avantageusement, l'orthodontiste est ainsi averti et peut intervenir en conséquence.

L'utilisation des seules images actualisées, sans le modèle de référence initial, ne permet pas de construire un modèle tridimensionnel précis. C'est l'utilisation de ces images pour modifier le modèle de référence initial (qui est d'une grande précision, notamment lorsqu'il est obtenu par un scan) qui conduit à un modèle de référence actualisé précis, alors même que les images actualisées ont été prises sans précaution particulière.

Le modèle de référence actualisé fournit ainsi des valeurs de paramètres de positionnement au moment de l'acquisition des images actualisées pour des points des dents.

De préférence, la valeur d'un paramètre de positionnement pour un point d'une dent, par exemple du barycentre de la dent, est représentée sur un graphique.

De préférence, le graphique représente, pour chaque cycle de contrôle, la valeur de ce paramètre de positionnement, pour ce point de la dent. De préférence, une courbe relie ces points. De préférence encore, l'échelle des temps, de préférence en abscisse, est linéaire. La dynamique du traitement, pour ce qui concerne ce paramètre, est avantageusement immédiatement perceptible. L'orthodontiste et/ou le patient peuvent ainsi immédiatement percevoir une perte d'efficacité de l'appareil orthodontique et agir en conséquence.

**L'étape d')** de détermination d'un modèle de comparaison peut être exécutée à tout moment avant l'étape e').

De préférence, le modèle de comparaison fournit des positions théoriques des dents, ou de points de dents, à des instants intermédiaires entre le début et la fin du traitement. De préférence, le modèle de comparaison est un modèle numérique tridimensionnel. Il permet avantageusement une comparaison visuelle avec le modèle de référence actualisé. De préférence, la position théorique d'une dent ou d'un point d'une dent résulte d'un traitement du modèle de référence initial et du modèle de référence objectif, en particulier d'une interpolation, de préférence non linéaire, entre les positions de cette dent ou de ce point entre ces deux modèles.

Par exemple, la position (x,y,z) d'un point M d'une dent peut être définie dans un repère tridimensionnel, par exemple orthonormé. La position de ce point dans le modèle de référence initial, à l'instant to, et dans le modèle de référence objectif, à l'instant t_{G}, peut être notée (x₀,y₀,z₀) et (x_{G},y_{G},z_{G}), respectivement. Si on considère que le déplacement est rectiligne et évolue linéairement avec le temps, la position théorique intermédiaire de ce point à l'instant intermédiaire ti au milieu du traitement sera (xᵢ,yᵢ,zᵢ), avec xi = (x₀ + x_{G})/2, yᵢ = (y₀ + Y_{G})/2 et zᵢ = (z₀ + z_{G})/2.

De préférence encore, le modèle de comparaison dépend d'un ou plusieurs modèles de référence actualisés antérieurs et/ou d'un ou plusieurs modèles de comparaison antérieurs (établis lors de cycles de contrôle précédents, c'est-à-dire notamment d'étapes c') antérieures). La précision en est considérablement améliorée.

De préférence, la détermination du modèle de comparaison dépend des évolutions entre plusieurs modèles de référence actualisés, de préférence successifs. Par exemple, des modèles de référence actualisés peuvent avoir été réalisés il y a trois mois, deux mois et un mois, à t₋₃, t₋₂ et t₋₁, respectivement. Le déplacement du barycentre d'une dent le long de l'axe Ox) entre t₋₃ et t₋₂ peut être par exemple de 100 µm et le déplacement entre t₋₂ et t₋₁ peut être de 80 µm. On peut alors, par simple extrapolation linéaire, considérer que le déplacement, selon l'axe Ox), entre t₋₁ et aujourd'hui (t₀), sera de 60 µm, ce qui permet de créer une modèle de comparaison à t₀ avec une grande précision.

Toutes les méthodes classiques de prédiction peuvent être utilisées, une méthode de prédiction étant un procédé permettant d'établir, à partir d'un modèle prédictif, une prédiction en fonction de données connues.

Un modèle prédictif est donc un modèle permettant de simuler le déplacement futur des dents à partir de données passées, et notamment des déplacements passés desdites dents et/ou du comportement passé ou calculé de l'appareil orthodontique.

De préférence, le modèle prédictif tient compte de l'action de l'appareil orthodontique sur les dents. Ainsi, dans l'exemple ci-dessus s'il est connu que l'action de l'appareil orthodontique sur le déplacement du barycentre d'une dent selon l'axe Ox) décroît avec le temps, le déplacement, selon l'axe Ox), entre t₋₁ et aujourd'hui (t₀) pourra-t-il être estimé à 50 µm et non pas à 60 µm.

Des outils permettant de simuler les déplacements des dents entre deux positions extrêmes en tenant compte des interactions entre les dents, comme Clincheck de Align Technology, Suresmile^{®} de Orametrix, Virtual Setup de Harmony ou Insignia de Ormco, sont bien connus. De préférence, on utilise également un modèle de l'appareil orthodontique permettant de simuler son action, dans le temps, sur les dents, en fonction de leurs positions, et notamment de leur position dans le modèle de référence initial et/ou de leur position dans le modèle de référence actualisé. Comme on le verra plus en détail dans la suite de la description, un dit procédé d'évaluation du comportement d'un appareil orthodontique permet de concevoir et/ou d'affiner de tels modèles prédictifs.

De préférence, on construit un modèle dynamique, capable de fournir la position théorique des dents ou d'un ensemble de points de dents, de préférence de chaque modèle de dent, à tout instant entre le début et la fin du traitement ou, plus généralement, entre les instants correspondant aux modèles de référence initial et objectif. Un logiciel peut être utilisé à cet effet.

L'étape d') peut être réalisée immédiatement après que les étapes a') et b') ont été réalisées, par exemple moins de 3 mois, moins d'un mois ou moins d'une semaine après que les étapes a') et b') ont été réalisées.

De préférence, le modèle de comparaison est déterminé pour correspondre à une simulation de la position des dents traitées à un instant t_{c} le plus proche possible de l'instant d'exécution de l'étape c'), et en particulier de l'instant tₐ auquel les images actualisées ont été acquises. De préférence, ces deux instants sont séparés de moins de 1 mois, moins de deux semaines, de préférence de moins d'une semaine, de préférence de moins d'une journée.

Sur la figure 13, les deux instants t_{c} et ta sont confondus, ce qui est notamment possible lorsqu'on dispose d'un modèle tridimensionnel dynamique, capable de fournir la position théorique des dents, à tout instant entre le début et la fin du traitement. Connaissant l'instant ta, il suffit en effet de choisir, comme modèle de comparaison, le modèle dynamique à l'instant ta.

Dans un mode de réalisation, le modèle de comparaison est toujours le modèle de référence objectif. La précision de l'adaptation de l'appareil orthodontique n'est cependant pas optimale.

**A l'étape e')**, le modèle de référence actualisé, qui correspond à la situation observée au moment du contrôle, est comparé avec le modèle de comparaison, théorique.

La comparaison peut être faite par le patient et/ou l'orthodontiste.

La comparaison comporte de préférence une détermination, pour chaque point d'un ensemble de points des dents du modèle de référence actualisé, de préférence pour au moins trois points non coplanaires de chaque dent du modèle de référence actualisé, du déplacement (vecteur) entre les représentations de ce point sur les modèles de comparaison et actualisé. Ce déplacement est de préférence quantifié au moyen de distances et/ou d'angles dans un repère tridimensionnel Oxyz, par exemple dans un repère orthonormé. Le déplacement peut par exemple être déterminé en coordonnées cartésiennes ou cylindriques.

L'analyse du déplacement de quelques points des dents peut être difficile à présenter ou à analyser. Les points d'une même dent sont considérés comme rigidement liés les uns aux autres. De préférence, l'analyse du déplacement des points d'une même dent est généralisée pour évaluer le déplacement de tous les points de cette dent, c'est-à-dire le déplacement du modèle de dent correspondant.

En particulier, la connaissance du déplacement de trois points non coplanaires d'une dent permet d'évaluer le mouvement de cette dent entre sa représentation (c'est-à-dire le modèle de dent correspondant) dans le modèle de référence actualisé et sa représentation dans le modèle de comparaison, ou « quantité de mouvement ». La quantité de mouvement peut être exprimée par des différences de valeurs d'un ensemble de paramètres de positionnement permettant de fixer la position et l'orientation d'un objet dans l'espace, par exemple de coordonnées cartésiennes d'un point de la dent, par exemple de son barycentre, et de valeurs d'angles autour d'un repère fixe, par exemple orthonormé (différences entre les valeurs dans le modèle de référence actualisé et dans le modèle de comparaison).

De préférence, on détermine pour chacun des paramètres de positionnement, le chemin à parcourir, depuis le modèle de référence actualisé, pour atteindre le modèle de comparaison, par exemple la distance à parcourir par le barycentre de la dent selon chacun des axes d'un repère orthonormé fixe et la rotation à effectuer autour de chacun de ces axes.

De préférence, à l'étape e'), on évalue la vitesse des déplacements des points considérés, en utilisant de préférence le modèle de référence initial, et de préférence un ou plusieurs modèles de référence actualisés déterminés antérieurement, en particulier lors de cycles de contrôle antérieurs. La vitesse de déplacement d'un point d'une dent, selon une direction, entre deux modèles de référence actualisés successifs peut être simplement évaluée en divisant la distance entre les représentations de ce point sur ces deux modèles, suivant cette direction, par la durée écoulée entre la réalisation de ces deux modèles.

De préférence encore, on évalue le ralentissement ou l'accélération du déplacement des points des dents, par comparaison des vitesses de deux périodes successives.

Si le modèle de référence objectif est un set-up final ou intermédiaire, la comparaison permet d'évaluer les quantités de mouvement encore nécessaires pour terminer le traitement ou atteindre la fin de l'étape intermédiaire de traitement, respectivement. La comparaison permet aussi d'évaluer la vitesse des déplacements des dents jusqu'à la fin de l'étape intermédiaire ou la fin du traitement, respectivement.

Cette comparaison ne permet cependant pas d'estimer avec précision si le traitement se déroule comme prévu si elle n'est pas effectuée à un instant proche de la fin de l'étape intermédiaire ou de la fin du traitement. La détection d'une situation anormale, ou plus généralement d'une situation nécessitant une adaptation de l'appareil orthodontique, nécessite alors de grandes capacités d'analyse.

C'est pourquoi, de préférence, comme indiqué ci-dessus, le modèle de comparaison est un modèle qui correspond à une estimation de la position des dents espérée sensiblement à l'instant ta de l'étape c'), par exemple déterminée en début de traitement ou suite au cycle de contrôle immédiatement précédent.

La comparaison entre la situation réelle, évaluée avec le modèle de référence actualisé déterminé à l'étape c'), et la situation attendue, évaluée avec le modèle de comparaison, permet de déterminer si l'appareil orthodontique remplit correctement sa fonction.

Le cas échéant, elle permet également de déterminer ce qui fait défaut, par exemple une force trop élevée sur une dent, selon une direction.

On considère que le résultat de la comparaison est insatisfaisant si un écart entre le modèle de référence actualisé et le modèle de comparaison dépasse une valeur de seuil. Par exemple, si le modèle de comparaison correspond à un positionnement des dents prévu pour l'instant de la création du modèle de référence actualisé, un écart de plus de 50 µm de la position du barycentre selon l'axe Ox) peut être considéré comme insatisfaisant. Si le modèle de comparaison est le modèle de référence objectif, la valeur de seuil à dépasser pour qu'un écart produise un résultat insatisfaisant dépend bien entendu de la durée résiduelle prévue pour atteindre le positionnement correspondant au modèle de référence objectif.

La comparaison aboutit de préférence à une quantification des écarts, de préférence effectuée et présentée par un ordinateur. L'ordinateur présente de préférence les résultats de la comparaison en mettant en valeur les écarts les plus significatifs.

La comparaison peut être également visuelle. De préférence, un orthodontiste visualise et compare le modèle de référence actualisé et le modèle de comparaison. De préférence, un écran d'ordinateur présente une image mettant en évidence les écarts.

La décision qu'un écart est insatisfaisant ou pas peut dépendre d'une décision de l'orthodontiste et/ou d'un résultat d'un calcul effectué par ordinateur.

De préférence, la comparaison effectuée à l'étape e') conduit à des préconisations qui sont présentées à l'orthodontiste. En particulier, le procédé permet de déterminer finement le comportement de l'appareil orthodontique, et donc de déterminer le ou les facteurs qu'il convient de modifier pour optimiser son efficacité.

Dans un mode de réalisation, l'étape e') conduit à la génération d'un rapport précisant comment modifier la tension de l'arc de l'appareil orthodontique posé ou comment fabriquer une nouvelle gouttière.

Dans un mode de réalisation, l'étape e') conduit à la génération d'un score « actualisé » qui peut être comparé à un score de référence pour des traitements équivalents. De préférence, ces scores sont présentés au patient. Avantageusement, la réussite du traitement est ainsi présentée comme un objectif ludique, les scores motivant le patient, et notamment les enfants, à suivre au mieux le traitement afin d'augmenter leur score actualisé.

De préférence, l'étape e') conduit à la génération d'une information présentant au patient la situation et les différentes options qui se présentent. En cas d'écart insatisfaisant, le patient peut ainsi, par exemple, décider de rallonger son traitement ou choisir un nouveau modèle de référence objectif.

De préférence, les résultats de la comparaison sont transmis, de préférence en temps réel, au patient, de préférence sur son téléphone mobile. Avantageusement, le patient peut ainsi prendre conscience des conséquences d'une mauvaise observance du traitement.

**A l'étape f'),** si le résultat est insatisfaisant, on fabrique un appareil orthodontique de substitution, par modification de l'appareil orthodontique porté jusque-là ou par fabrication d'un nouvel appareil orthodontique.

L'étape f') est une étape classiquement exécutée lors d'un contrôle chez un orthodontiste.

La fabrication d'un appareil orthodontique de substitution n'est cependant réalisée que si le contrôle, effectué de préférence à distance, indique qu'elle est nécessaire.

Dans un mode de réalisation, la fabrication de l'appareil de substitution est effectuée à distance, de sorte que, lorsque le patient se présente chez l'orthodontiste, l'appareil orthodontique de substitution peut être immédiatement posé. En particulier, un arc orthodontique ou une gouttière peuvent être préparés à partir de l'évaluation effectuée à l'étape e'). L'arc orthodontique ou la gouttière peuvent être fabriqués dans un laboratoire à distance du cabinet de l'orthodontiste, puis être envoyés par courrier à ce dernier.

Dans un mode de réalisation, la gouttière peut être envoyée directement au patient, sans intervention de l'orthodontiste.

De préférence, à l'étape f'), on modifie le planning de traitement en fonction de la modification apportée à l'appareil orthodontique.

Dans un mode de réalisation, plusieurs appareils orthodontiques de substitution pouvant immédiatement remplacer l'appareil porté sont déterminés, de préférence en fonction de la vitesse de traitement souhaitée par le patient, une vitesse augmentée correspondant potentiellement à des douleurs supplémentaires. De préférence, le choix de la vitesse de traitement est offert au patient et l'appareil orthodontique de substitution à fabriquer est déterminé en conséquence.

Bien entendu, à l'étape f), si le résultat est insatisfaisant, on peut fabriquer, et de préférence envoyer à l'orthodontiste ou au patient, une série d'appareils orthodontiques de substitution.

En particulier on peut déterminer, comme au début du traitement avec des gouttières, une nouvelle série de gouttières adaptée pour que les dents atteignent une position correspondant au modèle de référence objectif. Cette détermination peut être réalisée de manière classique. De préférence cependant, une seule gouttière est fabriquée et, de préférence, envoyée au patient.

### Cycles de contrôle

De préférence, le procédé comporte plusieurs cycles de contrôle comportant des étapes c'), d'), e') et f'), de préférence réalisés chacun à un instant par exemple déterminé en début de traitement et/ou à la demande de l'orthodontiste et/ou à l'initiative du patient.

Deux cycles de contrôle consécutifs sont de préférence séparés d'une durée inférieure à 10 semaines, de préférence inférieure à 8 semaines, de préférence inférieure à 6 semaines, de préférence inférieure à 4 semaines, de préférence inférieure à 3 semaines, de préférence inférieure à 2 semaines.

De préférence, le nombre de cycles de contrôle lors du traitement est supérieur à 1, supérieur à 3, supérieur à 5, supérieur à 10, supérieur à 15, voire supérieur à 20.

De préférence, un rappel est envoyé au patient, par exemple par SMS, pour qu'il effectue un cycle de contrôle.

Dans un mode de réalisation préféré, le modèle de comparaison d'une étape d') d'un cycle de contrôle est déterminé à partir du modèle de référence actualisé réalisé lors de l'étape c') d'un cycle de contrôle précédent, en particulier du cycle de contrôle immédiatement précédent, ou à partir de plusieurs modèles de référence actualisés réalisés lors d'étapes c') de cycles de contrôle antérieurs, par exemple par extrapolation.

La figure 13 illustre un procédé qui comporte plusieurs cycles de contrôle. Elle représente les courbes des évolutions temporelles théorique (C_{T}) et réelle (C_{R}) d'un paramètre de quantité de mouvement d'une dent, et plus précisément, l'évolution temporelle de la position d'un point M d'une dent, par exemple du barycentre de cette dent, selon l'axe Ox), en pourcentage par rapport à la valeur souhaitée en fin de traitement.

Un cycle de contrôle a été effectué chaque semaine.

Chaque semaine, un point de chaque courbe a donc été déterminé, au moyen d'un modèle de comparaison établi à partir d'un modèle dynamique simulant une évolution anticipée des dents du modèle de référence initial (point de la courbe C_{T}), et d'un modèle de référence actualisé établi à partir de photographies prises par le patient cette semaine (point de la courbe C_{R}).

Les premiers cycles de contrôle ont permis de constater très rapidement une dérive. L'appareil orthodontique avait tendance à déplacer trop rapidement le point M. Cette information a été présentée à l'orthodontiste, avec des préconisations relatives à la façon de modifier l'appareil orthodontique, par exemple pour lui préciser qu'il convenait de réduire la tension exercée par l'arc orthodontique entre deux dents déterminées.

Un rendez-vous entre le patient et l'orthodontiste a été convenu la troisième semaine. L'appareil orthodontique a été modifié pendant ce rendez-vous. Cette modification a conduit au rapprochement des deux courbes les semaines suivantes.

Après cinq semaines, les deux courbes se sont croisées. L'appareil orthodontique a donc de nouveau été modifié, à la semaine 8. Les deux courbes se sont alors lentement rapprochées, jusqu'à la semaine 18.

Elles ont ensuite à nouveau divergé sensiblement, ce qui a conduit à modifier encore l'appareil orthodontique, en semaine 20.

A l'issue de la semaine 25, l'objectif était sensiblement atteint, dans le délai prévu initialement. Le traitement est alors terminé.

Dans un mode de réalisation, le patient reçoit, par exemple sur son téléphone, des informations lui permettant de comparer le modèle de référence actualisé et le modèle de référence objectif, par exemple des représentations graphiques de ces deux modèles. Si modèle de référence objectif représente les dents à la fin du traitement (« set-up final »), il peut ainsi décider lui-même de la fin du traitement.

La courbe de l'évolution temporelle théorique peut être déterminée, dans sa totalité, dès le début du traitement, en tenant compte des modifications de l'appareil orthodontique prévues pendant le traitement.

Cette courbe peut être également recalculée en fonction des modifications apportées à l'appareil orthodontique et qui n'étaient pas prévues initialement. Par exemple, si la modification de l'appareil orthodontique effectuée en semaine 3 n'était pas prévue initialement, le modèle prédictif utilisé pour établir le modèle de comparaison utilisé après la semaine 3, et donc la courbe de l'évolution temporelle théorique, ont de préférence été modifiés pour tenir compte de cette modification imprévue. Le modèle prédictif a ainsi avantageusement tenu compte d'une évolution beaucoup plus lente du déplacement du point M selon l'axe Ox) que celle qui avait été initialement anticipée.

Comme cela apparaît clairement à présent, le procédé d'adaptation permet notamment un diagnostic de la situation, voire la fabrication d'un appareil orthodontique de substitution, sans que le patient ait à se déplacer chez un orthodontiste.

### Description détaillée d'un procédé d'évaluation du comportement d'un appareil orthodontique

La multiplication des cycles de contrôle permet avantageusement d'acquérir de nombreuses données permettant d'établir des corrélations entre des paramètres de l'appareil orthodontique, son comportement et des configurations des dents.

De préférence, les valeurs des paramètres de l'appareil orthodontique utilisé initialement, mais aussi, à chaque cycle de contrôle, les paramètres de l'appareil orthodontique de substitution sont enregistrés dans un ordinateur. Les paramètres de l'appareil orthodontique comprennent des paramètres intrinsèques, comme le matériau ou la forme d'une gouttière, ou le matériau et le diamètre d'un arc orthodontique. Ils comprennent également des paramètres extrinsèques, résultant de son utilisation, comme les points d'attache de l'arc orthodontique sur les dents ou la tension de l'arc orthodontique.

Cet ordinateur a également accès aux différents modèles tridimensionnels établis dans le cadre du traitement.

Un module d'analyse permet de comparer toutes les données collectées, selon des méthodes d'analyse statistique conventionnelles. Il en résulte une meilleure connaissance du comportement de l'appareil orthodontique, en fonction des valeurs de ses paramètres et des configurations des dents correspondantes.

De préférence, les données collectées sont issues de plusieurs traitements, de préférence de plus de 10, plus de 100, plus de 1 000, plus de 10 000, plus de 100 000 traitements.

La précision de l'analyse statistique en est améliorée.

Les données permettent ainsi d'observer que, dans des configurations similaires des dents, un type d'appareil orthodontique ou un paramètre d'un appareil orthodontique est particulièrement efficace pour assurer un déplacement dans une direction particulière.

Elles permettent donc de modéliser le comportement de l'appareil orthodontique avec une grande précision, et ainsi de mieux évaluer, dans une situation particulière, son comportement futur.

Dans un mode de réalisation, l'analyse des données permet de quantifier la difficulté d'un traitement, par comparaison de ce traitement avec des traitements équivalents. L'orthodontiste peut ainsi informer le patient sur les chances de succès et sur la nature des difficultés potentielles. L'orthodontiste peut également avantageusement décider de modifier le traitement initialement envisagé, notamment en modifiant les set-ups intermédiaires.

Les connaissances ainsi acquises peuvent donc être utilisées pour optimiser
- la conception des appareils orthodontiques, et/ou
- le choix d'un appareil orthodontique pour un traitement particulier, et/ou
- l'établissement d'un diagnostic par l'orthodontiste, et/ou
- la fabrication d'un appareil orthodontique de substitution, et/ou
- la détermination d'un modèle prédictif pour fabriquer le modèle de comparaison.

Cette optimisation peut avantageusement amener l'orthodontiste ou le concepteur d'appareils orthodontiques à prendre en compte un paramètre de l'appareil orthodontique classiquement considéré comme négligeable.

Avantageusement, des corrélations entre des paramètres de l'appareil orthodontique et son comportement peuvent être établies sans qu'il soit nécessaire de comprendre théoriquement cette corrélation, c'est-à-dire de comprendre comment ces paramètres produisent ce comportement.

### Description détaillée d'un procédé de contrôle de la forme des dents

On décrit également un procédé de contrôle de la forme de dents d'un patient. A l'étape a), la définition des modèles de dents n'est cependant pas indispensable pour ce contrôle.

De préférence, à l'étape e), on recherche, pour chaque image actualisée, un modèle de référence actualisé correspondant à la forme des dents lors de l'acquisition de l'image actualisée, la recherche étant de préférence effectuée au moyen d'une méthode métaheuristique, de préférence évolutionniste, de préférence par recuit simulé, en particulier au moyen d'une des méthodes métaheuristiques décrites précédemment.

De préférence, comme décrit pour l'étape e), cette recherche comporte deux boucles d'optimisation imbriquées.

Lors de la première opération d'optimisation, on optimise d'abord, dans un modèle de référence à tester qui est initialement le modèle de référence initial, des conditions d'acquisition virtuelles qui correspondent au mieux aux conditions d'acquisition réelles. En particulier, on recherche la position virtuelle de l'appareil d'acquisition par rapport au modèle de référence à tester qui offre la vue de ce modèle de référence à tester, c'est-à-dire l'image de référence, qui est la plus proche de l'image actualisée.

De préférence, comme décrit précédemment, on recherche également une calibration virtuelle susceptible de correspondre à la calibration réelle de l'appareil d'acquisition lors de la capture de l'image actualisée.

Lors de la deuxième opération d'optimisation, on modifie ensuite le modèle de référence à tester, on relance le première l'opération d'optimisation, puis on recommence ces deux opérations jusqu'à trouver le modèle de référence à tester et la position virtuelle de l'appareil d'acquisition qui permettent d'obtenir l'image de référence qui est la plus proche de l'image actualisée.

Ces opérations sont similaires à celles décrites pour le procédé de contrôle du positionnement des dents et les caractéristiques optionnelles de ce dernier contrôle sont optionnellement applicables.

Cependant, suivant le procédé de contrôle du positionnement des dents décrit précédemment, la modification du modèle de référence résulte d'un déplacement d'un ou plusieurs modèles de dents. Aucune déformation des modèles de dent ou du modèle de référence initial n'est nécessaire.

Pour contrôler la forme des dents, la modification du modèle de référence résulte d'une modification de la forme du modèle de référence à tester, en particulier d'un ou plusieurs modèles de dents. Aucun déplacement des modèles de dent n'est nécessaire.

Bien entendu, il est préférable d'effectuer les deux types de modifications du modèle de référence à tester afin de déterminer un modèle de référence actualisé qui tienne compte à la fois du déplacement des dents et de leur déformation.

Par exemple, on peut mettre en œuvre une troisième opération d'optimisation portant sur le déplacement des dents et encadrant les deux premières opérations d'optimisation, la deuxième optimisation portant sur la forme des modèles de dent ou du modèle de référence à tester. Il est également possible de ne mettre en œuvre que les deux premières opérations d'optimisation, en modifiant, éventuellement simultanément, la forme et la position des modèles de dents lors de la deuxième opération d'optimisation.

On peut aussi mettre en œuvre une troisième opération d'optimisation portant sur la forme des modèles de dent ou du modèle de référence à tester et encadrant les deux premières opérations d'optimisation, la deuxième opération d'optimisation portant sur le déplacement des modèles de dents. Par exemple, on recherche d'abord un modèle de référence actualisé « à tester » tenant compte, au mieux, du déplacement des modèles de dent, le modèle de référence actualisé à tester correspondant au modèle de référence actualisé d'une étape e) d'un procédé de contrôle du positionnement des dents décrit précédemment, puis on recherche si une déformation du modèle de référence actualisé à tester peut conduire à une meilleure concordance (« matching ») avec l'image actualisée. Pour cette recherche, on déforme le modèle de référence actualisé à tester, on recommence les deux premières opérations d'optimisation, puis, en fonction de la concordance obtenue, on arrête la recherche ou on la poursuit en effectuant une nouvelle déformation du modèle de référence actualisé à tester et en relançant une exécution des deux premières opérations d'optimisation.

De préférence, la première opération d'optimisation et/ou la deuxième opération d'optimisation et/ou la troisième opération d'optimisation, de préférence la première opération d'optimisation et la deuxième opération d'optimisation et la troisième opération d'optimisation mettent en œuvre une méthode métaheuristique, de préférence évolutionnistes, de préférence un recuit simulé, en particulier d'une des méthodes métaheuristiques citées précédemment.

De préférence, pour contrôler la déformation des dents, l'étape e) comporte
- une première opération d'optimisation permettant de rechercher des conditions d'acquisition virtuelles correspondant au mieux aux conditions d'acquisition réelles dans un modèle de référence à tester déterminé à partir du modèle de référence initial, et
- une deuxième opération d'optimisation permettant de rechercher, en testant une pluralité de dits modèles de référence à tester, le modèle de référence correspondant au mieux à la forme des dents du patient lors de l'acquisition de l'image actualisée à l'étape b), de préférence correspondant au mieux à la forme et au positionnement des dents du patient lors de l'acquisition de l'image actualisée à l'étape b).

De préférence, une première opération d'optimisation est effectuée pour chaque test d'un modèle de référence à tester lors de la deuxième opération d'optimisation.

L'étape e) peut notamment comporter les étapes suivantes :
e'1) définition du modèle de référence à tester comme étant le modèle de référence initial puis,
e'2) suivant les étapes suivantes, test de conditions d'acquisition virtuelles avec le modèle de référence à tester afin d'approximer finement lesdites conditions d'acquisition réelles ;
   e'21) détermination de conditions d'acquisition virtuelles à tester;
   e'22) réalisation d'une image de référence bidimensionnelle du modèle de référence à tester dans lesdites conditions d'acquisition virtuelles à tester ;
   e'23) traitement de l'image de référence pour réaliser au moins une carte de référence représentant ladite information discriminante ;
   e'24) comparaison des cartes actualisée et de référence de manière à déterminer une valeur pour une première fonction d'évaluation, ladite valeur pour la première fonction d'évaluation dépendant des différences entre lesdites cartes actualisée et de référence et correspondant à une décision de poursuivre ou de stopper la recherche de conditions d'acquisition virtuelles approximant lesdites conditions d'acquisition réelles avec plus d'exactitude que lesdites conditions d'acquisition virtuelles à tester déterminées à la dernière occurrence de l'étape e'21) ;
   e'25) si ladite valeur pour la première fonction d'évaluation correspond à une décision de poursuivre ladite recherche, reprise à l'étape e'21) en modifiant les conditions d'acquisition virtuelles à tester ;
e'3) sinon détermination d'une valeur pour une deuxième fonction d'évaluation, ladite valeur pour la deuxième fonction d'évaluation dépendant des différences entre les cartes actualisée et de référence dans les conditions d'acquisition virtuelles approximant au mieux lesdites conditions d'acquisition réelles et résultant de la dernière occurrence de l'étape e'2), ladite valeur pour la deuxième fonction d'évaluation correspondant à une décision de poursuivre ou de stopper la recherche d'un modèle de référence approximant la forme, et éventuellement le positionnement des dents lors de l'acquisition de l'image actualisée avec plus d'exactitude que ledit modèle de référence à tester utilisé à la dernière occurrence de l'étape e'2), et
   si ladite valeur pour la deuxième fonction d'évaluation correspond à une décision de poursuivre ladite recherche, modification du modèle de référence à tester par déformation d'un ou plusieurs modèles de dents, ou du modèle de référence à tester, et éventuellement par déplacement d'un ou plusieurs modèles de dents, puis reprise à l'étape e'2).

A l'issue de ce procédé, on aboutit à un modèle de référence à tester, dit « modèle de référence actualisé », qui correspond au modèle de référence initial déformé pour correspondre au mieux à l'image actualisée.

La comparaison des modèles de référence initial et actualisé comprend la comparaison des coordonnées spatiales des points des surfaces définies par ces deux modèles de référence. On peut ainsi en déduire les modifications de forme éventuelles entre l'étape a) et l'étape b).

De préférence, on produit une cartographie de la dent faisant apparaître les changements de forme. De préférence, comme représenté sur la figure 8, la couleur d'une zone de la cartographie est fonction de l'ampleur de changement de forme de cette zone. La cartographie de la figure 8 fait en particulier apparaître une zone 16 indiquant une rupture d'une dent.

Bien entendu, le procédé de contrôle de la forme de dents d'un patient peut être utilisé pour détecter une addition ou une soustraction de matière, mais aussi une déformation à volume constant. Ce procédé permet également de détecter des déplacements de dents, même sans modèle de dents. Cependant, en l'absence de modèle de dent, il ne permet pas de distinguer les déformations des dents d'une part et les déplacements des dents d'autre part.

### Description détaillée d'un procédé de contrôle de l'aspect des dents

Le contrôle de l'évolution de la couleur de dents à partir de photographies prises dans des positions de l'appareil photo ou dans des environnements lumineux différents montre que cette comparaison ne permet pas d'évaluer une évolution de l'aspect de ces dents.

Un tel contrôle de la couleur des dents nécessite donc des précautions particulières, notamment pour définir avec précision la position de l'appareil photo et son environnement lumineux.

Il existe donc un besoin pour un procédé permettant de contrôler la couleur, et plus généralement une propriété d'aspect, des dents de manière plus simple, et notamment en évitant ces précautions particulières.

Un objectif est de répondre à ce besoin.

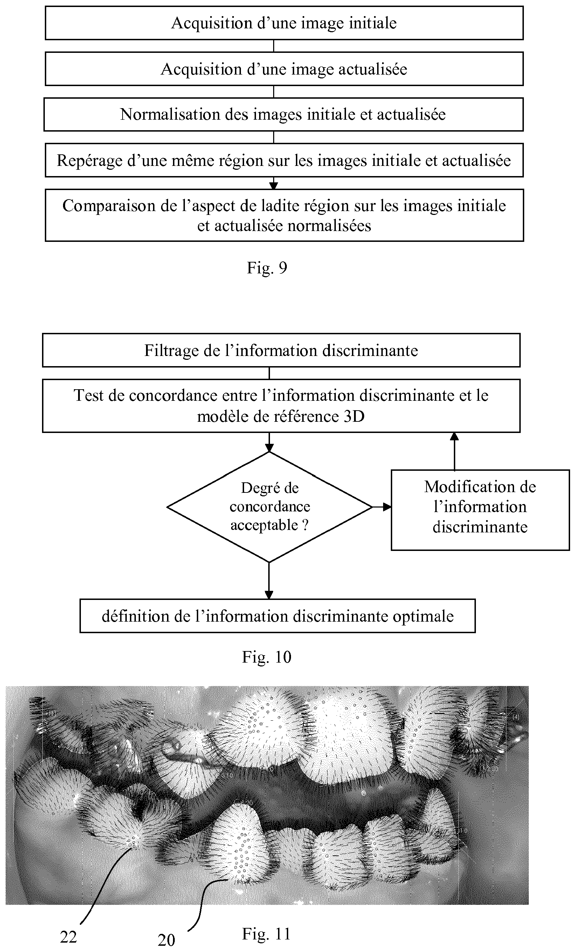

On décrit un procédé de contrôle d'une propriété d'aspect de dents d'un patient, ledit procédé comportant les étapes A. à E. décrites ci-dessus, représentées sur la figure 9.

Comme on le verra plus en détail dans la suite de la description, ce procédé permet d'évaluer si l'aspect, en particulier la couleur d'une ou plusieurs des dents a été modifié, même lorsque les conditions d'acquisition des photographies de dents d'un patient ne sont pas prédéfinies, par exemple parce que les photographies ont été prises dans un environnement lumineux ou dans une position quelconque de l'appareil d'acquisition, en particulier par le patient.

Par «propriété d'aspect », on entend une propriété relative à l'apparence. La propriété d'aspect peut être en particulier choisie dans le groupe constitué par la couleur, l'opalescence, la fluorescence, la brillance (« gloss »), la transparence, et les combinaisons de ces propriétés.

Par « aspect », on entend une valeur ou un ensemble de valeurs permettant de quantifier une propriété d'aspect. Sauf indication contraire, les « aspects » mentionnés dans la présente description concernent la propriété d'aspect que le procédé permet de contrôler.

### Etapes A. et B.

Les étapes A. et B. peuvent être réalisées comme l'étape b) décrite ci-dessus.

Les étapes A. et B. sont de préférence réalisées par le patient ou un proche du patient, mais peuvent être réalisées par un dentiste.

L'intervalle de temps entre ces étapes peut être celui décrit ci-dessus entre les étapes a) et b), par exemple supérieur à 1 semaine, à 2 semaines, à 1 mois ou à 3 mois.

Le premier appareil d'acquisition peut être identique ou différent du deuxième appareil d'acquisition. Il peut être un appareil d'acquisition choisi parmi ceux pouvant être utilisés pour l'étape b), notamment un appareil photo ou un téléphone mobile.

De préférence, les acquisitions aux étapes A. et/ou B. sont effectuées en ayant recours à un flash. Les résultats en sont améliorés. De préférence, l'image initiale et/ou actualisée est surexposée.

Les jauges de référence utilisées pour chacune des étapes A. et B. présentent un même aspect. De préférence, lors de chacune des étapes, elles sont disposées dans la même position par rapport aux dents du patient.

De préférence, des écarteurs dentaires sont utilisés pour chacune des étapes A. et B.. Ces écarteurs peuvent être identiques ou différents. De préférence la jauge de référence est portée par un écarteur pour au moins une des étapes A. et B., de préférence pour chacune des étapes A. et B.. De préférence, même si les écarteurs utilisés pour chacune des étapes A. et B. sont différents, les jauges de référence sont disposées sur les écarteurs dans la même position par rapport à l'ouverture 14 de l'écarteur qui laisse apparaître les dents du patient (figure 5a).

De préférence, la jauge de référence est disposée sur l'écarteur de manière à être à proximité des dents dont la propriété d'aspect doit être contrôlée. De préférence, la jauge de référence est disposée à moins de 3 cm, de préférence moins de 2 cm, de préférence moins de 1 cm de la partie de l'écarteur destinée à être introduite dans la bouche du patient.

De préférence, chaque écarteur comporte plusieurs jauges de référence, identiques ou différentes. De préférence, plusieurs jauges de référence différentes d'un même écarteur présentent des aspects différents. Les conclusions tirées de la comparaison des images normalisées peuvent être avantageusement plus riches.

Une jauge de référence peut être par exemple un point repérable sur l'écarteur et dont l'aspect est connu, par exemple dont les paramètres de couleur L^{∗}, et/ou a^{∗} et/ou b^{∗}, mesurés selon la norme NF ISO 7724, sont connus. La jauge de référence peut être notamment une marque de repérage d'un écarteur tel que décrit ci-dessus.

Les conditions d'acquisition précisent la position dans l'espace et/ou l'orientation de l'appareil d'acquisition par rapport à l'écarteur.

Pour améliorer la précision du contrôle d'aspect, il est préférable que les conditions d'acquisition soient sensiblement les mêmes aux étapes A. et B.. Par exemple, il est préférable que les deux images soient prises sensiblement de face. De préférence, l'appareil d'acquisition d'images utilisé pour au moins une, de préférence pour chacune des étapes A. et B. comporte des moyens détrompeurs facilitant son positionnement par rapport au patient avant l'acquisition de l'image.

Les moyens détrompeurs interagissent de préférence avec des marques de repérage disposées sur l'écarteur. De préférence, l'appareil d'acquisition est programmé de manière à, en temps réel, repérer les marques de repérage sur l'écarteur, analyser leurs positions relatives ou leurs dimensions et, en conséquence, informer l'utilisateur de l'appareil d'acquisition afin qu'il modifie en conséquence la position de l'appareil d'acquisition par rapport aux dents du patient.

Ces moyens détrompeurs peuvent présenter une ou plusieurs des caractéristiques des moyens détrompeurs décrits ci-dessus pour l'étape b).

De préférence, pour au moins une, de préférence pour chacune des étapes A. et B. on utilise un dit kit d'acquisition et, de préférence, un procédé d'acquisition comportant des étapes (a) à (e). De préférence, les conditions d'acquisition cibles sont les mêmes dans la mémoire des premier et deuxième appareils d'acquisition de sorte que l'appareil d'acquisition guide son utilisateur pour que les images initiale et actualisée soient prises dans des conditions d'acquisition sensiblement identiques.

De préférence, les conditions d'acquisition cibles sont déterminées en fonction des dents dont la propriété d'aspect est à contrôler. Par exemple, les conditions d'acquisition cibles correspondent de préférence à une prise d'image face au patient pour un contrôle portant sur une incisive et elles correspondent de préférence à une prise d'image latérale pour un contrôle portant sur une molaire.

### Etape C.

L'étape C. consiste à normaliser, c'est-à-dire «corriger» l'image initiale et/ou l'image actualisée de manière que, après correction, les représentations de la jauge de référence sur ces images présentent un même aspect. La jauge de référence n'ayant pas changé d'aspect entre les étapes A. et B., les différences d'aspect éventuelles présentées par les représentations des dents sur les images initiale et actualisée normalisées correspondent donc à des différences d'aspect réelles sur lesdites dents.

On recherche d'abord la jauge de référence sur l'image initiale et sur l'image actualisée. Une simple analyse d'image suffit à cet effet.

La normalisation peut être effectuée sur l'image initiale seulement afin de modifier la représentation de la jauge de référence pour que son aspect soit identique à celui de la représentation de ladite jauge de référence sur l'image actualisée. La normalisation peut être alternativement effectuée sur l'image actualisée seulement afin de modifier la représentation de la jauge de référence pour que son aspect soit identique à celui de la représentation de ladite jauge de référence sur l'image initiale. Enfin la normalisation peut être effectuée sur l'image actualisée et sur l'image initiale afin de modifier les représentations des jauges de référence pour que leurs aspects soient identiques à celui d'une jauge étalon.

La normalisation d'une image est une technique bien connue dans le domaine du traitement d'images. La balance de blancs est un exemple de normalisation d'images.

### Etape D.

Avant ou après l'étape C., il convient d'identifier, sur chacune des images initiale et actualisée, une région des dents dont on souhaite évaluer l'évolution d'aspect.

L'utilisation de marques de repérage ou de jauges de référence est possible, mais reste imprécise. De préférence, les images initiale et actualisée sont analysées afin de représenter une information discriminante, du type de celles décrites ci-dessus, par exemple le contour des dents.

L'analyse des images initiale et actualisée peut comporter une ou plusieurs caractéristiques de l'étape c), notamment relatives à la nature de l'information discriminante et au traitement pour déterminer l'information discriminante. L'information discriminante est de préférence optimisée au moyen d'un procédé d'optimisation comportant des étapes C1 à C3.

On recherche alors des informations discriminantes communes aux deux images initiale et actualisée.

Les informations discriminantes communes aux deux images initiale et actualisée peuvent alors servir de référentiel pour localiser ladite région sur ces deux images. Par exemple, le contour des gencives peut présenter une succession de « pointes » entre les dents. Ce contour dépend des dents considérées et peut donc servir de référentiel.

Dans un mode de réalisation perfectionné, les images initiale et actualisée sont repérées par rapport à un modèle de référence, de préférence réalisé conformément à l'étape a) (modèle de référence initial) ou résultant de la mise en œuvre d'un dit procédé de contrôle de la forme et/ou du positionnement des dents (modèle de référence actualisé).

Ce repérage peut être effectué comme décrit ci-dessus pour repérer l'image actualisé dans le cadre des procédés de contrôle de la forme et/ou du positionnement des dents. A la différence de ces procédés, la modification du modèle de référence initial pour parvenir au modèle de référence actualisé est cependant optionnelle.

Pour repérer une image par rapport au modèle de référence, il suffit de rechercher les conditions d'acquisition virtuelles dans lesquelles l'appareil d'acquisition aurait acquis ladite image en observant ledit modèle de référence. Cette recherche est de préférence effectuée au moyen d'une méthode métaheuristique, telle que celles décrites ci-dessus.

Pour cette recherche, on utilise de préférence un procédé d'évaluation des conditions d'acquisition réelles décrit ci-après. Ce procédé est de préférence mis en œuvre pour chacune des images initiale et actualisée au moyen du modèle de référence. Il permet de « projeter » ces images sur le modèle de référence et donc de repérer un point de ces images sur le modèle de référence.

Une région des dents dont on souhaite évaluer l'évolution d'aspect peut ainsi être identifiée avec une grande précision sur chacune des images initiale et actualisée.

### Etape E.

Il est alors possible de mesurer les aspects de ladite région sur chacune des images initiale et actualisée et de les comparer afin de détecter et évaluer des différences de la propriété d'aspect.

Un dit procédé de contrôle de l'aspect des dents peut être utilisé à des fins thérapeutiques ou non thérapeutiques. Il peut en particulier être utilisé pour :
- détecter et/ou mesurer un changement de couleur des dents ou l'apparition et/ou l'évolution de tâches sur les dents, ou détecter et/ou mesurer une calcification des dents;
- vérifier les effets sur l'aspect des dents d'une habitude alimentaire ou d'une hygiène alimentaire ou d'un traitement, par exemple d'un traitement de blanchiment, ou d'un produit, notamment d'un dentifrice, en particulier pour blanchir les dents ou pour lutter contre la calcification ou l'apparition de tâches.

Par exemple, un dit procédé de contrôle de l'aspect des dents peut être utilisé pour vérifier les effets sur l'aspect des dents du mâchage d'un chewing-gum ou de l'ingestion de café ou de thé, ou de la consommation de tabac ou de drogue, ou du brossage des dents.

Dans un mode de réalisation préféré, il suffit au patient de prendre régulièrement des photographies avec son téléphone mobile pour constituer des images actualisées. De préférence, grâce à un applicatif chargé dans ce téléphone, il peut ensuite comparer les aspects des dents sur ces photographies.

Dans un mode de réalisation, l'applicatif normalise les photographies afin de les rendre comparables, puis propose une visualisation dynamique des photographies corrigées, par exemple sous la forme d'un diaporama ou d'un film.

### Description détaillée d'un procédé d'évaluation de conditions d'acquisition réelles

Notamment pour la mise en œuvre d'un dit procédé de contrôle de la forme, du positionnement et/ou de l'aspect de dents ou pour optimiser la qualité d'une l'information discriminante, on décrit un procédé d'évaluation, à partir d'une image bidimensionnelle des arcades dentaires d'un patient, dite « image acquise », des conditions d'acquisition réelles (position de l'appareil d'acquisition dans l'espace, orientation de cet appareil, et, de préférence, calibration de l'appareil d'acquisition) de ladite image acquise, ledit procédé comportant les étapes suivantes :
001) réalisation d'un modèle de référence tridimensionnel numérique d'au moins une partie d'une arcade du patient, de préférence d'une arcade, de préférence de deux arcades du patient,
002) analyse de l'image acquise et réalisation d'une carte relative à une information discriminante, dite « carte acquise » ;
003) recherche des conditions d'acquisition virtuelles approximant, de manière optimale, lesdites conditions d'acquisition réelles, suivant les étapes 01) à 05) suivantes :
   01) optionnellement, détermination de conditions d'acquisition virtuelles grossières approximant lesdites conditions d'acquisition réelles, de préférence par analyse de la représentation, sur l'image acquise, d'un écarteur utilisé lors de l'acquisition de l'image acquise ;
   02) détermination de conditions d'acquisition virtuelles à tester;
   03) réalisation d'une image de référence bidimensionnelle du modèle de référence observé dans les conditions d'acquisition virtuelles à tester ;
   04) traitement de l'image de référence pour réaliser au moins une carte de référence représentant de ladite information discriminante ;
   05) comparaison des cartes acquise et de référence de manière à déterminer une valeur pour une fonction d'évaluation, ladite valeur pour la fonction d'évaluation dépendant des différences entre lesdites cartes acquise et de référence et correspondant à une décision de poursuivre ou de stopper la recherche de conditions d'acquisition virtuelles approximant les conditions d'acquisition réelles avec plus d'exactitude que lesdites conditions d'acquisition virtuelles à tester ;
   06) si ladite valeur pour la fonction d'évaluation correspond à une décision de poursuivre ladite recherche, modification desdites conditions d'acquisition virtuelles à tester, puis reprise à l'étape 03) ; sinon, évaluation des conditions d'acquisition réelles par lesdites conditions d'acquisition virtuelles à tester.

De préférence, les conditions d'acquisition réelles à évaluer comprennent un ou plusieurs des paramètres de calibration suivants : l'ouverture de diaphragme, le temps d'exposition, le temps d'exposition, la distance focale et la sensibilité.

Dans le procédé d'évaluation, la modification apportée aux conditions d'acquisition virtuelles à tester à l'étape 06) est de préférence effectuée au moyen d'une méthode métaheuristique, de préférence évolutionniste, de préférence par recuit simulé, de préférence au moyen d'une des méthodes métaheuristiques citées précédemment.

Un tel procédé d'évaluation est de préférence utilisé à chaque fois qu'il est nécessaire d'évaluer les conditions d'acquisition réelles d'une image. Cette image, qui peut en particulier être une image actualisée acquise lors d'une étape b) ou B. ou une image initiale acquise lors d'une étape A., est appelée « image acquise ».

La réalisation du modèle de référence à l'étape 001) peut comporter une ou plusieurs des caractéristiques, même optionnelles, de l'étape a).

L'acquisition de l'image acquise peut comporter une ou plusieurs des caractéristiques, même optionnelles, de l'étape b). De préférence, elle met en œuvre un dit kit d'acquisition, et de préférence un dit procédé d'acquisition.

L'étape 01) de détermination des conditions d'acquisition virtuelles grossières peut comporter une ou plusieurs des caractéristiques, même optionnelles, de l'étape c).

Les étapes 02) à 06) peuvent comporter une ou plusieurs des caractéristiques, même optionnelles, des étapes e21) à e25), respectivement.

### Description détaillée d'un procédé d'optimisation d'une information discriminante

Un dit procédé d'optimisation ou de "sélection" d'une information discriminante est destiné à améliorer la fiabilité d'une information discriminante initiale extraite d'une image bidimensionnelle des arcades dentaires d'un patient, ou « image acquise », notamment d'une image initiale issue d'une étape A. ou d'une image actualisée issue d'une étape B. ou b), acquise dans des conditions d'acquisition réelles. "L'optimisation" de l'information discriminante est donc une sélection d'informations discriminantes, suivant une démarche itérative, de manière à sélectionner de l'image l'information discriminante la plus pertinente pour contrôler au mieux le positionnement et/ou la forme des dents du patient.

Ce procédé repose sur un modèle de référence tridimensionnel numérique d'au moins une partie d'une arcade du patient, en particulier d'un modèle de référence initial d'une étape a).

Comme illustré sur la figure 10, il comporte les étapes suivantes :
C1. évaluation de la qualité de l'information discriminante initiale et d'un seuil de qualité, filtrage de manière à ne retenir que de l'information discriminante initiale, de préférence toute l'information discriminante initiale présentant une qualité supérieure au seuil de qualité, et définition de « l'information discriminante à tester » comme étant l'information discriminante retenue ;
C2. test de la concordance (en anglais « matching ») entre l'information discriminante à tester et le modèle de référence ;
C3. en fonction du résultat et d'une fonction d'évaluation du résultat du test :
   - ajout d'information discriminante non retenue à l'information discriminante à tester et/ou suppression d'information discriminante dans l'information discriminante à tester, puis reprise à l'étape C2. ou,
   - définition de l'information discriminante optimale comme étant l'information discriminante à tester.

L'information discriminante peut être notamment une quelconque des informations discriminantes décrites précédemment. A titre d'exemple, l'information discriminante peut être une information de contour.

L'information discriminante initiale résulte classiquement de l'analyse de l'image acquise, comme décrit pour l'étape c).

Les procédés décrits qui utilisent une telle information discriminante initiale mettent en œuvre des optimisations qui fournissent de meilleurs résultats si l'information discriminante est à la fois abondante et de bonne qualité. Un objectif du procédé d'optimisation est donc d'améliorer la qualité de l'information discriminante initiale.

A l'étape C1., on évalue la qualité de l'information discriminante initiale. Dans l'exemple d'un contour, l'analyse du contraste fournit par exemple des informations plus ou moins fiables : une zone de fort contraste peut être assimilée à une zone correspondant à un contour avec une probabilité élevée et la qualité des points de cette zone sera donc élevée. Au contraire, une zone de faible contraste, par exemple une zone floue, peut être assimilée à une zone correspondant à un contour avec une probabilité faible et la qualité des points de cette zone sera donc faible. Dans cet exemple, la probabilité pour un point de l'image acquise d'appartenir au contour peut être choisie comme indicateur de la « qualité » de l'information discriminante.

Un seuil de qualité est utilisé pour filtrer l'information discriminante initiale. Si le seuil de qualité est élevé, l'information discriminante retenue suite au filtrage sera peu nombreuse, mais très fiable. Si le seuil de qualité est faible, l'information discriminante retenue sera abondante, mais peu fiable. Dans l'exemple d'une information de contour, l'analyse de l'image conduira alors à retenir des « faux » points de contour, c'est-à-dire, des points qui, du fait de l'analyse, seront considérés par erreur comme appartenant au contour des dents et des gencives.

Dans un mode de réalisation préféré, le seuil de qualité est élevé afin de ne retenir que de l'information discriminante à tester très fiable.

A l'étape C2., on teste la concordance, c'est-à-dire que l'on détermine un degré de concordance, entre l'information discriminante à tester et le modèle de référence.

De préférence, on réalise une carte « acquise » de l'information discriminante à tester résultant du traitement de l'image acquise.

De préférence, on procède ensuite suivant les étapes 01) à 06), et en particulier les étapes suivantes :
02) détermination de conditions d'acquisition virtuelles à tester;
03) réalisation d'une image de référence bidimensionnelle du modèle de référence observé dans les conditions d'acquisition virtuelles à tester ;
04) traitement de l'image de référence pour réaliser au moins une carte de référence représentant de l'information discriminante ;
05) comparaison des cartes acquise et de référence de manière à déterminer une valeur pour une fonction d'évaluation, ladite valeur pour la fonction d'évaluation dépendant des différences entre lesdites cartes acquise et de référence et correspondant à une décision de poursuivre ou de stopper la recherche de conditions d'acquisition virtuelles approximant les conditions d'acquisition réelles avec plus d'exactitude que lesdites conditions d'acquisition virtuelles à tester ;
06) si ladite valeur pour la fonction d'évaluation correspond à une décision de poursuivre ladite recherche, modification desdites conditions d'acquisition virtuelles à tester, puis reprise à l'étape 03) ; sinon, évaluation des conditions d'acquisition réelles par lesdites conditions d'acquisition virtuelles à tester.

A l'étape 04), le traitement de l'image de référence permet de réaliser une carte de référence représentant de ladite information discriminante. Les critères de sélection de l'information discriminante représentée sur la carte de référence peuvent être identiques ou différents de ceux utilisés pour sélectionner l'information discriminante à tester.

De préférence, l'information discriminante représentée sur la carte de référence est sélectionnée avec les mêmes critères que l'information discriminante à tester.

Dans l'exemple d'un contour, le traitement de l'image de référence peut consister à retenir les points de l'image de référence correspondant à un contour avec une probabilité élevée. La probabilité pour un point de l'image de référence d'appartenir au contour peut être déterminée comme pour le traitement de l'image acquise et également servir d'indicateur de la qualité de l'information discriminante. Le seuil de qualité peut être également identique à celui utilisé pour le traitement de l'image acquise. Le contour représenté sur la carte de référence est alors similaire à celui représenté sur la carte acquise, et en particulier présente une longueur sensiblement identique.

Les étapes 01) à 06) permettent de déterminer, avec le modèle de référence, des conditions d'acquisition virtuelles approximant les conditions d'acquisition réelles de l'image acquise. L'observation du modèle de référence dans ces conditions d'acquisition virtuelles fournit donc une vue qui correspond au mieux à l'image acquise. La recherche des conditions d'acquisition virtuelles se fonde cependant sur l'information discriminante à tester. Le degré de correspondance dépend donc de l'information discriminante à tester. Plus la qualité et la quantité de l'information discriminante à tester sont élevées, meilleur est le degré de correspondance entre la vue du modèle de référence dans les conditions d'acquisition virtuelles et l'image acquise, et plus élevé est le degré de concordance entre l'information discriminante à tester et le modèle de référence.

Le degré de concordance peut être par exemple mesuré par l'inverse de l'écart entre
- la carte de référence relative à l'image du modèle de référence observé dans les conditions d'acquisition virtuelles approximant au mieux les conditions d'acquisition réelles en conséquence de l'exécution des étapes 01) à 06), et
- la carte « acquise » représentant l'information discriminante à tester correspondant à l'image acquise
pondéré par la quantité d'information discriminante à tester.

Pour un contour par exemple, le degré de concordance peut être le rapport du nombre de points qui appartiennent à la fois au contour de la carte de référence et au contour de la carte acquise, sur le nombre de points total du contour de la carte acquise, ou le produit de l'inverse de la distance moyenne entre les contours représentés sur lesdites cartes acquise et de référence, et de la longueur du contour représenté sur la carte acquise. L'approximation «au mieux» des conditions d'acquisition réelles à partir d'une information discriminante à tester peut être évaluée par un résultat ou « score », par exemple par le degré de concordance. Le cycle des étapes C2. et C3. a pour but d'optimiser ce résultat en agissant sur l'information discriminante à tester.

Cette optimisation est analogue à celle mise en œuvre pour les procédés de contrôle du positionnement et/ou de la forme des dents. Ces procédés agissent cependant sur le modèle de référence initial, par déplacement des modèles de dents et/ou par déformation du modèle de référence, alors que le procédé d'optimisation de l'information discriminante agit sur l'information discriminante utilisée pour établir la carte acquise.

L'opération effectuée à l'étape C3. est déterminée par une fonction d'évaluation du résultat du test de l'étape C2.. De préférence, la fonction d'évaluation prend en considération des résultats obtenus lors de cycles C2.-C3. précédents.

En particulier, le procédé peut être arrêté si la fonction d'évaluation indique que la poursuite de cycles C2.-C3. ne permet pas d'améliorer le résultat, par exemple parce que un ou plusieurs cycles C2.-C3. n'ont pas permis de l'améliorer ou n'ont pas permis de l'améliorer significativement. L'information discriminante testée lors du cycle C2.-C3. ayant conduit au meilleur résultat est alors considérée comme optimale.

Sinon, un nouveau cycle C2.-C3. peut être lancé, après modification de l'information discriminante à tester. La modification à apporter à l'information discriminante qui vient d'être testée peut consister en un ajout ou en une suppression d'information discriminante. Un ajout d'information discriminante peut par exemple être décidé si le dernier résultat est le meilleur obtenu jusqu'alors et si, d'après la fonction d'évaluation, le résultat peut être encore amélioré. De préférence, l'information discriminante ajoutée est celle qui, parmi l'information discriminante non retenue à l'étape C1. et qui n'a pas encore été testée, présente la meilleure qualité.

Par exemple, lorsque l'information discriminante est une information de contour, l'ajout d'information discriminante peut consister à ajouter des points de l'image non retenus initialement, encore jamais ajoutés et dont la qualité, telle qu'évaluée à l'étape C1., est la plus élevée, c'est-à-dire dont l'ajout est le plus susceptible d'améliorer le résultat du test de l'étape C2..

Une suppression d'information discriminante peut par exemple être décidée si le dernier résultat est pire que le précédent. En particulier, l'information discriminante ajoutée lors du cycle précédent peut être supprimée et un ajout d'une autre information discriminante peut être effectué, comme décrit précédemment.

La détermination de l'information discriminante à ajouter et/ou à supprimer peut être aléatoire. Cependant, de préférence, elle résulte de la mise en œuvre d'une méthode métaheuristique, de préférence évolutionniste, de préférence par recuit simulé, de préférence du type de celles décrites ci-dessus.

### Exemple

A titre d'exemple, la figure 11 représente, superposée à l'image acquise, une carte de référence relative à une information de reflet (information discriminante).

L'image acquise, de préférence acquise au moyen d'un dit kit d'acquisition, est une photographie prise dans une position et une orientation particulières de l'appareil photo. Cette position et cette orientation constituent les conditions d'acquisition réelles de l'image acquise.

L'homme de l'art sait que, sur l'image acquise, plus la luminosité d'un point est élevée, plus la probabilité que ce point appartienne à une zone de reflet est élevée. La luminosité peut donc servir d'indicateur de la « qualité » de l'information de reflet.

Le filtrage à l'étape C1. peut consister à ne retenir que les zones de l'image acquise qui présentent une luminosité supérieure à un seuil de qualité, par exemple de 70%.

La carte acquise représente ces zones, qui constituent l'information discriminante à tester. A l'étape C2., on procède de préférence selon les étapes 01) à 06) pour tester si les zones retenues concordent avec l'observation du modèle de référence.

A l'étape 01), on analyse l'image acquise pour évaluer grossièrement les conditions d'acquisition réelles par des conditions d'acquisition virtuelles. Cette évaluation résulte de préférence d'une analyse de la position et/ou de la forme de la représentation, sur l'image acquise, de marques de repérage d'un écarteur utilisé lors de l'acquisition.

Les conditions d'acquisition virtuelles évaluées grossièrement peuvent constituer les « conditions d'acquisition virtuelles à tester » à l'étape 02).

A l'étape 03), en observant le modèle de référence à partir de conditions d'acquisition virtuelle à tester, on obtient une image de référence.

A l'étape 04), comme représenté sur la figure 11, on peut projeter, sur l'image de référence, des vecteurs (traits noirs 20), tous de même longueur, perpendiculaires aux faces du modèle de référence. Les cercles 22 représentent ces vecteurs lorsqu'ils sont observés selon leur longueur. La carte de référence est ainsi constituée par ces traits noirs 20 et cercles 22.

L'homme de l'art sait que les cercles 22 correspondent normalement à des zones de l'image correspondant à des reflets. Sur la carte de référence, l'information discriminante, à savoir l'information de reflet, est donc représentée par les traits noirs 20 et par les cercles 22, l'inverse de la longueur des traits noirs pouvant servir d'indicateur de la « qualité » de l'information de reflet sur la carte de référence, un cercle correspondant à une longueur nulle, et donc à une qualité maximale.

A l'étape 05), une comparaison des cartes acquise et de référence peut par exemple consister à vérifier si les traits 20 et les cercles 22 sont à l'intérieur de zones de la carte acquise (qui correspondent initialement à une luminosité supérieure à 70%). La fonction d'évaluation peut être par exemple le rapport R entre
- la différence entre le nombre de cercles 22 et le nombre de traits qui sont à l'intérieur de zones de l'image acquise et
- le nombre total de cercles 22.

La décision peut être de poursuivre la recherche en modifiant les conditions d'acquisition virtuelles à tester jusqu'à atteindre un maximum pour la fonction d'acquisition.

A l'étape 06), si ce maximum est considéré comme atteint, on sort de la boucle des étapes 03) à 06). Sinon, on détermine, de préférence au moyen d'une méthode métaheuristique, de préférence évolutionniste, une modification à apporter aux conditions d'acquisition virtuelles à tester et on reprend l'étape 03).

A la sortie des étapes 03) à 06), le rapport R est donc maximal, pour cette image acquise, par exemple de 95%. Ce rapport R représente alors le résultat du test de concordance effectué à l'étape C2.

A l'étape C3, ce résultat est évalué au moyen d'une fonction d'évaluation.

Cette fonction d'évaluation détermine de préférence si le résultat est optimal ou non. Si le résultat est considéré optimal, par exemple parce qu'aucun meilleur résultat ne peut plus être obtenu après plusieurs cycles des étapes C2. - C3., on considère que les zones de la carte acquise correspondante constituent une information discriminante optimale.

Sinon, on modifie la carte acquise, par exemple en ajoutant des points de l'image acquise, par exemple en ajoutant des points qui présentent une luminosité supérieure à 71%. Cet ajout conduit à l'ajout de cercles dans les zones de la carte acquise, ce qui améliore le rapport R, mais aussi à l'ajout de traits noirs, ce qui dégrade le rapport R. Réciproquement, il est possible d'extraire les points qui présentent une luminosité supérieure à 69%. Cet suppression conduit à la suppression de cercles dans les zones de la carte acquise, ce qui dégrade le rapport R, mais aussi à la suppression de traits noirs, ce qui améliore le rapport R.

La modification à apporter est de préférence guidée par une méthode métaheuristique de préférence évolutionniste.

A partir de la nouvelle carte acquise, qui définit l'information discriminante à tester, on reprend à l'étape C2..

Le cyclage des étapes C2. et C3. peut être poursuivi jusqu'à déterminer un résultat optimal. L'information discriminante à tester est alors considérée comme optimale.

Comme cela apparaît clairement à présent, un dit procédé d'optimisation d'une information discriminante permet de construire une information discriminante de bonne qualité et abondante. En particulier, il permet, à partir d'un contour initial partiel, mais de bonne qualité, de construire progressivement un contour plus complet mais toujours de bonne qualité.

Bien entendu, l'invention n'est pas limitée aux modes de réalisation décrits ci-dessus et représentés.

En particulier, sauf indication contraires, les caractéristiques optionnelles décrites dans le cadre d'une étape d'un premier procédé décrit sont applicables dans le cadre d'une étape analogue ou portant la même référence d'un deuxième procédé décrit.

Le procédé de contrôle du positionnement et/ou de la forme des dents peut être mis en œuvre successivement pour chacune des deux arcades ou simultanément pour les deux arcades. En outre, pour ces procédés, plusieurs appareils différents peuvent être mis en œuvre. Par exemple, l'acquisition peut être effectuée avec un téléphone mobile et les étapes suivantes avec un ordinateur fixe.

Enfin, le patient n'est pas limité à un être humain. En particulier, un dit procédé de contrôle du positionnement de dents peut être utilisé pour un autre animal.

## Revendications

1. Système de fabrication d'un appareil orthodontique de substitution à un appareil orthodontique porté par un patient, le système comportant :
- un scanner pour réaliser un modèle de référence tridimensionnel numérique d'au moins une partie d'une arcade du patient, ou « modèle de référence initial » ;
- un ordinateur pour réaliser un modèle de référence tridimensionnel numérique, dit « modèle de référence objectif », correspondant à un positionnement, souhaité à un instant du traitement, des dents de ladite au moins une partie de ladite arcade du patient ;
- un appareil d'acquisition d'images pour acquérir au moins une image actualisée bidimensionnelle, l'appareil d'acquisition d'images étant positionné et orienté dans l'espace, relativement aux dents du patient, dans des conditions d'acquisition dites « réelles » ;
- un ordinateur pour déformer le modèle de référence initial, par déplacement de modèles de dent, jusqu'à obtenir un modèle de référence actualisé correspondant au positionnement des dents sur l'image actualisée bidimensionnelle ;
- un dispositif de fabrication d'un nouvel appareil orthodontique si une comparaison réalisée par ordinateur
- du modèle de référence actualisé avec
- un modèle de comparaison dans lequel les dents du modèle de référence initial sont dans un positionnement anticipé, tel que prévu sensiblement pour l'instant de l'acquisition, et déterminé en fonction du modèle de référence objectif et optionnellement du modèle de référence initial
aboutit à un résultat insatisfaisant, le nouvel appareil orthodontique étant configuré en fonction dudit résultat.

2. Système selon la revendication immédiatement précédente, dans lequel, pour déformer le modèle de référence initial, l'ordinateur est configuré pour réaliser les étapes suivantes :
c) Analyse de chaque image actualisée et réalisation, pour chaque image actualisée, d'une carte actualisée relative à une information discriminante ;
d) Optionnellement, détermination, pour chaque image actualisée, de conditions d'acquisition virtuelles grossières approximant lesdites conditions d'acquisition réelles ;
e) Recherche, pour chaque image actualisée, d'un modèle de référence actualisé correspondant au positionnement des dents lors de l'acquisition de l'image actualisée, la recherche étant de préférence effectuée au moyen d'une méthode métaheuristique, de préférence évolutionniste, de préférence par recuit simulé, l'étape e) comportant les étapes suivantes :
e1) définition d'un modèle de référence à tester comme étant le modèle de référence initial puis,
e2) suivant les étapes suivantes, test de conditions d'acquisition virtuelles avec le modèle de référence à tester afin d'approximer finement lesdites conditions d'acquisition réelles ;
e21) détermination de conditions d'acquisition virtuelles à tester;
e22) réalisation d'une image de référence bidimensionnelle du modèle de référence à tester dans lesdites conditions d'acquisition virtuelles à tester ;
e23) traitement de l'image de référence pour réaliser au moins une carte de référence représentant, au moins partiellement, ladite information discriminante ;
e24) comparaison des cartes actualisée et de référence de manière à déterminer une valeur pour une première fonction d'évaluation, ladite valeur pour la première fonction d'évaluation dépendant des différences entre lesdites cartes actualisée et de référence et correspondant à une décision de poursuivre ou de stopper la recherche de conditions d'acquisition virtuelles approximant lesdites conditions d'acquisition réelles avec plus d'exactitude que lesdites conditions d'acquisition virtuelles à tester déterminées à la dernière occurrence de l'étape e21) ;
e25) si ladite valeur pour la première fonction d'évaluation correspond à une décision de poursuivre ladite recherche, modification des conditions d'acquisition virtuelles à tester, puis reprise à l'étape e22) ;
e3) détermination d'une valeur pour une deuxième fonction d'évaluation, ladite valeur pour la deuxième fonction d'évaluation dépendant des différences entre les cartes actualisée et de référence dans les conditions d'acquisition virtuelles approximant au mieux lesdites conditions d'acquisition réelles et résultant de la dernière occurrence de l'étape e2), ladite valeur pour la deuxième fonction d'évaluation correspondant à une décision de poursuivre ou de stopper la recherche d'un modèle de référence approximant le positionnement des dents lors de l'acquisition de l'image actualisée avec plus d'exactitude que ledit modèle de référence à tester utilisé à la dernière occurrence de l'étape e2), et si ladite valeur pour la deuxième fonction d'évaluation correspond à une décision de poursuivre ladite recherche, modification du modèle de référence à tester par déplacement d'un ou plusieurs modèles de dents, puis reprise à l'étape e2).

3. Système selon l'une quelconque des revendications précédentes, dans lequel le modèle de comparaison fournit une estimation du positionnement, prévu pour un instant intermédiaire, des dents du modèle de référence initial, la durée entre ledit instant intermédiaire et l'instant de l'acquisition étant inférieur à 1 mois.

4. Système selon l'une quelconque des revendications précédentes, dans lequel l'appareil d'acquisition d'images est un téléphone du patient configuré pour réaliser la recherche du modèle de référence actualisé et transmettre le modèle de référence actualisé à un orthodontiste.

5. Système selon l'une quelconque des revendications précédentes, dans lequel le nouvel appareil orthodontique est une gouttière orthodontique pour remplacer l'appareil orthodontique porté.

## Patentansprüche

1. System zur Fertigung einer orthodontischen Vorrichtung zur Ersetzung einer orthodontischen Vorrichtung, die von einem Patienten getragen wird, wobei das System Folgendes aufweist:
- einen Scanner zur Erstellung eines digitalen dreidimensionalen Referenzmodells wenigstens eines Abschnitts eines Zahnbogens des Patienten, oder "initiales Referenzmodell";
- einen Computer zur Erstellung eines digitalen dreidimensionalen Referenzmodells, "objektives Referenzmodell" genannt, das einer zu einem Zeitpunkt der Behandlung gewünschten Positionierung der Zähne des wenigstens einen Abschnitts des Zahnbogens des Patienten entspricht;
- eine Bilderfassungsvorrichtung zur Erfassung wenigstens eines zweidimensionalen aktualisierten Bilds, wobei die Bilderfassungsvorrichtung in Bezug auf die Zähne des Patienten unter "realen" Erfassungsbedingungen im Raum positioniert und ausgerichtet ist;
- einen Computer zur Verformung des initialen Referenzmodells durch Verschiebung von Zahnmodellen, bis ein aktualisiertes Referenzmodell erhalten wird, das der Positionierung der Zähne auf dem zweidimensionalen aktualisierten Bild entspricht;
- eine Vorrichtung zur Fertigung einer neuen orthodontischen Vorrichtung, wenn ein computererstellter Vergleich
- des aktualisierten Referenzmodells mit
- einem Vergleichsmodell, bei dem sich die Zähne initialen Referenzmodells in einer antizipierten Positionierung befinden, wie im Wesentlichen für den Zeitpunkt der Erfassung vorgesehen, und je nach dem objektiven Referenzmodell und wahlweise dem initialen Referenzmodell bestimmt, zu einem unzufriedenstellenden Ergebnis führt, wobei die orthodontische Vorrichtung je nach dem Ergebnis ausgebildet ist.

2. System nach dem unmittelbar vorhergehenden Anspruch, wobei zur Verformung des initialen Referenzmodells der Computer dazu ausgebildet ist, die folgenden Schritte durchzuführen:
c) Analysieren jedes aktualisierten Bilds und Erstellen, für jedes aktualisierte Bild, einer aktualisierten Karte in Bezug auf eine unterscheidende Information;
d) Wahlweise, Bestimmen, für jedes aktualisierte Bild, grober virtueller Erfassungsbedingungen, die sich an die realen Erfassungsbedingungen annähern;
e) Suchen, für jedes aktualisierte Bild, nach einem aktualisierten Referenzmodell, das der Positionierung der Zähne bei der Erfassung des aktualisierten Bilds entspricht, wobei die Suche vorzugsweise mittels eines metaheuristischen, vorzugsweise evolutionistischen Verfahrens, vorzugsweise durch simuliertes Ausglühen durchgeführt wird, wobei der Schritt e) die folgenden Schritte aufweist:
e1) Festlegen eines zu testenden Referenzmodells als das initiale Referenzmodell, dann
e2) je nach den folgenden Schritten, Testen von virtuellen Erfassungsbedingungen mit dem zu testenden Referenzmodell zur Feinannäherung an die realen Erfassungsbedingungen;
e21) Bestimmen von zu testenden virtuellen Erfassungsbedingungen;
e22) Erstellen eines zweidimensionalen Referenzbilds des zu testenden Referenzmodells unter den zu testenden Erfassungsbedingungen;
e23) Verarbeiten des Referenzbilds zur Erstellung wenigstens einer Referenzkarte, die wenigstens teilweise die unterscheidende Information darstellt;
e24) Vergleichen der aktualisierten und der Referenzkarte, um einen Wert für eine erste Evaluierungsfunktion zu bestimmen, wobei der Wert für die erste Evaluierungsfunktion von den Differenzen zwischen der aktualisierten und der Referenzkarte abhängt und einer Entscheidung entspricht, die Suche nach virtuellen Erfassungsbedingungen fortzusetzen oder zu stoppen, die sich an die realen Erfassungsbedingungen genauer als die zu testenden virtuellen Erfassungsbedingungen annähern, die beim letzten Auftreten des Schritts e21) bestimmt werden;
e25) wenn der Wert für die erste Evaluierungsfunktion einer Entscheidung entspricht, die Suche fortzusetzen, Ändern der zu testenden virtuellen Erfassungsbedingungen, dann Wiederaufnahme bei Schritt e22);
e3) Bestimmen eines Werts für eine zweite Evaluierungsfunktion, wobei der Wert für die zweite Evaluierungsfunktion von den Differenzen zwischen der aktualisierten und der Referenzkarte abhängt, unter den virtuellen Erfassungsbedingungen, die sich den realen Erfassungsbedingungen bestmöglich annähern und sich aus dem letzten Auftreten des Schritts e2) ergeben, wobei der Wert für die zweite Evaluierungsfunktion einer Entscheidung entspricht, die Suche nach einem Referenzmodell fortzusetzen oder zu stoppen, das sich der Positionierung der Zähne bei der Erfassung des aktualisierten Bilds genauer als das zu testende Referenzmodell annähert, das beim letzten Auftreten des Schritts e2) genutzt wird, und wenn der Wert für die zweite Evaluierungsfunktion einer Entscheidung entspricht, die Suche fortzusetzen, Ändern des zu testenden Referenzmodells durch Verschiebung eines oder mehrerer Zahnmodelle, dann Wiederaufnahme bei Schritt e2).

3. System nach einem der vorhergehenden Ansprüche, wobei das Vergleichsmodell eine Schätzung der für einen Zwischenzeitpunkt vorgesehenen Positionierung der Zähne des initialen Referenzmodells liefert, wobei die Zeitdauer zwischen dem Zwischenzeitpunkt und dem Zeitpunkt der Erfassung kleiner als 1 Monat ist.

4. System nach einem der vorhergehenden Ansprüche, wobei die Bilderfassungsvorrichtung ein Telefon des Patienten ist, das dazu ausgebildet ist, die Suche nach dem aktualisierten Referenzmodell durchzuführen und das aktualisierte Referenzmodell an einen Kieferorthopäden zu übermitteln.

5. System nach einem der vorhergehenden Ansprüche, wobei die neue orthodontische Vorrichtung eine orthodontische Schiene zur Ersetzung der getragenen orthodontischen Vorrichtung ist.

## Claims

1. System for manufacturing an orthodontic appliance for replacing an orthodontic appliance worn by a patient, the system including:
- a scanner for creating a numerical three-dimensional reference model of at least a part of a patient's arch, or the "initial reference model";
- a computer for creating a numerical three-dimensional reference model, called the "objective reference model", corresponding to a desired positioning, at a time point of the treatment, of the teeth of said at least a part of said patient's arch;
- an image-acquiring apparatus for acquiring at least one two-dimensional updated image, the image-acquiring apparatus being positioned and oriented in space, relatively to the teeth of the patient, under acquisition conditions called "actual" acquisition conditions;
- a computer for deforming the initial reference model, by moving tooth models, until an updated reference model corresponding to the positioning of the teeth in the two-dimensional updated image is obtained;
- a device for manufacturing a new orthodontic appliance if a comparison, made by computer,
- of the updated reference model with
- a comparison model in which the teeth of the initial reference model are in an anticipated positioning, such as essentially planned for the time point of the acquisition, and determined depending on the objective reference module and optionally on the initial reference module
leads to an unsatisfactory result, the new orthodontic appliance being configured depending on said result.

2. System according to the immediately preceding claim, wherein, to deform the initial reference model, the computer is configured to carry out the following steps:
c) Analyzing each updated image and creating, for each updated image, an updated map relating to a discriminant information;
d) Optionally, determining, for each updated image, virtual acquisition conditions roughly approximating said actual acquisition conditions;
e) Searching, for each updated image, for an updated reference model corresponding to the positioning of the teeth when the updated image is acquired, the search preferably being done by means of a metaheuristic method, preferably an evolutionary method, preferably by simulated annealing, step e) including the following steps:
e1) defining a reference model to test as being the initial reference model then,
e2) following the next steps, testing virtual acquisition conditions with the reference model to test in order to finely approximate said actual acquisition conditions;
e21) determining virtual acquisition conditions to test;
e22) creating a two-dimensional reference image of the reference model to test under said virtual acquisition conditions to test;
e23) processing the reference image to create at least one reference map representing, at least partially, said discriminant information;
e24) comparing the updated and reference maps so as to determine a value for a first evaluation function, said value for the first evaluation function depending on the differences between said updated and reference maps and corresponding to a decision to continue or to stop the search for virtual acquisition conditions approximating said actual acquisition conditions with more accuracy than said virtual acquisition conditions to test determined at the last occurrence of step e21);
e25) if said value for the first evaluation function corresponds to a decision to continue said search, modification of the virtual acquisition conditions to test, then resumption at step e22);
e3) determining a value for a second evaluation function, said value for the second evaluation function depending on the differences between the updated and reference maps under the virtual acquisition conditions best approximating said actual acquisition conditions and resulting from the last occurrence of step e2), said value for the second evaluation function corresponding to a decision to continue or stop the search for a reference model approximating the positioning of the teeth during the acquisition of the updated image with more accuracy than said reference model to test used at the last occurrence of step e2), and if said value for the second evaluation function corresponds to a decision to continue said search, modification of the reference model to test by movement of one or more tooth models, then resumption at step e2).

3. System according to any one of the preceding claims, wherein the comparison model provides an estimation of the positioning, planned for an intermediate time point, of the teeth of the initial reference model, the duration between said intermediate time point and the time point of the acquisition being less than 1 month.

4. System according to any one of the preceding claims, wherein the image-acquiring apparatus is a telephone of the patient configured to do the search for the updated reference model and to transmit the updated reference model to an orthodontist.

5. System according to any one of the preceding claims, wherein the new orthodontic appliance is an orthodontic aligner for replacing the orthodontic appliance worn.
